(19) Europäisches Patentamt
European Patent Office
Office européen des brevets

(11) **EP 4 610 345 A1**

(12) **EUROPEAN PATENT APPLICATION**

(43) Date of publication:
**03.09.2025 Bulletin 2025/36**

(21) Application number: **24161030.2**

(22) Date of filing: **01.03.2024**

(51) International Patent Classification (IPC):
**C12M 1/36** (2006.01)   **G05B 13/04** (2006.01)
**G01N 30/86** (2006.01)   **C12M 1/00** (2006.01)

(52) Cooperative Patent Classification (CPC):
**C12M 41/48; C12M 47/12; G01N 30/8662;
G05B 13/048; B01D 15/08**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Sartorius Stedim Data Analytics AB
903 33 Umeå (SE)**

(72) Inventors:
• **McCREADY, Christopher Peter
903 33 Umeå (SE)**
• **VALIPOUR, Mashad
903 33 Umeå (SE)**
• **CORBETT, Brandon
903 33 Umeå (SE)**

(74) Representative: **Mewburn Ellis LLP
Aurora Building
Counterslip
Bristol BS1 6BX (GB)**

(54) **CONTROL OF CONTINUOUS BIOPROCESSES**

(57)    The present disclosure provides methods, systems and products for controlling a bioprocess comprising a cell culture in a bioreactor, wherein the bioprocess is operated as a perfusion bioprocess. the methods use a model predictive control to identify values of manipulated variables that optimise an objective function, the objective function including one or more constraints and/or one or more terms that are associated with predictions obtained from a method of simulating a chromatographic process that is coupled with the bioprocess for processing of a harvest stream of the bioprocess.

Fig. 6

**Description**

Field of the Present Disclosure

The present disclosure relates to computer implemented methods, computer programs and systems for the controlling of continuous bioprocesses. Particular methods, programs and systems of the disclosure use model predictive control to determine adjustments of manipulated variables that satisfy requirements associated with a downstream process.

Background

Bioprocesses use biological systems to produce a specific product, for example a biomolecule with therapeutic effects (e.g. antibody, viral particle, etc.). This process typically involves placing cells and/or microbes into a bioreactor with media containing nutrients under controlled atmospheric conditions. The media is consumed by the cells and used for growth and other metabolic functions, including production of the specific product and by-products.

Perfusion operating modes, where fresh media is continuously added to the bioreactor while spent media is continuously removed to maintain a constant working volume while retaining cells in the bioreactor, are becoming increasingly popular. Indeed, such operating modes can enable to reach high viable cell concentration maintained over prolonged periods of time. However, stable and reliable process performance in terms of cell growth and productivity of the bioprocess is crucially dependent on the precise and robust control of the process throughout its duration.

A simple approach that is often used to control such processes is to control the feed flow rate to maintain a predefined ratio of feed flow rate and viable cell density (VCD, also referred to as "viable cell concentration", VCC). This is commonly referred to as "cell specific perfusion rate" (CSPR) control. Such a control strategy can be implemented inline or off-line using VCD measurements or estimations. These can then be used to adapt the feed flow rate accordingly, to ensure cell-specific perfusion rate (CSPR) control, via three simple PID control loops where feed flow rate is adjusted to maintain a target CSPR, bleed flow rate is adjusted to maintain a desired VCD target and harvest (or permeate) flow rate is adjusted to maintain the working volume. This type of simplistic control approaches can result in cyclic behaviour, which is detrimental to the process, and is also unable to adapt to changes in the cell culture such as metabolic shifts.

Therefore, there is still a need for improved systems and methods for control of perfusion flow bioreactors.

Summary

In previous work described in WO 2023/237614, the inventors provided a method for controlling a bioprocess using a controller that identifies a value of manipulated variables that optimises a control objective using a prediction of the value of the subset of controlled variables from a dynamic model of the bioprocess describing the rate of change of controlled variables (i.e. a model predictive controller). While this addresses some of the problems of the prior art such as stability, the present inventors have recognised that the approach can still be improved upon. In particular, the present inventors have recognised that operation of continuous biopharmaceutical manufacturing systems practically require coordination between the upstream cell culture process (USP) and downstream product purification steps (DSP), and that the use of supervisory control methods could enable end-to-end system level optimization, i.e. coordination of USP and DSP processes through supervisory control. They therefore designed a new control approach as described herein, where a predictive model of DSP is used to communicate to USP one or more of operational constraints in inlet flow, product concentration and quality as well as economic cost/value. For example, a predictive model of USP can be used to search through the DSP feasible region to determine a most profitable operating point.

According to a first aspect of the disclosure, there is provided a method for controlling a bioprocess comprising a cell culture in a bioreactor, wherein the bioprocess is operated as a perfusion bioprocess , the method including the steps of: (i) receiving one or more constraints that apply to one or more controlled and/or manipulated variables of the controlled bioprocess; and (ii) identifying a value of one or more manipulated variables of the controlled bioprocess that optimises an objective function that is a function of one or more variables of the bioprocess including one or more controlled variables, wherein values of the controlled variables are predicted using a dynamic model of the bioprocess , and the identified values are such that the constraints that apply to one or more controlled and/or manipulated variables of the controlled bioprocess are satisfied. The constraints comprise constraints identified using a method of simulating a chromatographic process that is coupled with the bioprocess for processing of a harvest stream of the bioprocess and/or the objective function comprises a term that is associated with predictions obtained from a method of simulating a chromatographic process using chromatographic process operating parameters derived from one or more manipulated and/or controlled variables of the bioprocess.

The method of the present aspect may have any one or any combination of the following optional features.

The objective function can comprise a cost of goods function that captures the costs associated with values of one or more variables of the bioprocess, wherein said costs comprise costs associated with the chromatographic process estimated using the method of simulating a chromatographic process, optionally wherein said costs further comprise costs associated with the bioprocess. The cost of goods function can be a function of at least the harvest flow rate and concentration of one or more products in the harvest flow rate ( $\sum_{i=k+1}^{k+P} Cost_{total}(F_{h_i}, \varphi_i)$ )

The cost of goods function can be a function that maps: (i) a pair of operating parameters comprising a value of a harvest flow rate or range of harvest flow rates, and a value of a harvest product concentration or range of harvest product concentration, and optionally the concentration of one or more further compounds in the harvest flow, to (ii) one or more metrics derived from a prediction of the method of simulating a chromatographic process over a predetermined period of time which are associated with positive or negative costs due to the materials and devices used in the chromatographic process, and/or the characteristics of the product recovered through the chromatography process.

Said identifying can comprises determining the costs associated with one or more candidate sets of variables of the bioprocess comprising a harvest flow rate and a harvest product concentration using said harvest flow rate and harvest product concentration as input to a method of simulating a chromatographic process, or receiving previously determined costs associated with a plurality of candidate sets of variables of the bioprocess comprising a harvest flow rate and a harvest product concentration using said harvest flow rate and harvest product concentration as input to a method of simulating a chromatographic process.

The objective function can comprise a term that penalises differences between the prediction of the value of the controlled variables from the dynamic model and corresponding predetermined target values. The objective function can comprise a term that penalises changes in the value of the one or more manipulated variables. The objective function can comprise a term that penalises differences between a feed flow rate and a target feed flow rate associated with a predetermined perfusion target perfusion rate. The objective function can comprise a term that rewards maximisation of one or more predetermined variables. The term that rewards maximisation of one or more predetermined variables can be

expressed as $\Upsilon_{z_i} \sum_{i=k+1}^{k+P} \frac{1}{z_i^2+1}$ where z is any predetermined variable to be maximized, and $\Upsilon_{z_i}$ is a weight set to a non-zero scalar value that sets the importance of the term relative to other terms of the objective function, k is a current time and k+P is a prediction horizon over which the dynamic model is evaluated.

The method can comprise using a state observer to provide an estimate of the value of one or more state variables of the bioprocess, the one or more state variables of the bioprocess comprising the one or more controlled variables, using one or more measurements of the state variables of the bioprocess and the dynamic model of the bioprocess . The state observer can be a moving horizon estimator. The state observer can be a moving horizon estimator that treats one or more coefficients of the dynamic model of the bioprocess as state variables to be estimated.

The method can comprise identifying one or more values of the one or more manipulated variables that minimises the control objective over a time period, and controlling the bioprocess to use one of the one or more values for each of the one or more manipulated variables. Identifying a value of one or more manipulated variables of the controlled bioprocess that optimises an objective function can be performed according to equation (1). The objective function can be a function as described in equation (2). Identifying a value of the one or more manipulated variables that optimises a control objective can comprise using the dynamic model of the bioprocess to predict a state trajectory of the bioprocess with one or more candidate values of the one or more manipulated variables, by finding values of the one or more state space variables that represent a solution of the dynamic model at one or more future time points, and using the prediction of the value of the subset of controlled variables in said state trajectory as variables of the control objective. The method can comprise selecting and comparing candidate values using an optimisation algorithm.

The dynamic model can comprise a kinetic growth model, optionally a model according to equations (11) to (14).

The method of simulating a chromatographic process can comprise, for a chromatography process whereby a feed flow comprising one or more products is passing through a chromatography unit comprising a stationary phase that the one or more products interact with: solving a bulk flow mass balance model over one or more discrete volume elements along the chromatography unit by numerical integration, wherein the bulk flow mass balance model is an ordinary differential equations model that represents the change in concentration of bound and unbound fractions of the one or more products in a discrete volume element, wherein the ordinary differential equations model comprises binding and diffusion terms, wherein a binding term captures the binding of a product to the stationary phase and a diffusion term captures the diffusion of a bound product, wherein said binding and diffusion terms are each parameterised by a single respective parameter that is predicted by a machine learning model trained to take inputs comprising the concentration of the bound and unbound fractions of the one or more products in a discrete volume element and produce as output a prediction of the parameters of the binding and diffusion terms.

The bulk flow mass balance model can comprise linear terms representing the flow of unbound compounds (products and optionally inerts) in and out of the discrete volume element, and the binding and diffusion terms, wherein a binding term captures non-linearities of the process of adsorption of unbound product on the static phase through the prediction of the respective parameter of the term by the machine learning model at each iteration of the numerical integration. The bulk flow mass balance model can comprise equations (8) and (9) and optionally equation (10) below:

$$\frac{d\vec{c}_{u,n}}{dt} = \frac{f_k}{\Delta V}\left(\vec{c}_{u,n-1} - \vec{c}_{u,n}\right) - \tilde{k}_a \circ \vec{c}_{u,n} + \vec{k}_d \circ \vec{c}_{b,n} \tag{8}$$

$$\frac{d\vec{c}_{b,n}}{dt} = \tilde{k}_a \circ \vec{c}_{u,n} - \vec{k}_d \circ \vec{c}_{b,n} \tag{9}$$

$$\frac{d\vec{c}_{i,n}}{dt} = \frac{f_k}{\Delta V}\left(\vec{c}_{i,n-1} - \vec{c}_{i,n}\right) \tag{10}$$

where $f_k$ is the feed flow rate, $\Delta V$ is the volume of the discrete volume element $n$, $\vec{c}_{u,n}$ is a vector of concentrations of the unbound products in the discrete volume element n, $\vec{c}_{b,n}$ is a vector of concentrations of the bound products in the discrete volume element $n$, $\vec{c}_{u,n-1}$ is a vector of concentrations of the unbound products in the discrete volume element preceding the discrete volume element $n$, where discrete volume elements are sequentially labelled from input to output of the chromatography unit, $\vec{c}_{i,n}$ is a vector of concentrations of one or more inerts in the discrete volume element $n$, $\vec{c}_{i,n-1}$ is a vector of concentrations of the inerts in the discrete volume element preceding the discrete volume element $n$, and $\circ$ is an elementwise product.

The machine learning model can be a recurrent machine learning model, wherein a recurrent machine learning model is a machine learning model that is able to account of the values of one or more predictions made at one or more preceding iterations of the numerical integration when making predictions at a current iteration of the numerical integration.

All of the steps of the methods described herein are computer implemented unless context indicates otherwise. In particular, any of the steps of the present method may be implemented by a computing device, optionally in operable communication with one or more sensors, other computing devices and/or user interfaces.

According to a second aspect, there is provided a system for controlling a bioprocess, the system including: at least one processor; and at least one non-transitory computer readable medium containing instructions that, when executed by the at least one processor, cause the at least one processor to perform operations comprising: the steps of any method of any preceding aspect. In particular, the at least one non-transitory computer readable medium may contain instructions that, when executed by the at least one processor, cause the at least one processor to perform operations comprising any of the operations described in relation to the first aspect.

The system can further comprise, in operable connection with the processor, one or more of: a user interface; one or more biomass sensor(s); one or more metabolite sensor(s); one or more volume/level sensors; a control unit; one or more effector device(s), optionally wherein the one or more effector devices are operable to set the value of one or more manipulated variables, and a chromatography control unit.

According to a further aspect, there is provided a non-transitory computer readable medium comprising instructions that, when executed by at least one processor, cause the at least one processor to perform the method of any embodiment of any aspect described herein.

According to a further aspect, there is provided a computer program comprising code which, when the code is executed on a computer, causes the computer to perform the method of any embodiment of any aspect described herein.

Brief Description of the Drawings

Embodiments of the present disclosure will now be described by way of example with reference to the accompanying drawings in which:

**Figure 1** shows a simplified process diagram for a generic bioprocess according to embodiments of the present disclosure.

**Figure 2** illustrates a comparative example using PID control of a continuous cell process; **A.** control diagram; **B.** oscillatory operation of cell culture process using PID control loops as illustrated in A.

**Figure 3** is a flowchart illustrating a bioprocess control method according to embodiments of the disclosure.

**Figure 4** illustrates schematically a method of simulating a chromatographic process usable in embodiments of the present disclosure **(A),** and components used in a method of simulating a chromatographic process usable in embodiments of the disclosure **(B).**

**Figure 5** illustrates schematically a system according to embodiments of the present disclosure.

**Figure 6** illustrates schematically the general structure of a closed loop autonomous control method and system according to the disclosure.

**Figure 7** illustrates schematically the concept of nonlinear model predictive control (NMPC) and moving horizon estimation (MHE) finite time horizons.

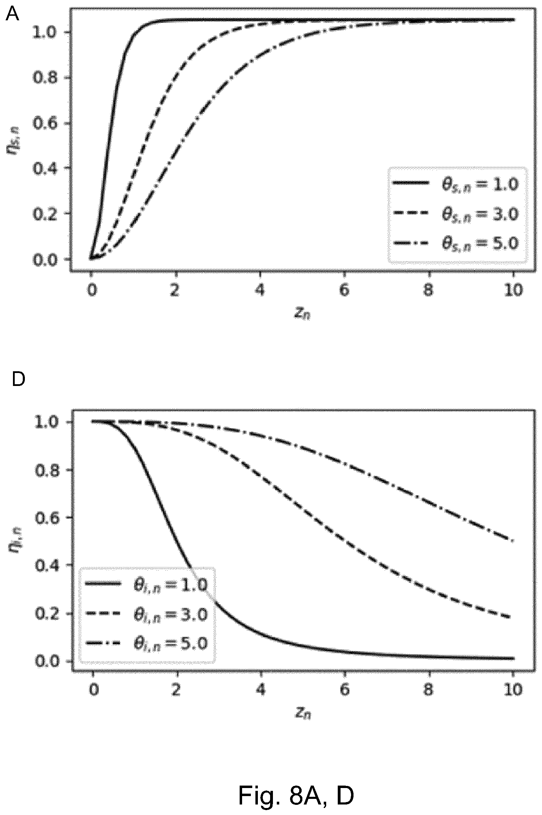

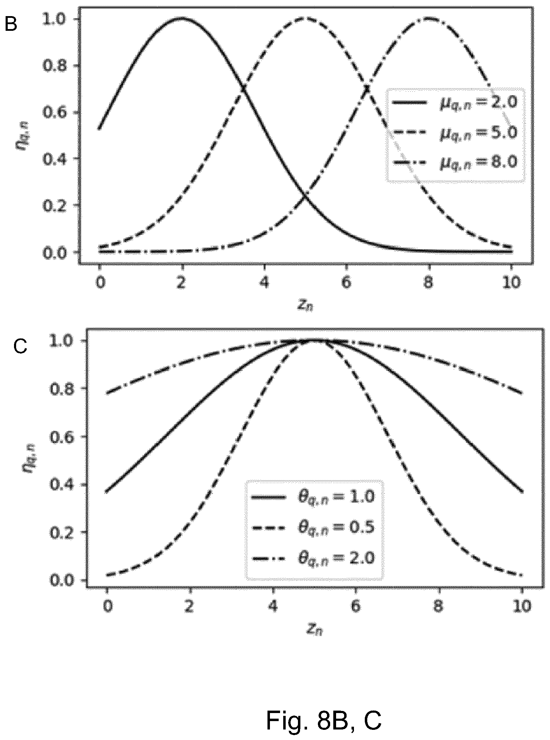

**Figure 8** illustrates the impact of selected parameters on variables of a kinetic growth model according to embodiments of the present disclosure. A shows the effect of a substrate limiting variable. D shows the effect of an inhibitory variable as a function of the concentration of the inhibitory variable, for different values of a parameter $\theta_{i,n}$ representing the level of the variable above which inhibition occurs. B-C show the value of a correction factor (y-axis) capturing the effect of a variable that has a quadratic effect on growth as a function of the value of the of the quadratic effect variable (x-axis, e.g. temperature, pH) for three exemplary values of the parameter parameter $\theta_{q,n}$ at a fixed value of $\mu_{q,n}$ (C) and three exemplary values of the parameter $\mu_{q,n}$ at a fixed value of $\theta_{q,n}$ (A), where in these examples parameter $\theta_{q,n}$ represents the spread of the effect and parameter $\mu_{q,n}$ represents the value at which maximum growth occurs.

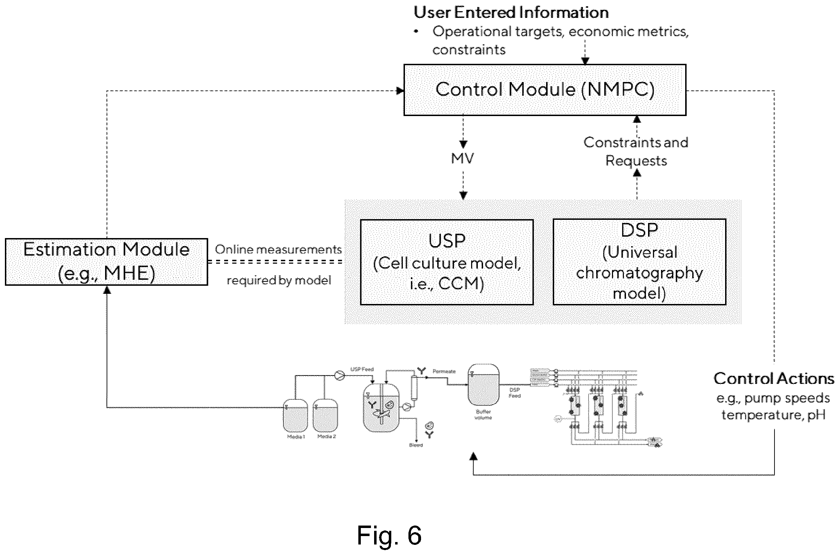

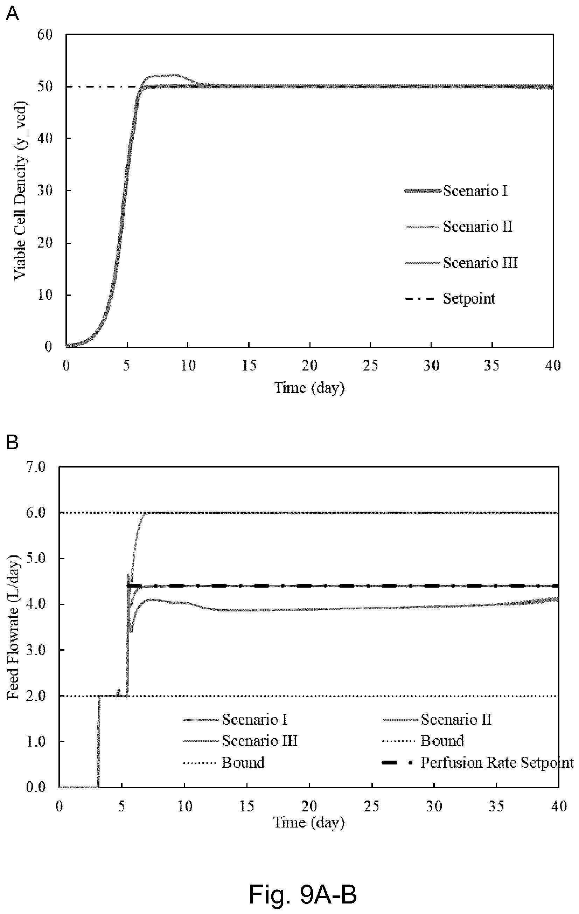

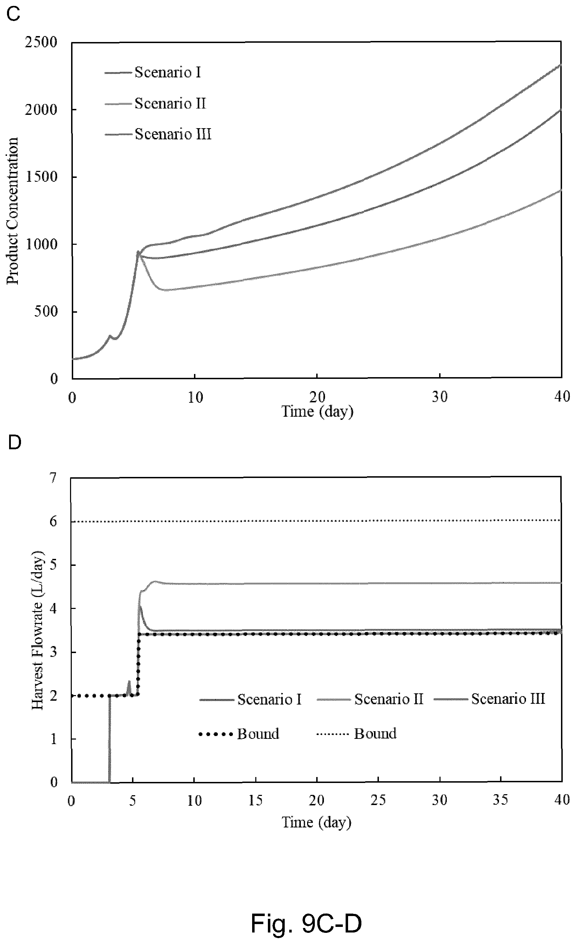

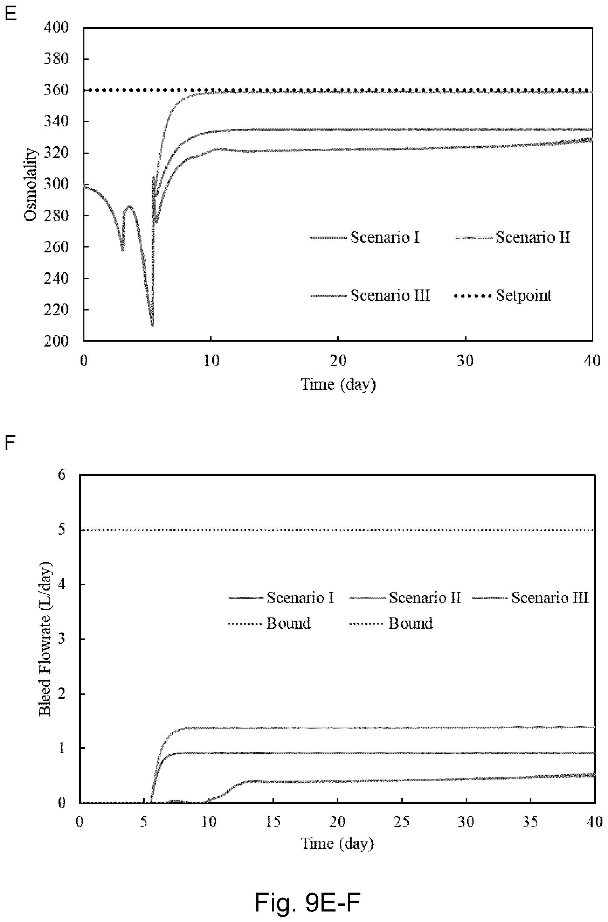

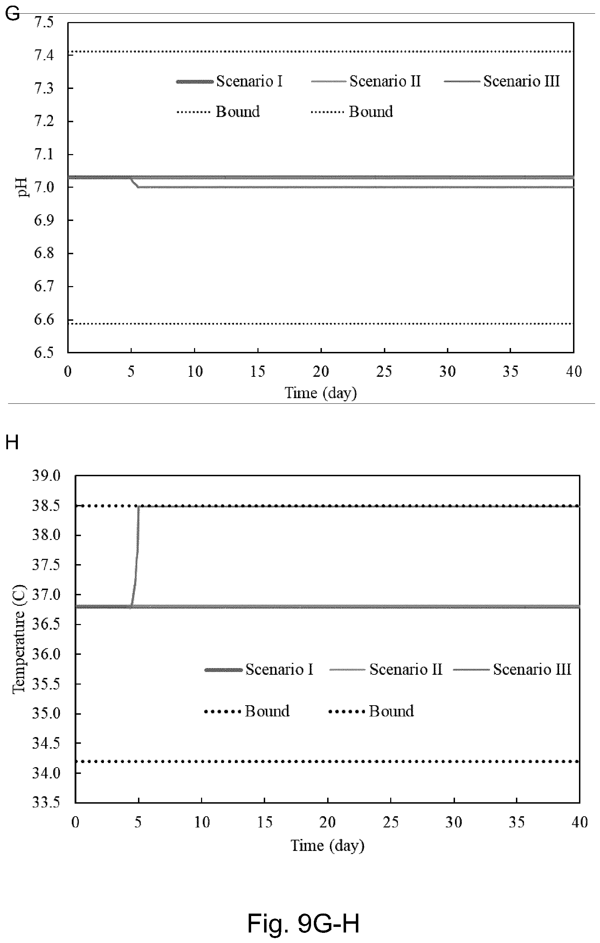

**Figure 9** shows results of the use of the system of Figure 6 to control a perfusion flow bioreactor under 3 different control scenarios (labelled Scenario I, II and III) explored in Example 2. Each plot shows a different controlled or manipulated variable as a function of time in the process (in days): A. viable cell density, B. feed flow rate, C. product concentration (titer), D. harvest flow rate, E. osmolality, F. bleed flow rate, G. pH, H. temperature. Plots D, F, G and H also shows as dashed lines any bounds applied to the variable as process operating constraints. Plots A, B and E also show set points of the process as dashed-dotted lines.

**Figure 10** illustrates schematically the structure of a closed loop autonomous control method and system according to embodiments of the disclosure which take into accounts the economics of the process (see Example 3).

**Figure 11** shows an example of the use of a method described herein for control of a continuous culture process using economic modeling of a downstream chromatography process; the heatmaps show the profit associated with running a chromatographic process with the indicated values of titer and flow rate for purification of Protein A, using either a MabSelect™ **(A)** or a Sartobind® Rapid A Membrane **(B)** chromatography column.

**Figure 12** illustrates schematically a discretisation scheme used to describe a chromatographic process for simulation according to embodiments of the disclosure (Example 4).

**Figure 13** illustrates schematically a machine learning model structure used to parameterise a mass balance model according for simulation of a chromatographic process according to embodiments of the disclosure.

Figure 14 A-B illustrate schematically a method of training a machine learning model for simulation of a chromatographic process according to embodiments of the disclosure.

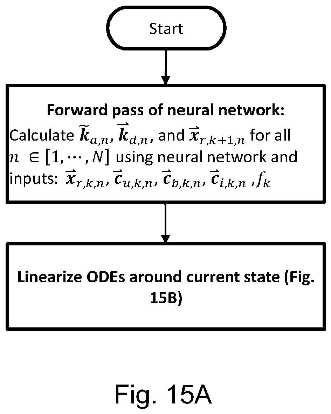

Figures 15A-B illustrate schematically a method of simulating a chromatographic process according to embodiments of the disclosure. **(A:** flow chart illustrating the process used and **B:** mass balance model equations used - note that each of B-1 to B-3 show the same system of equations at the top, with the states of the system in vector form, and develops the terms of the A and B matrices for the states vector corresponding to the concentration of unbound products (B-1), bound products (B-2) and inerts (B-3) along the differential volume elements 1 to n; B-4 combines the information in B-1 to B-3).

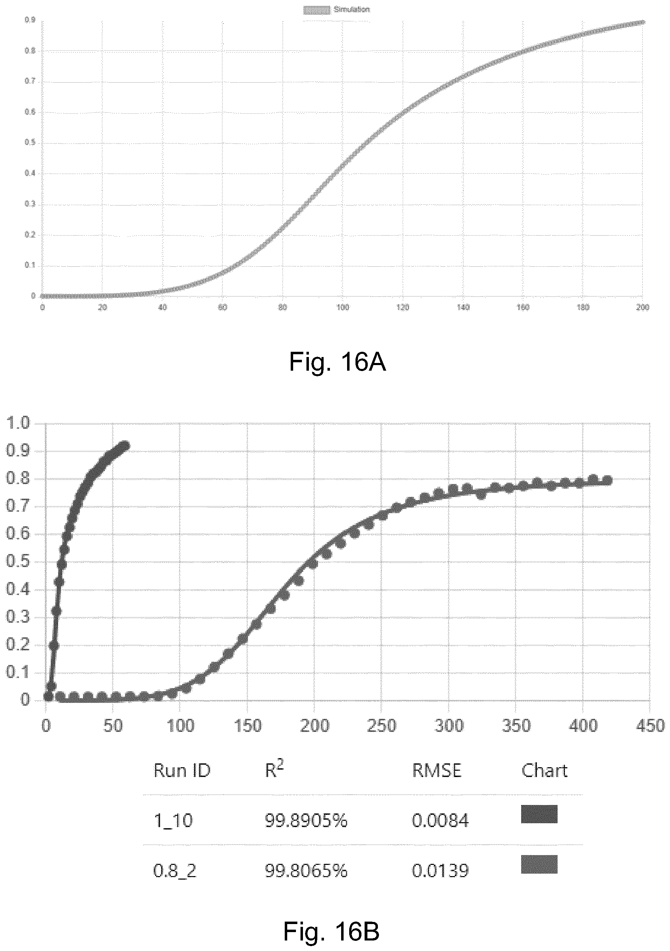

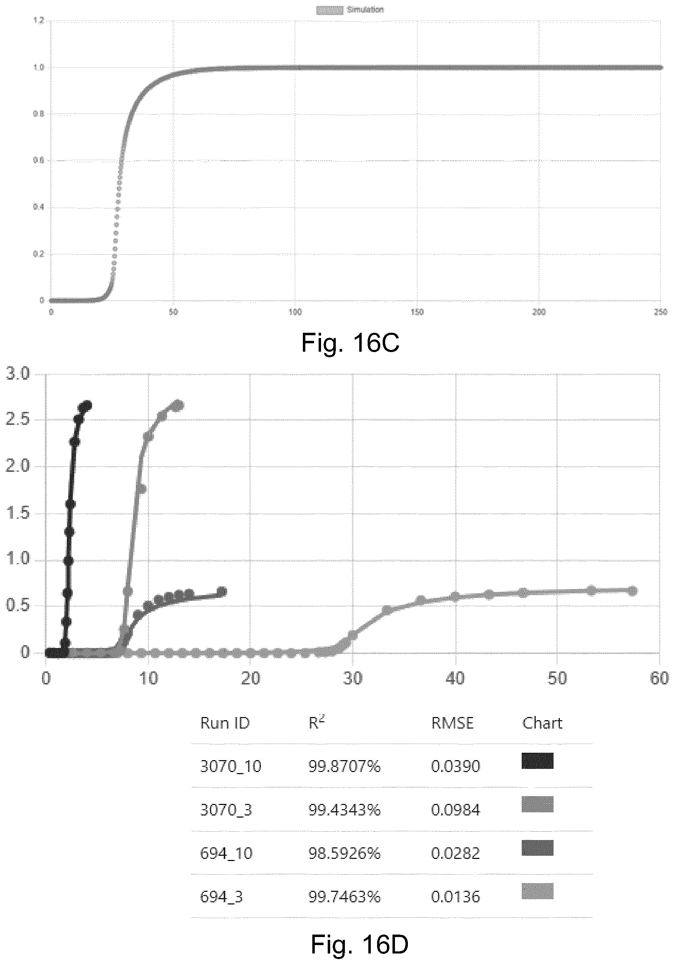

**Figure 16** shows examples results of a chromatographic process simulation method used in embodiments of the disclosure applied to the purification of protein A using the same operating parameters (feed rate=2.5 ml/min, feed concentration=1, simulation duration=200 minutes, breakthrough threshold %=5) but using **(A-B)** a MabSelect™ SuRe chromatography resin from Cytiva (calculated breakthrough time=53.77 min, calculated dynamic binding capacity=134.42 mg/ml) and **(C-D)** a Sartobind® Rapid A Membrane from Sartorius Stedim Biotech GmbH (calculated breakthrough time=24.10 min, calculated dynamic binding capacity=50.20mg/ml); A and C show the results of the simulation, and B and D show model validation data (from experiments at different feeding rates and feed concentrations to demonstrate the validity of the model predictions) and associated $R^2$ and RMSE between predicted (continuous lines) and observed (points) breakthrough curves; each model was trained using real data (4 experimentally obtained breakthrough curves) using the respective substrates and columns; each plot shows breakthrough curves, i.e. product concentration in the effluent (mg/ml) as a function of time (minutes).

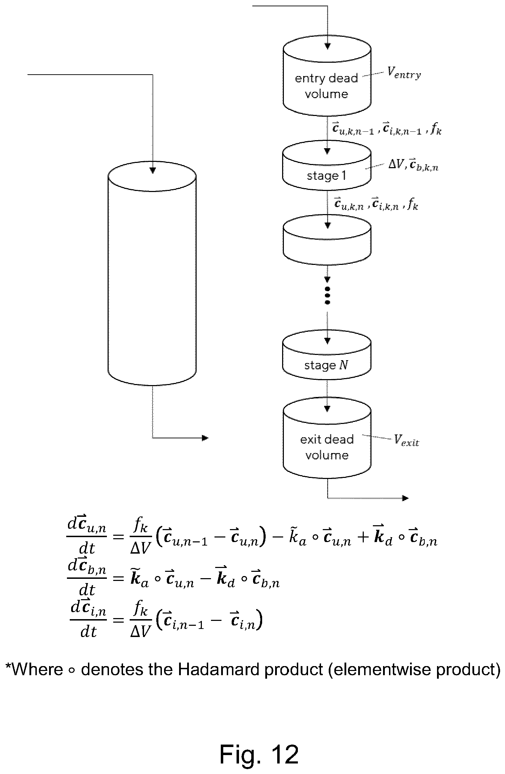

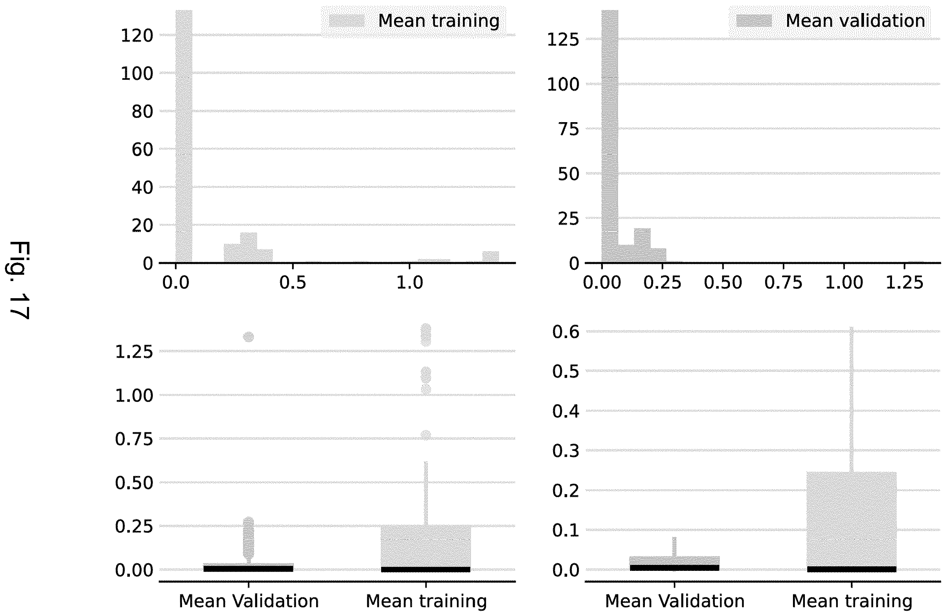

**Figure 17** shows results of an exhaustive assessment of methods of simulating a chromatographic process described herein using data generated with a numerically evaluated general rate model (CADET simulation). Simulated data (CADET simulated breakthrough curves) was obtained from a numerically evaluated general rate model for training and validation of a model as described in relation to Figures 12 to 15. For each experiment (set of operating parameters) the model was trained on four timeseries and validated on a final series, and loss (difference between predicted and "observed" breakthrough curves) was calculated for each time series. The figure shows histograms of mean losses across validation and training datasets (respectively top left and top right panels) for each of 180 experiments (see Example 5), and distributions of mean losses across validation and training datasets (respectively bottom left and bottom right panels) illustrated as boxplots (thick black line=median, whiskers show 1.5 of interquartile range, points show lowest data point above Q1-1.5*(Q3-Q1) and highest data point below Q3+1.5*(Q3-Q1) where Q1 and Q3 are the first and third quartiles).

Where the figures laid out herein illustrate embodiments of the present invention, these should not be construed as limiting to the scope of the invention. Where appropriate, like reference numerals will be used in different figures to relate to the same structural features of the illustrated embodiments.

Detailed Description

Specific embodiments of the invention will be described below with reference to the figures. The present disclosure provide methods of controlling continuous bioprocesses (also referred to as perfusion flow bioprocesses) which take into accounts effects of the operating of the bioprocess on a downstream product processing step (e.g. a chromatographic process).

Broadly, the present disclosure provides methods, systems and products for controlling a bioprocess comprising a cell culture in a bioreactor, wherein the bioprocess is operated as a perfusion bioprocess. the methods use a model predictive control to identify values of manipulated variables that optimise an objective function, the objective function including one or more constraints and/or one or more terms that are associated with predictions obtained from a method of simulating a chromatographic process that is coupled with the bioprocess for processing of a harvest stream of the bioprocess.

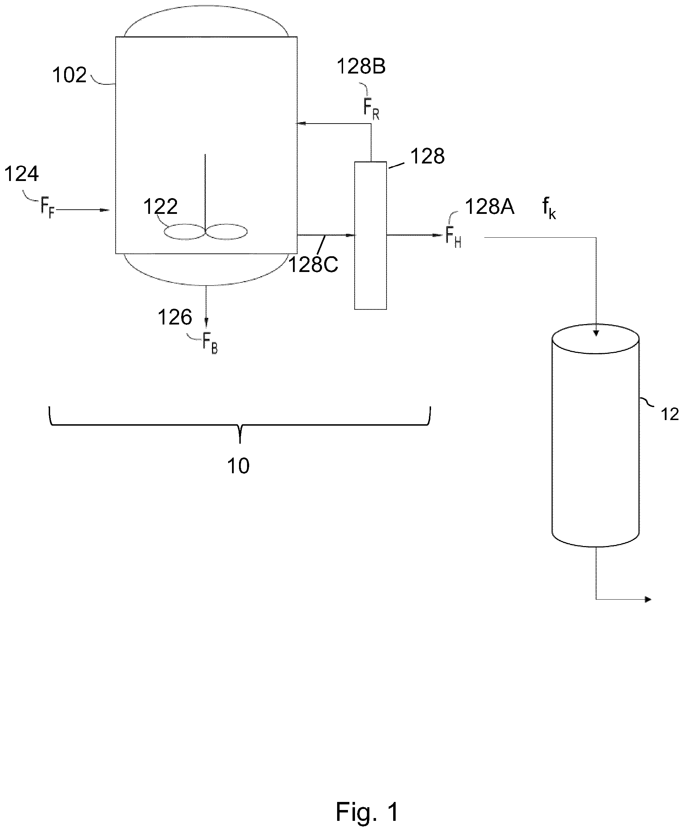

**Figure 1** shows a simplified process diagram for a system in which embodiments of the present disclosure can be used. This comprises an upstream process 10, in which a product is being produced, here illustrated as a bioreactor and associated flows, and a downstream process 12 in which the product of the upstream process (USP) is processed, typically through a chromatographic process. In embodiments where the bioreactor is operated as a perfusion process, as illustrated and as explained further below, a permeate (also referred to as "harvest flow" or "harvest stream") is obtained from the bioreactor with a flow rate $f_H$. The permeate is typically the primary output of the upstream process, comprising one or more products of interest. In such embodiments the output of the upstream process 10 can be continuously processed in a downstream process 12, which is typically a chromatography process. The downstream process 12 comprises a chromatography unit, here illustrated as a single chromatography column although multiple columns in parallel or in series may in fact be used. A chromatography unit can include one or more chromatography columns and a flow control system. The chromatography unit receives an input feed with flow rate $f_k$. The chromatography unit produces an output feed with flow rate $f_k$, where the output feed has a different concentration of the one or more products of interest. The input feed can be obtained directly from the upstream process, i.e. in the case of a perfusion process the input feed of the chromatography unit can be directly linked to (or equal) to the harvest flow of the upstream process.

A "chromatographic process" refers to any process that makes use of liquid chromatography, particularly in the context of purifying one or more products present in a feed solution. A chromatographic process is typically performed in a chromatography unit comprising one or more individual chromatography columns. Each column comprises a stationary phase (e.g. resin, membrane, monoliths), and a mobile phase. The methods of the present disclosure are not limited in any way in terms of the geometry of the column(s), organisation of multiple columns (e.g. connection of multiple columns in series), type of stationary or mobile phase. Liquid chromatography is typically used to purify one or more molecules, compounds or particles of interest (collectively referred to as "products" or "bioproducts" by reference to the fact that chromatography is commonly use in the purification of products of bioprocesses). The products can be proteins or particles such as viral particles. Purification of products is performed by loading the product(s) onto the column buy flowing a feed liquid comprising the product(s) through the column, where the products interact with the stationary phase and are immobilised thereon (where they are referred to as "bound product"). When the column has been loaded with product(s), the column is typically washed, and then a buffer solution is flowed through the column in an elution phase, where the buffer is chosen such that the affinity of products to the stationary phase is reduced and the products are eluted out of the column. The outcoming flow of a chromatography column can be referred to as "effluent", whether in the loading or elution phase.

As used herein, the term "bioprocess" (also referred to herein as "biomanufacturing process") refers to a process where biological components such as cells, parts thereof such as organelles or multicellular structures such as organoids or spheroids are maintained in a liquid medium in an artificial environment such as a bioreactor. In the context of the present disclosure, a bioprocess typically refers to a cell culture. A bioprocess typically results in a product, which can include biomass and/or one or more compounds that are produced as a result of the activity of the biological components. For example, live cells can be cultured to a desired cell density then used in a fermentation process to produce one or more desired products in a bioreactor. This is typically referred to as "upstream process" (USP). The one or more desired products may be extracted from the cells or the culture medium in a downstream process (DSP). A downstream process may comprise one or more steps, such as separation and/or purification steps. A bioreactor can be a single-use vessel or a multi-use vessel in which a liquid medium suitable for carrying out a bioprocess can be contained. For example, a bioreactor may be chosen from: multi-parallel single-use bioreactors (such as e.g. Ambr® 250 or Ambr® 15 bioreactors from The Automation Partnership Ltd.), single use bag-based bioreactors (e.g. such as Biostat® STR bioreactors from Sartorius Stedim Biotech GmbH, available in 50 to 2000L capacity for process development up to commercial manufacturing), stainless steel bioreactors (such as e.g. Biostat® D-DCU bioreactors available in capacities from 10 to 200L or Biostat® Cplus available in capacities from 5 to 30L, all from Sartorius Stedim Systems GmbH), benchtop systems (such as e.g. Biostat® B and Biostat® B-DCU from Sartorius Stedim Systems GmbH, which support either 2L single-use rigid wall vessel or 1, 2, 5 and 10L glass vessels , like Univessel® SU in capacity of 2L and Univessel® Glass available in capacities from 2 to 10L, all from Sartorius Stedim Biotech GmbH), etc. The present invention is applicable in the context of downstream processes associated with upstream processes in any type of bioreactor and in particular in bioreactors from any vendor and at any scale from benchtop systems to manufacturing scale systems.

A cell culture refers to a bioprocess whereby live cells are maintained in an artificial environment such as a bioreactor. The methods, tools and systems described herein are applicable to bioprocesses that use any types of cells that can be maintained in culture, whether eukaryotic or prokaryotic. The invention can in particular be used to monitor and/or control bioprocesses using cells types including but not limited to mammalian cells (such as Chinese hamster ovary (CHO) cells, human embryonic kidney (HEK) cells, Vero cells, etc.), non-mammalian animal cells (such as e.g. chicken embryo fibroblast (CEF) cells), insect cells (such as e.g. *D. melanogaster cells, B. mori* cells, etc.), animal cells of any cell type (such as e.g. induced pluripotent stem cells (iPSCs), stem cells such as e.g. mesenchymal stem cells, immune cells such as T cells and natural killer cells), bacterial cells (such as e.g. *E. coli* cells), fungal (e.g. yeast) cells (such as e.g. *S. cerevisiae* cells), and plant cells (such as e.g. *A. thaliana* cells). A bioprocess typically results in the production of a product, which can be the cells themselves (e.g. a cell population for use in further bioprocesses, a cell population for use in cell therapy, a cell population for use as a product such as a probiotic, feedstock, etc.), a macromolecule or macromolecular structure such as a protein, peptide, nucleic acid or viral particle (e.g. a monoclonal antibody, immunogenic protein or peptide, a viral or non-viral vector for gene therapy, enzymes such as e.g. for use in the food industry, for environmental applications such as water purification, decontamination, etc.), or a small molecule (e.g. alcohols, sugars, amino acids, etc.).

When the product comprises a macromolecule or macromolecular structure such as a protein, peptide, nucleic acid or viral particle, the downstream process typically comprises at least one chromatographic separation step, i.e. a chromatographic process. The present disclosure provides methods that apply to such processes. The methods of the present disclosure are applicable to USP that are operated in combination with any DSP (e.g. any chromatographic process), including those performed on single and multi-column platforms of any scale (including but not limited to e.g. the Resolute® BioSC Platform and the Resolute® BioSMB Platform from Sartorius Stedim Chromatography Systems LTd.), those performed using any chromatographic stationary phase known in the art such as resins, membranes, and monoliths, and those performed for separation of any types of analytes including proteins (e.g. protein A, antibodies and fragments thereof including e.g. monoclonal antibodies, enzymes), viral particles, small molecules (e.g. metabolites), etc.

A product of a bioprocess (also referred to herein as "target biologic") may include a metabolite, a cell, a desired protein, an antibody, an immunoglobulin, a toxin, one or more by-products, a target molecule, or any other type of molecule manufactured using a bioprocess. There may be more than one product of interest. Products of a bioprocess may have one or more critical quality attributes (CQAs). As used herein, a "critical quality attribute" is any property of a product (including in particular any chemical, physical, biological and microbiological property) that can be defined and measured to characterise the quality of a product. The quality characteristics of a product (in terms of the values of one or more CQAs) may be defined to ensure that the safety and efficacy of a product is maintained within predetermined boundaries. The term "metabolite" refers to any molecule that is consumed or produced by a cell in a bioprocess. Metabolites include in particular nutrients such as e.g. glucose, amino acids etc., by-products such as e.g. lactate and ammonia, desired products such as e.g. recombinant proteins or peptides, complex molecules that participate in biomass production such as e.g. lipids and nucleic acids, as well as any other molecules such as oxygen ($O_2$) that are consumed or produced by the cell. Depending on the particular situation, the same molecule may be considered a nutrient, a by-product or a desired product, and this may even change as a bioprocess is operated. However, all molecules that take part in cellular metabolism (whether as an input or output of reactions performed by the cellular machinery) are referred to herein as "metabolites". In particular, metabolites may include any suitable analyte, including but not limited to: amino acids (e.g., alanine, arginine, aspartic acid, asparagine, cysteine, cysteine, glutamic acid, glutamine, glycine, histidine, hydroxyproline, isoleucine, leucine, lysine, methionine, phenylalanine, proline, serine, threonine, tryptophan, tyrosine, valine, etc.), saccharides (e.g., fucose, galactose, glucose, glucose-1-phosphate, lactose, mannose, raffinose, sucrose, xylose, etc.), organic acids (e.g., acetic acid, butyric and 2-hydroxy- butyric acids, 3-hydroxybutyric acid, citric acid, formic acid, fumaric acid, isovaleric acid, lactic acid, maleic acid, propionic acid, pyruvic acid, succinic acid, etc.), other organic compounds (e.g., acetone, ethanol, pyroglutamic acid, etc.).

As illustrated on **Figure 1,** a generic bioprocess is typically implemented in a bioreactor 102, which in the embodiment shown is equipped with agitation means 122. Four flows (also referred to herein as "streams") are depicted, although depending on the particular situation any or all of these flows may be absent. A first flow 124 is a feed flow $F_F$ (also referred to herein as "perfusion flow", or $f_F$) containing anything that is added to the culture in the bioreactor (typically this includes fresh medium, in which case the bioprocess may be referred to as a "fed-batch" process, "perfusion" process or "continuous" process), a second flow 126 is a bleed flow $F_B$ (also referred to as $f_B$) that has the same composition as the culture in the bioreactor, a third flow 128A is a permeate $F_H$ (also referred to as "harvest flow" or "harvest stream", or $f_H$) which is obtained through the processing of an auxiliary harvest stream 128C through a cell retention device 128 (CRD, which can be any cell separation means adapted to separate cells from medium) to produce the third (harvest) and fourth flow 128B, the fourth flow 128B being a recycle flow $F_R$ (also referred to as "retentate") comprising the cells and any culture medium that has not been completely separated out in the cell retention device1 28. In embodiments, the recycle flow $F_R$ may be ignored as considering only the permeate flow $F_H$ is sufficient to capture the flow that is effectively output from the bioreactor through the harvesting and cell separation process. Therefore, the reference to a permeate being present or absent may refer to the auxiliary harvest stream 128C (and derived permeate and retentate - $F_H$ and $F_R$) being present or absent. This permeate $F_H$ may be assumed to comprise medium that has the same composition as the medium in the reactor, but no or few cells. The feed, bleed and permeate flows ($F_F$, $F_B$, $F_H$ and $F_R$) may all be absent, in which case the bioprocess is referred to as an "unfed batch process" or simply "batch process". When a feed flow $F_F$ and a permeate flow $F_H$ are provided, the bioprocess may be referred to as a "perfusion" culture. The process may be referred to as "N-1 perfusion" when the feed flow and permeate flow are present, maintaining the volume of the culture constant in the absence of a bleed flow. The process may be referred to as a "dynamic perfusion" when the feed flow and the permeate and bleed flows are provided such that the bioprocess is operated at (pseudo) steady-state (from a process condition point of view, i.e. maintaining in particular the volume of the culture constant). In both cases, the bioprocess may be referred to as a "continuous" culture. The methods of the present disclosure relate in particular to the control of continuous bioprocesses (i.e. perfusion bioprocesses). When a feed flow $F_F$ is provided in the absence of output flows (bleed flow and permeate, $F_B$ and $F_H$), the bioprocess may be referred to as a "fed-batch" process. The terms "feed", "feed flow", or "feed rate" may be used interchangeably to refer to the flow rate of culture medium in the bioreactor in the feed flow, typically in units of volume per unit of time (such as e.g. litre/hour). The terms "harvest", "harvest flow", "permeate", "permeate rate", "permeate flow" or "harvest rate" may be used interchangeably to refer to the flow rate of culture medium in the permeate, typically in units of volume per unit of time (such as e.g. litre/hour). The terms "bleed", "bleed flow" or "bleed rate" may be used interchangeably to refer to the flow rate of culture medium in the bleed flow, typically in units of volume per unit of time (such as e.g. litre/hour). The feed flow and permeate flow are typically cell free culture media (or essentially cell free, depending on the performance of the cell retention device in the case of the permeate). The bleed flow typically includes culture media and cells. The feed flow may be characterised by one or more parameters including the concentration of one or more substrates therein. The harvest and bleed flows may be characterised by one or more variables including the flow itself (typically in units of volume per time, e.g. litre/hour), and/or the concentration of one or more substrates and/or one or more products therein, typically in units of mass per volume (e.g. g/l). The bleed flow may further be characterised by the viable cell density (also referred to as "viable cell concentration", typically in number of cells per unit of volume - where the viable cell density in the bleed flow is assumed to be the same as the viable cell density in the vessel). The VCD, and concentrations of one or more substrates or products may be assumed to be the same in the bleed flow and in the bioreactor. A viable cell density (the term being used interchangeably with viable cell concentration in the present disclosure) is typically expressed in units of E06/ml, i.e. $10^6$ cells/ml. Additional parameters may be known for each of the flows and/or additional variables may be measured or otherwise determined depending on the particular circumstances of the bioprocess.

The present disclosure relates to an advanced control strategy for cell culture processes, in particular for perfusion operating modes. As explained above, perfusion refers to the use of media exchange where fresh media is continuously added while spent media is continuously removed to maintain a constant working volume. Cells are retained in the cell culture through a separation device such as an alternating tangential flow (ATF) or tangential flow filtration (TFF), where cell free spent media is removed through a harvest stream (permeate), cells are retained via a membrane filter and returned to the bioreactor. In addition to the feed and permeate streams, a bleed stream is typically included to maintain a desired viable cell density (VCD or viable cell concentration, VCC). The bleed contains material contents identical to that in the working volume of the bioreactor. These three flow streams (feed, permeate and bleed) may be controlled to maintain a desired media exchange rate, supply of sufficient nutrients, cell density and working volume. A media exchange rate may be defined as the total volume of medium that flows into the bioreactor or out of the bioreactor per unit of time. The total volume of medium that flows into the bioreactor per unit of time is typically equal to the total volume of medium that flows out of the bioreactor per unit of time.

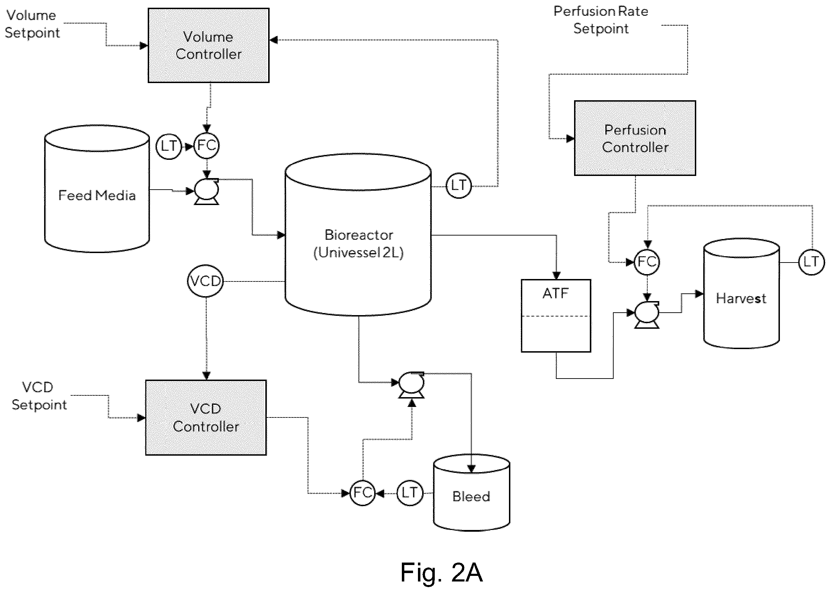

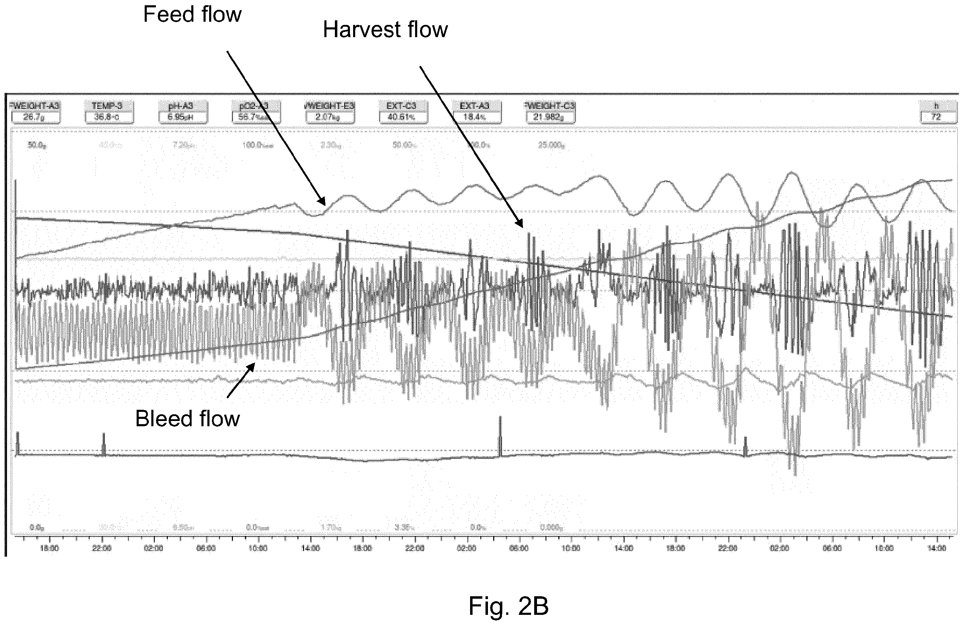

Control of such processes is particularly difficult. In a simple implementation illustrated on **Figure 2A,** three independent single loop controllers (such as PID - proportional integral derivative controller) could be used to control each of the feed, harvest and bleed flows. Control of the feed flow may be used to maintain the media exchange rate (constant target or a ratio of the VCD), or control the concentration of a substrate. Control of the harvest may be used to control the working volume. Control of the bleed flow may be used to control the VCD. In some cases the harvest and feed objectives may be reversed, where harvest is used to maintain the media exchange rate and feed to control volume. As illustrated on **Figure 2B,** in this type of configuration these three control loops interact with each other resulting in oscillatory type behavior, destabilizing the process. This oscillatory behavior may be exacerbated by the pulse type action that is typical for ATF separation devices.

By contrast, according to the present disclosure, state space models (optionally combined with machine learning for metabolic parameter estimation - also referred to as a hybrid cell culture model) are used within a model predictive control (MPC) structure to predict the future trajectory of the parameters to be controlled (referred to as control variable(s) or CV, such as e.g. target substrate concentration, productivity) to determine optimal adjustment to manipulated variables (MV, e.g. one or more of the three flow streams mentioned above) in order to maintain the CV at their targets at a specified future time point. The model predictive controller uses an objective function that takes into accounts constraints and/or costs associated with running the USP and/or the DSP that the USP is coupled to, where these constraints and/or costs are derived from a DSP simulation model. In embodiments, as will be explained further below, the objective function also aims to minimize adjustments to the MV (to control the process in a manner that is more stable than control methods of the prior art). Additionally, the control method may include a self-adaptive state space model update. This may further improve the stability of the control method, especially over long bioprocess runs where e.g. changes in physiology of the cells as well as DSP performance may occur. In embodiments, the objective function also aims to maintain a target permeate flow rate. This may reduce unwanted variations in permeate flow rates that may cause problems for downstream processes.

**Autonomous control of continuous bioprocesses**

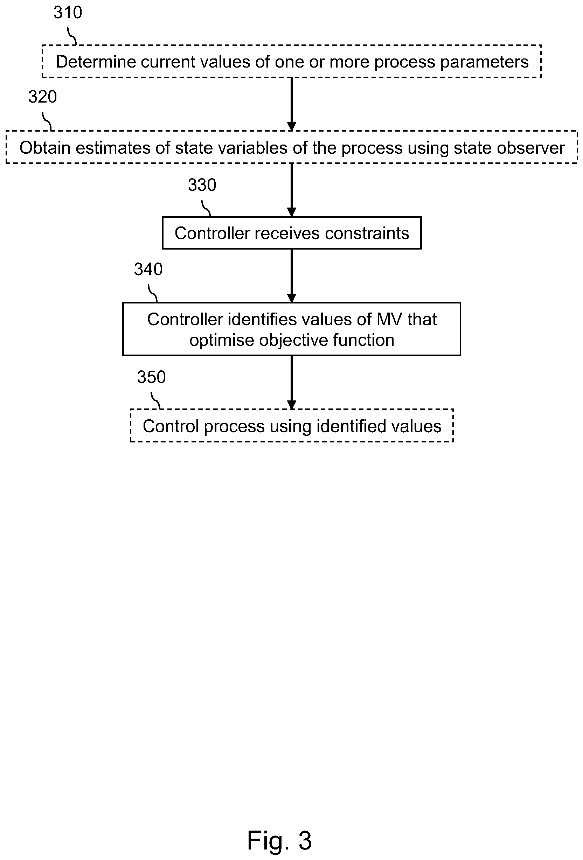

**Figure 3** illustrates schematically a bioprocess control method according to embodiments of the disclosure.

At each iteration of the control method, a set of parameters of the process are measured or otherwise determined at step 310. For example, the following parameters from the process can be obtained during operation of the process: Viable cell density (VCD also called viable cell concentration, VCC), Total cell density (or dead cell density), Volume, feed flow rate, permeate flow rate and bleed flow rate, and optionally also the concentration of a substrate such as e.g. glucose and one or more process parameters such as temperature, pH, osmolality, dissolved oxygen and $CO_2$. These may be measured or set by effectors (e.g. valves controlling a flow rate). In embodiments, one or more of these parameters may not be directly measured but rather calculated from other measurements. For example, the volume may be calculated from a weight from a scale. As another example, one or more flows may be calculated from a rate of change of a weight scale. One or more of these parameters may be measured continuously or close to continuously (e.g. a very frequent intervals), or at least at every iteration of the control method. This may be performed for example using on-line sensors that measure the one or more parameters or determine them from other measurements performed on medium or flows during operation of the bioprocess. Additionally, one of more of these parameters may optionally be measured at low frequency, such as less frequently than at each iteration of the control method. This may be performed for example using off-line protocols, such as e.g. by obtaining a sample of medium and analysing it in a laboratory. Thus, a first set of parameters may be measured at a frequency or set of frequencies that are higher than a frequency or set of frequencies at which a second set of parameters is measured. The first and second set may overlap, i.e. the same parameter may be measured both at a first (higher) frequency (e.g. using an on-line sensor) and at a second (lower) frequency (e.g. by off-line analysis). This may be useful for example when the off-line analysis provides a measurement that is more accurate than the on-line measurement. These measurements provide the current (or latest measured) value of one or more controlled variables and one or more manipulated variables of the system. The measurements are provided to a state observer. The state observer produces estimates of one or more state variables of the bioprocess using the measurements and a state space model, at step 320.

This is optional and the values determined at step 310 may be used directly instead. Thus, the method can comprise using a state observer to provide an estimate of the value of one or more state variables of the bioprocess, the one or more state variables of the bioprocess comprising one or more controlled variables, using one or more measurements of the state variables of the bioprocess and a dynamic model of the bioprocess. In embodiments, the state observer uses measurements obtained at a plurality of preceding iterations (also referred to as a estimation horizon), together with previous state estimates from the state observer over the estimation horizon to estimate a current value for the one or more state variables. thus, in embodiments, the state observer is a moving horizon estimator. The functioning of the state observer will be described in more detail below.

The estimates of the state observer are provided to a model predictive controller, and represent initial conditions for the model predictive controller to predict a trajectory of the bioprocess over a prediction horizon (i.e. a predetermined period of time) using a state space model and candidate control strategies (each corresponding to respective sets of values for one or more manipulated variables, MV). The MCP further receives receiving one or more constraints that apply to one or more controlled and/or manipulated variables of the controlled bioprocess at step 330. Using these and optionally the estimates from the state observer, at step 340, the MPC identifies one or more values of one or more manipulated variables (MV that optimize an objective function). The candidate control strategies typically comprise predicted values of manipulated variables at each of a predetermined number of subsequent iterations (together referred to as the control horizon), followed by maintenance of the last value of each manipulated variable for the rest of the prediction horizon. The controller may further take as input one or more parameters. The one or more parameters may include operational targets (e.g. setpoints and constraints, such a high and/or low thresholds for one or more controlled variables), controller parameters which determine e.g. how the controller balances the different objectives (e.g. the weights of a control objective as described further below), and data from a downstream process model that identifies one or both of operation targets (e.g. constraints defining an operating region that is suitable from the point of view of the DSP) and a set of costs associated with USP operation (i.e. costs associated with values of controlled variables, such as titer and harvest flow rate). Thus, identifying values of controlled variables comprises identifying a value of one or more manipulated variables of the controlled bioprocess that optimizes an objective function that is a function of one or more variables of the bioprocess including one or more controlled variables, wherein values of the controlled variables are predicted using a dynamic model of the bioprocess , and the identified values are such that the constraints that apply to one or more controlled and/or manipulated variables of the controlled bioprocess are satisfied. As will be described further below, the constraints comprise constraints identified using a method of simulating a chromatographic process that is coupled with the bioprocess for processing of a harvest stream of the bioprocess and/or wherein the objective function comprises a term that is associated with predictions obtained from a method of simulating a chromatographic process using chromatographic process operating parameters derived from one or more manipulated and/or controlled variables of the bioprocess. Using these inputs, the controller identifies an optimal control action (i.e. changes to one or more manipulated variables) at step 340. These can be provided to one or more control units, which controls effectors to implement these targets. Thus, at step 350, the bioprocess can be controlled using one of the one or more values for each of the one or more manipulated variables, that were identified at step 340.

As will be described further below, identifying a value of the one or more manipulated variables that optimises a control objective can comprise using the dynamic model of the bioprocess to predict a state trajectory of the bioprocess with one or more candidate values of the one or more manipulated variables, by finding values of the one or more state space variables that represent a solution of the dynamic model at one or more future time points, and using the prediction of the value of the subset of controlled variables in said state trajectory as variables of the control objective. The method can comprise selecting and comparing candidate values using an optimisation algorithm.

A dynamic model of the bioprocess is a model describing the rate of change of the one or more controlled variables (i.e. describing the rate of change of any dependent variables of the process including controlled variables). The dynamic model of the bioprocess can be any of the models described in the section "State Space Model" or in the Examples below. For example, the dynamic model can comprise a kinetic growth model, such as a model according to equations (11) to (14) below.

Identifying the values of the manipulated variables (independent variables of the bioprocess) can comprise determining the costs associated with one or more candidate sets of variables of the bioprocess comprising a harvest flow rate and a harvest product concentration (which are typically dependent variables of the process) using said harvest flow rate and harvest product concentration as input to a method of simulating a chromatographic process. Alternatively, identifying the values of the manipulated variables (independent variables of the bioprocess) can comprise receiving previously determined costs associated with a plurality of candidate sets of variables of the bioprocess comprising a harvest flow rate and a harvest product concentration using said harvest flow rate and harvest product concentration as input to a method of simulating a chromatographic process. Thus, Step 340 can comprise simulating a chromatography process using one or more candidate sets of variables of the bioprocess comprising a harvest flow rate and a harvest product concentration, each set associated with a candidate set of values of manipulated variables. The simulation can be performed using any of the methods described in the section "Simulation of downstream process" and in the Examples below.

The control method may be implemented using an initialization phase, in which the controller is not used for one or more iterations of the control method. This enables the state observer to be initialized. For example, an initial period may be used to collect enough measurements to obtain good predictions from a Kalman Filter or moving horizon estimator. Instead or in addition to this, the initial period may be set for a period such that the cells reach a density that is such that a calculated cell specific perfusion rate (CSPR) is above a minimum feed rate (where a minimum feed rate may be set to ensure that there is a feed flow). For example, at a first iteration, a first set of measurements may be used together with initial (user defined) values for one or more states of the state space model that cannot be directly measured. As explained above and further below, the state space model may be used both by the state observer and by the model predictive controller. These values are used to integrate the state space model to predict the value of state space variables until the next set of measurements is received. At the next iteration, the state space observer is used to calculate new values for the state space variables, using the latest set of measurements and optionally preceding measurements and state estimates. These are again used to integrate the state space model to predict the value of state space variables until the next set of measurements is received. This may be repeated for one or more iterations together forming the initialization period. This results in a number of estimates of the state space variables (also referred to as "system states"), which can be used by the state space observer to obtained improved predictions of state space variables in future iterations. After the initialization phase, the state space observer is still used to calculate new values for the state space variables at every iteration, using the latest set of measurements, but this is then used by the model predictive controller to determine a first control action for one or more manipulated variables. The model predictive controller uses the estimates of the state space variables from the state space observer to integrate the state space model to determine a control action to be taken which is optimal over a prediction horizon. The integration may be performed at a certain frequency until a new set of measurements is obtained, at which point a new iteration starts, and new state space variable estimates are obtained by the state observer.

The functioning of a model predictive controller will now be described in more detail. In the context of the present disclosure, a model predictive control (MPC) is a process control method that involves the prediction of the future trajectory of parameters to be controlled (e.g. titer, production rate etc.) in order to determine optimal adjustments to be made to manipulated variables (e.g. flow streams, temperature, etc.) to maintain the controlled variable at desired targets at a specified future timepoint. The terms "controlled variables" refer to variables of the process that the controller aims to influence (e.g. set to desired targets), by setting the values of manipulated variables (which are variables of the process that the controller has freedom to set in order to achieve the control of the controlled variables). Additional process variables can neither controlled nor manipulated, but can act as constraints to the controller. For example, the controller can control the titer by using a maximum stable cell density that is associated with a high production rate. This uses a model that represents the behaviour of the process, referred to herein as "state space model" (described below). Such models can predict the change in dependent variables (including controlled variables) of the modelled system that will be caused by changes in independent variables (including the manipulated variables) of the modelled system. MPC control involves obtaining measurements indicative of the current state of the bioprocess at time k (also referred to as iteration k), then using an internal dynamic model of the process to simulate one or more states of the system over a prediction horizon k+P. These are used as variables of a control objective function over the prediction horizon (also referred to herein as "objective", as further described below) and an optimisation algorithm is used to identify a control input strategy that minimises the control objective function over the prediction horizon k+P. The behaviour of the system is typically predicted over multiple time points included in the prediction horizon, such as e.g. time points between k and k+P separated by an interval $t_1$ (e.g. [t, t+$t_1$, t+2$t_1$, t+3$t_1$,... , t+T] referring to iterations k, k+1,...,k+P). Typically, the first step of the control input strategy (e.g. value of manipulated variable(s) at k+1) is implemented (or any step corresponding to time points between measurements of the state of the bioprocess), and the calculation is repeated every time a new set of measurements of the current state of the bioprocess is obtained. In particular, the calculation may be conducted every time a new set of one or more on-line measurements of the current state of the bioprocess is obtained. At every calculation step (also referred to herein as "iteration of the control loop", or simply "iteration"), the results of the calculation may include the values of one or more manipulated variables and the values of one or more controlled variables, that are optimal according to a control objective comprising set points for the controlled variables over the prediction horizon. The prediction horizon [t, t+T] corresponding to iterations [k,k+P] has a length T that may be selected to be sufficiently long to capture complex dynamics that are expected to be present, in order to ensure convergence to the optimum solution. For example, the length T of the time horizon may be selected to extend to the time necessary for the system to reach a steady state. Instead or in addition to this, the length of the prediction horizon may be determined using one or more factors selected from: the reactor residence time, availability of model-base estimates as a safety feature in case of sensor downtime or failure, and computational resources.

**Control Objective**

A control solution is found by finding a set of adjustments in manipulated variables that minimize a control objective (also referred to as "control objective function" or "objective function"). A control objective is a mathematics expression quantifying the desirability of operation. According to the present disclosure, the objective function is a function of one or more variables of the bioprocess including one or more controlled variables, and includes one or more constraints that apply to one or more controlled and/or manipulated variables of the controlled bioprocess that must be satisfied. The objective function comprises (through constraints or via a term of the objective function) factors that depend on results of a method of simulating a chromatographic process that is coupled with the bioprocess for processing of a harvest stream of the bioprocess. For example, the constraints can comprise constraints identified using the method of simulating a chromatographic process. Instead or in addition to this, the objective function can comprise a term that is associated with predictions obtained from the method of simulating a chromatographic process. The simulation uses chromatographic process operating parameters derived from one or more manipulated and/or controlled variables of the bioprocess. The constraints can comprise one or more of inequality constraints and bounds. Bounds are maximum and/or minimum values that apply to one or more variables of the bioprocess. Inequality constraints are expressions that must be verified for specific variables or combinations of variables. Inequality constraints can include one or more of: one or more constraints that are derived from a model predicting critical quality attributes (CQA) of the product, one or more constraints that apply to the product concentration (titer), such as e.g. a minimum required product concentration in the bulk fluid (i.e. in the harvest flow), one or more operational constraints, such as e.g. minimum and maximum possible values of flows, or any other process parameter such as pH, T etc. based for example on equipment constraints, one or more constraints that aim to avoid nutrient limitations, such as e.g. by maintaining a substrate such as glucose above a specified threshold, one or more constraints that apply to the cell density, such as e.g. maintaining the viable cell density below a maximum threshold due to known process limitations such as delivery of oxygen, and one or more constraints on biologically relevant states. The latter typically aim to assure a stable and healthy culture. For example, constraints that apply to biologically relevant states can a constraint that specifies a minimum viability (VCD/(VCD + dead cell density) and/or a constraint that maximum limit of unmeasured states from the process model such as lysed cell density or biomaterial. Typically, at least a constraint on the maximum viable cell density and one or more constraints on biologically relevant states are included.

In embodiments, the objective function comprises a cost of goods function that captures the costs associated with values of one or more variables of the bioprocess, wherein said costs comprise costs associated with the chromatographic process estimated using the method of simulating a chromatographic process. In some such embodiments, the costs of goods function can also comprise costs associated with the bioprocess itself. Costs associated with the bioprocess can include e.g. costs associated with the media used in the bioprocess. The cost of goods function can be a function of at least the harvest flow rate and concentration of one or more products in the harvest flow rate: i.e. a function that forms a term of the objective function of the form:

$$\sum_{i=k+1}^{k+P} Cost_{total}(F_{h_i}, \varphi_i)$$

where $F_{h,i}$ is the harvest feed at iteration i, $\varphi_i$ is the product concentration at iteration i, k is the current iteration and P is the prediction horizon of the model predictive controller. The cost of goods function can be a function that maps: (i) a pair of operating parameters comprising a value of a harvest flow rate or range of harvest flow rates, and a value of a harvest product concentration or range of harvest product concentration, and optionally the concentration of one or more further compounds in the harvest flow (which can be products or inerts for the purpose of the chromatography process simulation, see below), to (ii) one or more metrics derived from a prediction of the method of simulating a chromatographic process over a predetermined period of time which are associated with positive or negative costs due to the materials and devices used in the chromatographic process, and/or the characteristics of the product recovered through the chromatography process.

The costs can include one or more (or all of) the following negative costs: costs associated with regeneration (e.g. using a known fixed cost per regeneration associated with each column and an assumption of a fixed number of cycles lifespan for the columns, where any numbers corresponding to the known or expected lifespan of a particular column can be used), the cost of buffers consumed in the chromatography process, the cost of waste generated through the chromatography process. The costs can include one or more additional costs associated with the bioprocess (USP). The costs can include a positive cost (gain) associated with the amount of product recovered (assuming a price per mg of product), which can depend on the quality of the product (measured or predicted from the USP model, e.g. using a machine learning model or other data drive model to predict one or more critical quality attributes of the product, and/or measured or predicted from the chromatography simulation model, for example based on the predicted concentration in the effluent of one or more products or inerts that affect the quality of the product). All of these costs are obtained from metrics such as simulated time for loading, washing, and eluting the column, amounts of buffers associated with each of these phases, time for regenerating a column and frequency given the predicted length of the rest of the chromatography cycle, and predicted concentrations of products and inerts in the effluent. The costs and gains can be combined into a single metric of profit in $/hour of running the process.

A simple cost function could be specified as cost=1/profit, where profit = product_value * product_concentration * harvest_flow - media_cost * feed_flow. Such a cost function aims to maximise the profit associated with the product production (where product concentration can be the product concentration in the harvest flow of the USP or the product concentration in the effluent of the DSP, harvest flow can be the harvest flow of the USP or effluent flow of the DSP (which can be the same)), media cost can be one or more of the cost of feed media of the USP and feed flow of the USP, and the cost of DPS media (e.g. wash and elution buffer) and feed flow the feed flow of the DSP.

The objective function can include one or more additional terms. Each of the terms of the objective function can be associated with a respective weight. The weights can be used to set a term to 0 (i.e. remove it from the control objective) and/or to moderate the impact of each term on the control objective. This can be used to prioritise some terms over others, and to scale terms that have very different scales from others due to the particular functions or metrics that are quantified in the term (e.g. costs can be in units such that they have much higher order of magnitudes than other terms). The weights can vary depending on the iteration. For example, different terms can be prioritized differently (or removed completely) at different times during operation of the bioprocess. For example, the objective function can comprise a term that penalises differences between the prediction of the value of the controlled variables from the dynamic model and corresponding predetermined target values. For example, the objective function can include a term of the form:

$$\sum_{i=k+1}^{k+P} \left\| \boldsymbol{y}_{SP_i} - \boldsymbol{y}_i \right\|_{Q_{OUT_i}}^2$$

where y are dependent variables (output variables) of the state space model and $y_{sp}$ are the corresponding setpoints. The $Q_{OUT}$ diagonal matrix is a matrix comprising weights corresponding to each process output; that is, a non-zero diagonal element means the corresponding process output is considered as a controlled variable.

Instead or in addition to this, the objective function can comprise a term that penalises changes in the value of the one or more manipulated variables. For example, the objective function can include a term of the form:

$$\sum_{i=k}^{k+C-1} \left\| \Delta \boldsymbol{u}_i \right\|_{Q_{IN_i}}^2$$

where u is the manipulated variables (process inputs), i is an iteration, k is the current iteration and C is a control horizon. The diagonal matrix $Q_{IN}$ includes the corresponding weights for each process input.

Instead or in addition to this, the objective function can comprise a term that penalises differences between a feed flow rate and a target feed flow rate associated with a predetermined perfusion target perfusion rate. For example, the objective function can include a term of the form:

$$\sum_{i=k+1}^{k+P} \left\| F_{f_i} - y_{vol}^{SP} \times PR_i \right\|_{\Upsilon_{PR_i}}^2$$

where $PR_i$ is a desired perfusion rate. This term can be weighted by a weight $\Upsilon_{c_i}$ which is a weight for the term at iteration i.

Instead or in addition to this, the objective function can comprise a term that rewards maximisation of one or more predetermined variables. The term that rewards maximisation of one or more predetermined variables can be expressed as:

$$\Upsilon_{z_i} \sum_{i=k+1}^{k+P} \frac{1}{z_i^2+1}$$

where z is any predetermined variable to be maximized, and $\Upsilon_{z_i}$ is a weight set to a non-zero scalar value that sets the importance of the term relative to other terms of the objective function, k is a current time and k+P is a prediction horizon over which the dynamic model is evaluated.

The objective function can be a function as described in Equation (2) below. Identifying a value of one or more manipulated variables of the controlled bioprocess that optimises an objective function can be performed according to equation (1). Equation (1) integrates an objective function (i.e. an evaluated value that depends on state variables) as part of an optimisation function subject to constraints. The entire equation (1) can also be referred to as objective function, as it is the objective used by the controller, although strictly speaking the function that is evaluated is the ObjectiveFunction (such as e.g. as given in equation (2)).

**State Estimator**

Within the context of the present disclosure, a state estimator (also referred to herein as state observer) may be used to estimate the value of one or more variables of the bioprocess, such as the values of variables of a state space model used by a model predictive controller. A state observer is a model that provides an estimate of one or more internal states of a system (in the present case, the bioprocess), from measurements of inputs and outputs of the system (where the internal states and outputs may overlap, for example a substrate concentration may be both an internal state and an output of the system). State observers can be used to provide estimates of states in between at-line or off-line measurements. This allows the control action to be run at a higher frequency than the measurements of states. As another example, state observers can be used to combine multiple measurements of the same parameter. This may be useful when a continuous soft sensor is combined with a more precise infrequent off-line measurement (e.g. lab measurement). For instance, VCD may be estimated via an in-line conductivity or capacitance-based sensor but this measurement has a tendency to drift. VCD may also be measured once a day. A state observer can combine these two measurements in combination with the estimate from the state space model to provide an optimal estimate given the uncertainty in each source. Further, state observers are structured to take into account the relative uncertainty in measurements and the model to optimally handle noisy measurements. As yet another example, state observers can be adapted to handle cases where there is a delay between the time the sample is taken and the result is available. For example, when using a bioreactor with a sensor in the permeate line, rather than in the bioreactor itself, there is a distance of tubing outside of the bioreactor that the permeate needs to pass through before reaching the sensor. This results in a dead time until the permeate from the bioreactor reaches the sensor. When using state observers, this can be considered to update the measurement (e.g. substrate concentration) estimate used by the controller. For example, if there is a known 1 minute of flow time and a known 1 minute measurement time, a 2 minutes delay will be present on any measurement. This time delay can be taken into account in a state observer. For example, the predicted value of a measured variable at a current time k can be obtained based on a more recent measurement (corresponding to k-d, where d is a dead time / time delay) and a state space model that models the change of the measured variable over time. The time delay parameter in such a model may be provided by a user, for example depending on the configuration of the bioreactor system (including the bioreactor and sensor(s) used to provide the measurement, as well as possible physical connections between these) , or may be estimated for example by comparing predicted and measured values of the state space variable.

The state observer may be a linear state observer. The state observer may be a continuous time observer. A linear continuous time observer may obtain estimates for a linear system $\dot{x} = Ax + Bu$ and $y = Cx + Du$ (where x are the true internal states, u are inputs, y are outputs and A, B, C, D are coefficients of the model), as: $\hat{x} = A\hat{x} + Bu + L(y - \hat{y})$ and $\hat{y} = C\hat{x} + Du$ where $\hat{x}, \hat{y}$ are the estimates of the internal states and outputs in the observer, L is the observer gain, and the observer error $e = x - \hat{x}$ satisfies the equation $\dot{e} = (A - LC)e$. A state observer may be a Luenberg observer. A Luenberg observer uses a dynamic model of a physical system to estimate its internal states. A Luenberg observer is derived from a linear system assumed to satisfy equations (34a) and (34b):

$$X[k+1]=A*X[k]+ B\ U[k] \qquad (34a)$$

$$Y[k]=C*X[k]+ D\ U[k] \qquad (34b)$$

where at time k, X[k] are the internal states of the process (e.g. volume, concentration of one or more metabolites, and/or one or more cell densities including viable cell density), U[k] are the inputs of the process (e.g. manipulated variables such as feed, harvest and/or bleed flows), and Y[k] are the outputs of the process (e.g. volume, concentration of one or more metabolites, and/or viable cell density). The Luenberg observer is derived as:

$$\hat{x}(k + 1) = A\hat{x}(k) + L[y(k) - \hat{y}(k)] + Bu(k) \qquad (34c)$$

$$\hat{y}(k) = C\hat{x}(k) + Du(k) \qquad\qquad (34d)$$

where the variables denoted with a "^" are the variables of the observer, such that $\hat{y}(k)$ is the predicted output of the state observer and $y(k)$ is the measured output of the process.

A state observer may be a Kalman Filter (also known as linear quadratic estimation). A Kalman filter is a method to produce estimates of unknown variables using a dynamic model of a system, known inputs to the system, and measurements of states of the system observed over time, by estimating a joint probability distribution over the variables for each time. The method comprises providing estimate of the current state variables of a system and their uncertainties, and updating these estimates when a new measurement is obtained using a weighted average of the predicted states and the new measurement. The weights are determined to provide more weight to values with lower uncertainty than to values with higher uncertainty, and are calculated from the covariance. The updated estimate is then used to provide a prediction at a next iteration, which is itself updated when a new measurement is obtained. Thus, the method operates iteratively to provide improved estimates of a current state of the system. Kalman filters advantageously deal with uncertainty associated with noise in sensor data, providing better estimates of a system's internal state than can be obtained using sensor measurements alone.

A state observer may be a moving horizon estimation. A moving horizon estimation is a method to produce estimates of unknown variables using a dynamic model of a system, a set of measurements over a window of time (history of past measurements) and an optimization objective function over the estimation horizon. At every iteration (time t) where a new measurement is obtained, the dynamic model is integrated over a time horizon in the past ([t-T,t]) to identify state trajectories that minimize the optimization objective function over the time horizon. The last step of the estimation strategy is then used as an estimate of the current valuable of the variable that was measured. The calculation is then repeated at every iteration, shifting the horizon forward to the new time t. The optimization objective function may be defined as

$$J = \sum_{i=1}^{N} w_m\,(x_{p,i} - x_{m,i})^2 + \sum_{i=1}^{N} w_{\hat{x}}\,(x_{p,i} - \widehat{x_{p,i}})^2 + \sum_{i=1}^{N} w_{p_i}\,\Delta p_i^{\;2} \qquad (35)$$

where $x_{p,i}$ is the i$^{th}$ model predicted variable, $\widehat{x_{p,i}}$ are prior model predictions, $x_{m,i}$ is the ith measured variable, $p_i$ is an estimated parameter related to variable i, $w_m$ is a weighting coefficient reflecting the relative importance of measured values $x_{m,i}$, $w_{\hat{x}}$ is a weighting coefficient reflecting the relative importance of prior model predictions $\widehat{x_{p,i}}$, and $w_{p_i}$ is a weighting coefficient penalizing relative big changes in $p_i$. The optimization objective function can be defined as:

$$\min_{\{x_j,w_j\}_{j=k-N}^{k-1}} \sum_{j=k-N}^{k-1} \left\| w_j \right\|_{Q^{-1}}^2 + \sum_{j=k-N+1}^{k} \left\| v_j \right\|_{R^{-1}}^2 + \varphi_{k-N}$$

such that

$$x_{j+1} = f\!\left(x_j, \theta_j, u_j\right) + w_j\,; \qquad\qquad \forall j = k - N, \dots k - 1$$

$$y_j = h\!\left(x_j, \kappa_j, u_j\right) + v_j\,; \qquad\qquad \forall j = k - N + 1, \dots k \qquad\qquad (4)$$

$$q\!\left(x_j, u_j, y_j\right) \le 0; \qquad\qquad \forall j = k - N, \dots k$$

$$g\!\left(x_j, u_j, w_j, y_j\right) \le 0; \qquad\qquad \forall j = k - N, \dots k$$

$$x^l \le x_j \le x^u\,; \qquad\qquad \forall j = k - N, \quad k$$

where: $\quad w \in \mathbb{R}^{n_x}, v \in \mathbb{R}^{n_y}, Q \in \mathbb{R}^{n_x \times n_x}, R \in \mathbb{R}^{n_y \times n_y}$,

where $k$ represents the current time interval and index $j$ is a time index within the estimation horizon $N$. $Q$ and $R$ are the covariance matrices of process and measurement noises, respectively; *w, v, x, y,* and *u* represent vectors that describe the process noise, measurement noise, state variables, outputs, and inputs in the system, respectively. The penalty term $\varphi_{k-N}$ in Equation (4) provides the arrival cost estimated at time *k - N*, which is defined as follows:

$$\varphi_{k-N} = \left\| \boldsymbol{x}_{k-N} - \overline{\boldsymbol{x}}_{k-N} \right\|^{2}_{\boldsymbol{P}^{-1}_{k-N}} \tag{5}$$

where $\overline{\boldsymbol{x}}_{k-N}$ and $\boldsymbol{P}_{k-N}$ denote the expected value and covariance matrix of the approximated posterior distribution of the states at the time interval *k - N*. Note that *k - N* is the starting time point in the finite horizon window (start of the estimation horizon). The estimated states obtained from the MHE problem at the current time $(\hat{\boldsymbol{x}}_k)$ are used as the initial condition in the model predictive controller.

The time horizon may be set in terms of a number of iterations. As explained above, the time horizon may be set to have a length that is sufficiently long to capture complex dynamics that may be expected to occur. This is typically based on experience of the system at hand, and/or using simulations of the system to be controlled. As a safe and conservative selection, a prediction horizon length that includes the time to reach steady state may be used. The choice of a length of the prediction horizon may balance requirements to capture complex dynamics in the system and maintain a practically useful speed of operation of the control loop in view of the computational resources available for calculation. Instead or in addition to this, the length of the prediction horizon may be selected based on the reactor residence time. Methods for calculating an expected residence time or residence time distribution are known in the art. Instead or in addition to this, the length of the prediction horizon may be selected to be able to use the control algorithm as a safety measure, for example in case of a downtime or failure of a sensor. In such a case, the predicted values of one or more manipulated variables over the horizon could be used to control the process while problems with the sensor might be fixed. A moving horizon estimation may be advantageous when there are nonlinearities in the system that is modelled. Any optimization algorithm known in the art may be used to identify a solution that minimizes the objective function of the moving horizon estimation. When the moving horizon estimation uses a nonlinear dynamic model of the system, the optimization algorithm may be a nonlinear optimizer.
A state observer may additionally be used to update the value of one or more parameters (coefficients) of the state space model. This can be referred to as "augmentation", and essentially treats one or more parameters of the model as a state variable to be estimated by the state observer. For example, a state estimator can optionally perform an online parameter estimation to keep a key model parameters such as $\mu_{max}$ updated throughout the continuous bioprocess. Augmentation techniques treat the uncertain model parameters as state variables and perform an estimation over all the actual states and augmented states (uncertain model parameters), all at once. For example, a formulation of a MHE as described above that performs simultaneous state and parameter estimation can be used as follows:

$$\min_{\{\boldsymbol{x}_j, \breve{\boldsymbol{w}}_j\}^{k-1}_{j=k-N}} \sum_{j=k-N}^{k-1} \left\| \breve{\boldsymbol{w}}_j \right\|^{2}_{\breve{\boldsymbol{Q}}^{-1}} + \sum_{j=k-N+1}^{k} \left\| \boldsymbol{v}_j \right\|^{2}_{\boldsymbol{R}^{-1}} + \varphi_{k-N}$$

$$\tag{6}$$

such that:

$$\boldsymbol{x}_{j+1} = f\big(\boldsymbol{x}_j, (\boldsymbol{\theta}_j + \boldsymbol{\omega}_j), \boldsymbol{u}_j\big) + \boldsymbol{w}j ; \qquad \forall j = k - N, \quad k - 1$$

$$\boldsymbol{\theta}_{j+1} = \boldsymbol{\theta}_j + \boldsymbol{\omega}_j ; \qquad \forall j = k - N, \quad k - 1$$

$$\boldsymbol{y}_j = h\big(\boldsymbol{x}_j, \boldsymbol{\kappa}_j, \boldsymbol{u}_j\big) + \boldsymbol{v}_j ; \qquad \forall j = k - N + 1, \dots k$$

$$q\big(\boldsymbol{x}_j, \boldsymbol{u}_j, \boldsymbol{y}_j\big) \leq 0; \qquad \forall j = k - N, \dots k$$

$$g\big(\boldsymbol{x}_j, \boldsymbol{u}_j, \boldsymbol{w}_j, \boldsymbol{y}_j\big) \leq 0; \qquad \forall j = k - N, \dots k$$

$$x^l \leq x_j \leq x^u \; ; \qquad\qquad \forall j = k - N, \ldots k$$

$$\breve{w}_j = \left[ w_j, \omega_j \right]^T ; \qquad\qquad \forall j = k - N, \ldots k - 1$$

$$G = \begin{bmatrix} \dfrac{\partial f}{\partial w_{vcd}} & & \dfrac{\partial \theta_{k_{lac}}}{\partial w_{vcd}} \\ \vdots & \ddots & \vdots \\ \dfrac{\partial f}{\partial \omega_{k_{lac}}} & & \dfrac{\partial \theta_{k_{lac}}}{\partial \omega_{k_{lac}}} \end{bmatrix}$$

$$\breve{Q} = G^T . \overline{Q} . G$$

where

$$\omega = \left[ \omega_{u_{max}}, \omega_{k_l}, \omega_{u_d}, \omega_{k_t}, \omega_{k_b}, \omega_{k_p}, \omega_{k_{glu}}, \quad , \omega_{k_{lac}} \right]^T$$

$$\omega \in \mathbb{R}^{n_\theta}, \breve{w} \in \mathbb{R}^{n_x + n_\theta}, \widetilde{Q, G}, \overline{Q} \in \mathbb{R}^{(n_x + n_\theta) \times (n_x + n_\theta)}$$

where $n_\theta$ is the number of uncertain parameters that are going to be considered as augmented states, $G$ is the jacobian matrix that describes the correlation between the states (true states and the augmented states) and the uncertainties present in the augmented process model. That is, to capture the dynamic of the process with respect to the changes in the uncertain parameter, the corresponding Jacobian calculations in matrix $G$ are updated online, updating the changes in each state variable with respect to the parameter uncertainty (e.g., $\dfrac{\partial f}{\partial \omega_{u_{max}}}$) as well as changes in the model parameters with respect to the parameter uncertainty (e.g., $\dfrac{\partial \theta_{u_{max}}}{\partial \omega_{u_{max}}} = 1$ and $\dfrac{\partial \theta_{k_l}}{\partial \omega_{u_{max}}} = 0$).

The matrix $\overline{Q}$ represents the covariance matrix for the uncertainties assotiated with both states and augmented states (e.g., $w_{vcd}, \omega_{k_{lac}}$). Other terms have the same meaning as in relation to equation (4). When the parameter (*i.e.*, $\theta_m$) is certain (constant), the corresponding noise to that parameter is zero ($\theta_{j+1} = \theta_j$). For simplicity, the parameter estimation technique can assume that any uncertain parameter (augmented state) is associated with an uncertainty, where:

$$\frac{d\theta_m}{dt} = \omega_m; \qquad \forall m \in \left\{ u_{max}, k_l, u_d, k_t, k_b, k_p, k_{glu}, \ldots, k_{lac} \right\} \qquad (7)$$

**State Space Model**

A state space model is a model that provides a description of a process to be controlled. According to the present disclosure, a state space model is used in a model predictive controller, where it is used to generate a prediction of a system's internal state over a prediction horizon. A state space model is further used in embodiments of the disclosure in a state observer to estimate values of parameters that characterise the internal state of the system. The state space model may be a linear state space model or a linearised state space model. A linearised state space model is a model that comprises linear equations obtained as an approximation of the behaviour of a non-linear model around a stationary point (also referred to as "steady state").

In embodiments, the state space model comprises a kinetic growth model as described further below, such as e.g. equation (11), equations (11) to (14), or simplified versions thereof (see e.g. equation (26) below) expressed in matrix form as: $X[k+1]=A*X[k]+B\ U[k]$ where X are the states, such as e.g. $x_v$, $x_d$, $x_l$ (respectively the concentrations of live, dead and lysed cells), U are the inputs such as e.g. $F_b$, $F_h$ and $F_f$ (respectively the bleed, permeate and feed flow rates),

$$A = \begin{bmatrix} \mu_{eff} - \mu_d - \frac{F_b}{V} & 0 & 0 \\ \mu_d & -k_l - \frac{F_b}{V} & 0 \\ 0 & k_l & -\frac{F_h+F_b}{V} \end{bmatrix}$$

A is , B is

$$B = \begin{bmatrix} -\frac{x_v}{V} & 0 & 0 \\ -\frac{x_d}{V} & 0 & 0 \\ -\frac{x_l}{V} & -\frac{x_l}{V} & 0 \end{bmatrix}$$

for sufficiently small sampling time (note, these A and B can be converted for any sampling time using standard discretization formula - and the particular values of the matrices of coefficients A and B depend on the particular equations used for the dynamic model), and the effective growth, effective death and cell lysis rates can be estimated at every iteration of the control method by solving the model at pseudo steady state. Without wishing to be bound by theory, the inventors believe that the effective growth rate is more likely to significantly vary during a bioprocess, and is more directly related to the viable cell density that is controlled by the method, such that it may be more important to automatically update the estimate of the growth rate than that of the death and lysing rate. When using only equation (11) or a similar equation, the effective growth rate may capture contributions of both the growth and death processes, i.e. the effective growth rate may lump together the growth and death rates. Using a more complex predictive model such as one that models dead and lysed cells as well as live cells (e.g. a model that includes equations (11) to (14)) may strengthen the predictive power of the model.

For example, a growth model that explicitly models dead and lysed cells may be able to predict changes to growth kinetics due to changes in media exchange rate. By contrast, a model with a single equation that lumps together growth and death may only respond to changes in growth rate after the growth rate has already been impacted by a change in media exchange rate, through observation of model mismatch (where the state space model is then updated to account for the change in growth rate). Similarly, any change in a variable that is explicitly modelled (or that is directly -e.g. linearly-related to a variable that is explicitly modelled) in the model of equations (11) to (14) will have an impact on the cell related state variables (i.e. live, dead and/or lysed cell concentrations) and this will be captured by the model. By contrast, if a variable is not explicitly modelled in this way then its impact on the cell related state variables may only be captured through update of coefficient that effectively capture their effects (such as an effective growth rate) *a posteriori*. As another example, when using a predictive control to control the viable cell density, volume and a substate concentration, a state space model may comprise a matrix form of a system of equations comprising a kinetic growth model (e.g. equations (11), (11) to (14) or (26)), an equation describing volume change (e.g. equation (27)) and an equation describing the dynamics of the substrate concentration (e.g. equation (25) or (25b)). Thus, a state space model may comprise a kinetic growth model, as described further below. In embodiments, a state space model may comprise a model describing the growth kinetics of cell culture processes as described in WO 2021/165480. The parameters of such a model may be determined as described in WO 2021/165480 or WO 2021/164957, for example using a data driven approach such as a partial least square regression model or a machine learning model and/or a Bayesian method as described therein.

The term "kinetic growth model" refers to any model that captures the kinetic of a cell population in a bioprocess. A kinetic growth model can also be referred to herein as "cell culture model". Accordingly, a kinetic growth model may be used to model the number of live cells (and other culture-related parameters) in a bioreactor, for example to make predictions about the number of cells (e.g. viable cell density) in the bioreactor at a future point in time. For example, a kinetic growth model may include one or more differential equations that model the time-dependent behaviour of one or more cell population variables. Cell population variables may be variables that characterise the viable, dead, lysed and/or total cell population in a bioprocess. A kinetic growth model can include differential equations that capture the change in cell population variables including the viable cell density (VCD), dead cell density, and lysed cell density. In embodiments, a kinetic growth model uses the Monod equation to capture cell growth rates as a function of the concentration of a limiting nutrient. One example of a kinetic growth model is provided in equations (11) to (14) below, which describe respectively changes in the viable cell density $x_v$, the dead cell density $x_d$, the total cell density $x_t$ and the lysed cell density $x_l$ (concentration of lysed cells) as a function of time:

$$\frac{dx_v}{dt} = \left( \mu_{eff} - \mu_d - \frac{F_b}{V} - \frac{1}{V}\frac{dV}{dt} \right) x_v \qquad (11)$$

$$\frac{dx_d}{dt} = \mu_d x_v - \left( k_l + \frac{F_b}{V} + \frac{1}{V}\frac{dV}{dt} \right) x_d \qquad (12)$$

$$\frac{dx_l}{dt} = k_l x_d - \left( \frac{F_h+F_b}{V} + \frac{1}{V}\frac{dV}{dt} \right) x_l \qquad (13)$$

$$x_t = x_v + x_d + x_l \qquad (14)$$

where

$$\frac{dV}{dt} = F_f - (F_h + F_b) \qquad (14')$$

Note that any of the terms in equations (11)-(14) can be set to 0 depending on the circumstances (e.g. no cells leaving the bioreactor in bleed stream). For example, when no cells leave the bioreactor in the bleed stream, the terms $\frac{F_b}{V}$ can be removed (set to 0) and the term $\frac{F_h+F_b}{V}$ can be set to $\frac{F_h}{V}$ ($F_b$ set to 0). As another example, if the reactor volume is assumed to be constant, then equation (14') can be ignored and all terms $\frac{1}{V}\frac{dV}{dt}$ can be removed (set to 0).

In equations (11)-(14) (and (14') if used), $F_f$, $F_b$ and $F_h$ are the feed, bleed and harvest rates, respectively (see above and Figure 1), V is the reactor volume, $\mu_{eff}$, $\mu_d$, and $k_l$ are the effective growth, effective death, and lysing rates, respectively. Equation (11) captures the following assumptions: (i) live cells are formed from live cells at the effective growth rate $\mu_{eff}$ (see below for further detail on this term); (ii) live cells can exit the reactor through the bleed stream $F_b$; (iii) live cells can be transformed into dead cells at the effective death rate $\mu_d$; and (iv) no live cells exit the reactor through the harvest stream $F_h$ (in other words, where a harvest stream is present it includes a perfect cell retention filter - which may be a valid assumption when any cells exiting the bioreactor through the harvest stream represent a negligible amount). When no live cells exit the reactor through the bleed stream, equation (11) can also be expressed as $\frac{dx_v}{dt} = \left(\mu_{eff} - \mu_d\right)x_v$. Note that the above equations assume that the $F_h$ (harvest flow) is cell free, meaning that there are no viable and dead cells in the harvest flow.

In practice a separation system such as tangential flow filtration (TFF) or alternating tangential flow (ATF) are used to separate cells from spent media. The above model assumes an ideal separation filter where all metabolites and other biomaterials pass through and only viable and dead cells are retained.

Equation (12) captures the following assumptions: (i) dead cells are formed from live cells at the effective death rate $\mu_d$; (ii) dead cells are converted into lysed cells through a first-order process with rate $k_l$ (lysing rate); (iii) dead cells can exit the reactor through the bleed stream $F_b$ (if present); and (iv) no dead cells exit the reactor through the harvest stream $F_h$. The calculation of the effective death rate is presented in detail below. When no dead cells exit the reactor through the bleed stream, equation (12) can also be expressed as $\frac{dx_d}{dt} = \mu_d x_v - k_l x_d$.

Equation (13) captures the following assumptions: (i) lysed cells are formed from dead cells at rate $k_l$; (ii) lysed cells can exit the reactor through the bleed stream $F_b$ (if present); and (iv) lysed cells can exit the reactor through the harvest stream $F_h$ (if present). Equation (14) captures the assumption that cells are either viable, dead or lysed. This can be used to calculate one of the variables from the others, for example lysed cells (which typically cannot be measured directly), by closing the balance on living and dead cells. When no lysed cells exit the reactor through the bleed stream, equation (13) can also be expressed as $\frac{dx_l}{dt} = k_l x_d$.

In embodiments, the death process (captured through the parameter $\mu_d$, referred to as the effective death rate) can be modelled as resulting from a combination of a base death rate ($k_d$ or $k_{death}$) and a toxicity factor, for example as provided in equation (15) below:

$$\mu_d = k_d + k_t \phi_t \qquad (15)$$

In equation (15), $k_d$ is the primary (base) death rate, $k_t$ is the "toxicity rate" and $\phi_t$ is a measure of toxicity. The variable $\phi_t$ may represent the concentration of one or more components that have a toxic effect on cells. The motivation for including this factor is that as cell growth progresses, the byproducts of cell growth create a toxic environment which increases the death rate of the cells. The toxicity of the spent media is influenced by many factors. In embodiments, $\phi_t$ is assumed to be equal to the concentration of lysed cells $x_l$ (i.e. $\phi_t = x_l$) - such that equation (15) can be expressed as $\mu_d = k_d + k_t x_l$. This can be used as a default option. In other embodiments, $\phi_t$ is assumed to be equal to the concentration of an unknown biomaterial that accumulates in the culture as a result of cell growth, which is designated as $\phi_b$. This biomaterial can be toxic to the cells, such that $\phi_t = \phi_b$ in some embodiments. In embodiments, this biomaterial can also inhibit cell growth, and therefore can also have an effect on the effective growth rate as will be explained further below.

Thus, in embodiments the model additionally includes an equation that captures the concentration of unmeasured (which can be unspecified and can in practice encompass multiple compounds) biomaterials produced by the cells. The evolution of this variable can be captured for example using equation (16) below:

$$\frac{d\emptyset_b}{dt} = k_{inh}x_v - \left(\frac{F_h+F_b}{V} + \frac{1}{V}\frac{dV}{dt}\right)\emptyset_b \qquad (16)$$

which assumes that the unknown product is produced at a rate that is proportional to the viable cell density $x_v$ with a proportionality factor $k_{inh}$ (effective rate of production of the inhibitory product by live cells) and that the product can exit the bioreactor through the bleed and harvest streams ($F_b$, $F_h$). When the product does not leave the bioreactor through the bleed and harvest stream (e.g. it is directly associated with the cells and there is no bleed stream), equation (16) can be expressed as $\frac{d\emptyset_b}{dt} = k_{inh}x_v$. Further, when the volume of the bioreactor can be assumed to be constant, the term $\frac{1}{V}\frac{dV}{dt}$ can be removed (set to 0). In embodiments, $k_{inh}$ is set to 1 (i.e. removed from equation (16) and $\emptyset_b$ is interpreted as the relative concentration of the unmeasured biomaterials, which are assumed to be produced by the live cells at a constant rate. Including equation (16) (or simplified versions thereof depending on the configuration) enables to model the impact of accumulation on growth dynamics.

In embodiments, the model additionally includes an equation that captures the presence of antifoam in the bioreactor:

$$\frac{dA}{dt} = -\left(\frac{S_A F_h+F_b}{V} + \frac{1}{V}\frac{dV}{dt}\right)A \qquad (32)$$

where A is the antifoam concentration, $S_A$ is the antifoam sieving coefficient (default=0, meaning that no antifoam passes through the cell retention device). This equation assumes that antifoam is added through bolus additions rather than as part of the feed media. Equivalents equations assuming that the antifoam is added in the feed can be constructed using the same principles.

In embodiments, a fraction of the biomaterials $\emptyset_b$ and/or the lysed cells $x_l$ may not pass through the cell retention device. If this is the case values for biomaterial_holdup and lysed_holdup may be included in the growth_model to account for this. The states for biomaterial and lysed cells are then split into two separate states, one which represents the fraction that freely passes through the cell retention device and the other that does not pass through as in the following, where $h_l$ and $h_b$ are parameters representing the fraction of the lysed cells and the biomaterials, respectively, that is held up by the cell retention device:

$$\frac{dx_l}{dt} = (1-h_l)k_l x_d - \left(\frac{F_h+F_b}{V} + \frac{1}{V}\frac{dV}{dt}\right)x_l \qquad (13a)$$

$$\frac{dx_{l,h}}{dt} = h_l k_l x_d - \left(\frac{F_b}{V} + \frac{1}{V}\frac{dV}{dt}\right)x_l \qquad (13b)$$

$$\frac{d\emptyset_b}{dt} = (1-h_b)k_{inh}x_v - \left(\frac{F_h+F_b}{V} + \frac{1}{V}\frac{dV}{dt}\right)\emptyset_b \qquad (16a)$$

$$\frac{d\emptyset_b}{dt} = h_b k_{inh}x_v - \left(\frac{F_b}{V} + \frac{1}{V}\frac{dV}{dt}\right)\emptyset_b \qquad (16b)$$

Thus, references herein to the use of equations (13) and/or (16) also encompass use of equations (13a), (13b) and/or (16a), (16b), for example in embodiments where biomaterials holdup and/or lysed cells holdup is expected.

In embodiments, the growth process (captured through the parameter $\mu_{eff}$) is modelled as the product of a growth rate under ideal conditions $\mu_{max}$ (where growth rate is assumed to be maximised), and one or more factors that describe the influence of other system variables on growth. These factors may in some cases take one of three functional forms: substrate limited $\eta_s$, quadratic $\eta_q$ or inhibitory $\eta_i$. The relationship between these factors and the resulting effective growth rate can be captured in equation (17):

$$\mu_{eff} = \mu_{max}\eta_s\eta_q\eta_i \qquad (17)$$

where the correcting factor $\eta_s$ captures the contribution of $N_s$ substrate limiting variables, $\eta_q$ captures the contribution of $N_q$ quadratic influencing variables and $\eta_i$ captures the contribution of $N_i$ inhibitory variables. $N_s$ can be equal to 0 (no substrate limiting variable - equivalent to setting $\eta_s = 1$), 1 (one substrate limiting variable), or any natural number. $N_q$ can be equal to 0 (no quadratic influencing variable, equivalent to setting $\eta_q = 1$), 1 (one quadratic influencing variable), or any natural number. $N_i$ can be equal to 0 (no inhibitory variable, equivalent to setting $\eta_i = 1$), 1 (one inhibitory variable), or any natural number. Note that instead or in addition to $\eta_q$, equation (17) can include a function that captures the effect of physicochemical operating parameters on cell growth (such as e.g. pH, T, osmolality, etc.). The correction factors above can be calculated as products of a plurality of correcting factors that each capture the contribution of a substrate limiting / quadratic influencing / inhibitory variable, as shown in equations (18)-(20) below:

$$\eta_s = \prod_{n=1}^{N_s}\left(\eta_{s,n}\right) \qquad (18)$$

$$\eta_q = \prod_{n=1}^{N_q}\left(\eta_{q,n}\right) \qquad (19)$$

$$\eta_i = \prod_{n=1}^{N_i}\left(\eta_{i,n}\right) \qquad (20)$$

where $\eta_{s,n}$ is the contribution of the nth substrate variable, $\eta_{q,n}$ is the contribution of the nth quadratic variable, and $\eta_{i,n}$ is the contribution of the nth inhibitor variable.

In embodiments, inhibitory effects are captured using correction factors of the form provided by equation (21) below:

$$\eta_{i,n} = \frac{1}{\left(\frac{z_n}{2*\theta_{i,n}}\right)^3 + 1} \qquad (21)$$

where $z_n$ is the concentration of a substance that has a growth inhibitory effect, and $\theta_{i,n}$ is a parameter that represents the level of $z_n$ above which inhibition occurs. This is illustrated on **Figure 8D.** The variable $\phi_t$ in equations (15) and (16) above may be seen as a special case of an inhibitory effect where the concentration of the inhibitory substance depends on the viable cell density (e.g. because the inhibitory substance is produced by or as a result of the presence of the cells). The variable $\phi_t$ can also sometimes be modelled using a formulation as provided in equation (21). Therefore, equation (17) can in some embodiments be written as:

$$\mu_{eff} = \mu_{max}\eta_s\eta_q\eta_i \frac{1}{\left(\frac{\phi_t}{2*\theta_t}\right)^3 + 1} \qquad (22)$$

where $\theta_t$ (or $\theta_b$ as the case may be) is a coefficient representing the inhibition in growth from accumulation of the biomaterial $\phi_t$ (where $\phi_t$ can be e.g. equal to $x_l$ or $\phi_b$). As the skilled person understands, in some embodiments there may be more than one substance $\phi_b$, each of which can be modelled using a respective term in equations (17) and (22) and a respective equation (16). Examples of substances that have a growth inhibitory effect and that produced by or due to the presence of the cells in the culture (i.e. substances $\phi_b$) include toxic by-products such as e.g. ammonia. Examples of substances that have a growth inhibitory effect that is not necessarily related to the viable cell density include substances that are included in the culture medium to have a desired effect but that may also have a (ideally slight) growth inhibitory effect on the cell culture (e.g. antibiotics). Therefore, in embodiments an effective growth rate can be represented as:

$$\mu_{eff} = \mu_{max} \frac{1}{\left(\frac{\phi_{b,T}}{2*\theta_{\phi_b}}\right)^3 + 1} \qquad (22')$$

where $\theta_{\phi_b}$ represents the level at which $\phi_{b,T}$ begins inhibiting growth, and $\phi_{b,T}$ is the total biomaterial expressed as $\phi_{b,T} = \phi_b + \phi_{b,h}$ (where holdup is present, equivalent to $\phi_b$ when no holdup is present).

In embodiments, substrate limiting effects are captured using correction factors of the form provided by equation (23) below:

$$\eta_{s,n} = tanh\left(2\frac{z_n}{\theta_{s,n}}\right)^2 \quad (23)$$

where $\theta_{s,n}$ is a parameter which represents the approximate level below which the variable $z_n$ (substrate that is growth limiting) starts to have a limiting effect on growth. This is illustrated on **Figure 8A.** In equation (23) the factor 2 is used to give the parameter $\theta_{s,n}$ an intuitive biological meaning as the approximate concentration $z_n$ below which a limiting effect can be seen (such as e.g. a value such that the inhibitory function crosses the -0.95 threshold when the value of $z_n$ falls below that of $\theta_{s,n}$). Any other value can be used for this factor, resulting in the same behavior with the effect that the parameter $\theta_{s,n}$ is adjusted accordingly and no longer has the same intuitive interpretation. In particular, this factor can be omitted entirely (i.e. set to 1). Similarly, in equations (21) and (22), the term that is cubed (i.e. $(z_n/\theta_{i,n})$) may comprise a coefficient that gives the parameter $\theta_{i,n}$ an intuitive biological meaning as the approximate concentration $z_n$ above which a limiting effect can be seen (such as e.g. a value such that the inhibitory function crosses the -0.95 threshold when the value of $z_n$ reaches that of $\theta_{i,n}$, for example 0.37). Examples of substances that have a substrate limiting effect include nutrients such as glucose, amino acids, etc.

In embodiments, quadratic effects are captured using correction factors of the form provided by equation (24) below:

$$\eta_{q,n} = exp\left[-\frac{1}{25}\left(\frac{z_n - \mu_{q,n}}{\theta_{q,n}}\right)^2\right] \quad (24)$$

where $\mu_{q,n}$ is a parameter that represents the target value (i.e. the value at which maximum growth occurs) and $\theta_{q,n}$ is a parameter that represents the "spread"' of the effect. This is illustrated on **Figures 8B** and **8C.** The factor 1/25 used to give the parameter $\theta_{q,n}$ an intuitive meaning where a value of $\theta_{q,n}$ = 1 means that the quadratic effect crosses a 95% threshold at $\pm 1$ around the target value $\mu_{q,n}$. Any other value can be used for this factor, resulting in the same behavior with the effect that the parameter $\theta_{q,n}$ is adjusted accordingly and no longer has the same intuitive interpretation. In particular, this factor can be omitted entirely (i.e. set to 1). The use of factors that provide an intuitive interpretation of parameters such as $\theta_{s,n}$ and $\theta_{q,n}$ may be advantageous in that it may make it easier for a user to set these parameters (using e.g. biological knowledge or assumptions) or realistic boundaries for these parameters.

The formulation of the kinetic growth model described above provides a generalized model that can be trained from data and can be calibrated for new cell lines, or even culture types (e-coli, CHO, etc.) for providing simulation capabilities appropriate for optimization and system configuration decisions within biopharmaceutical process development and advanced control activities. The model aims to replicate the cell culture response to changes in media composition, process conditions and media exchange rather than to identify accurate kinetic parameters. As an example, lysed cells are included as a state in the model and are used to influence the toxicity of the spent media. Validation of the model fit is focused on replicating growth and death dynamics of the measured viable cell density (VCD) and viability rather than the true kinetic coefficients for the lysing rate of dead cells and toxicity of accumulated lysed cells. The model structure additional aims to achieve good identifiability and maximize extrapolation capabilities beyond the knowledge space of the training data including transferability between batch, fed-batch and media exchange. Thus, models as described above identified from batch or fed-batch can be used to forecast cell line performance for intensified growth or perfusion operating modes. Furthermore, the model can use a single set of kinetic coefficients representing all phases of a batch or fed-batch process including exponential growth, stationary and death phases as well as complex processes such as perfusion where there the operating mode transitions from an initial intensified growth phase followed by a pseudo steady-state perfusion phase. This contrasts with approaches that identify separate kinetic parameters as the cell culture progresses through different phases or operating modes or use of data driven methods such as physics informed neural-network where growth and death kinetics are determined based on process states or conditions. The motivation behind a single set of kinetic coefficients is to define a model structure that is comprehensive enough to explain variation in growth and death dynamics as biomaterials accumulate in spent media to enable transfer between batch, fed-batch and media exchange operating modes. However, as described further herein, the values of any of the coefficients of the models (i.e. any kinetic coefficients) can be updated during operation of a process based on measurements of the process.

In embodiments, such as when using the above cell culture model (CCM), the kinetic growth model consists of sets of ordinary differential equations (ODE) to describe dynamic material balances of viable, dead and lysed cells and various other biomaterials. It is not intended that concentration of lysed cells is measured. Tracking the material balance of viable and dead cells gives an indication of total cells generated, and by extension the number of cells that have lysed and are no longer detectable. In a CCM that models lysed cell concentration as described above, lysed cell concentration is used a metric to indicate the level of toxicity of the spent media influencing growth and/or death rates. Biomaterials such as substrates, amino acids and metabolic byproducts that are measured are defined as metabolites. In embodiments, such as the CCM described above, metabolites are included as dynamic states with various ways to represent their specific consumption or production rates. An additional unmeasured biomaterial is included representing a bulk set of metabolic byproducts that is generally used to model growth inhibition of accumulated byproducts. Variables such as temperature, pH, feed composition, feed rates and media exchange rates are treated as independent variables and defined as inputs. As such, there is no thermodynamic model to represent changes in temperature or models of aeration, base addition and other influences of pH. It is assumed the regulatory control layer provides a controllable process that maintains inputs at their setpoints. The above model removes scale and hardware dependent dynamics, producing a scale independent model.

As the skilled person understands, other equations can be used instead or in addition to the above to model cell population variables and factors that affect these variables. These equations together may form a state space model in that they describe the evolution of the state of the cell culture (also referred to herein as "cell state") in terms of a series of state variables $x_v$ , $x_d$ , $x_t$, $x_l$ as a function of inputs to the system including e.g. the concentration of various substances that influence the cell state.

Thus, a state space model may comprise a matrix form of a system of equations that comprises a kinetic growth model and/or one or more equations that capture the evolution of the concentration of one or more metabolites in the bulk culture (including in particular one or more nutrients/substrates, by-products and/or the desired product), as described below (such a e.g. equation (25)). The flows of metabolite may be expressed as mass flows or molar flows (as the latter can be converted to the former and vice-versa using molar mass, such that the conservation of mass expressed in equation (25) is verified regardless of the units chosen), and the skilled person would be able to convert one into the other. As such, references to mass flows are intended to encompass the use of the corresponding molar flows with corresponding adjustments for consistency of units within an equation. Similarly, references to concentrations may refer to the mass or molar concentrations. The flow of a metabolite into the bioreactor depends on the value of the feed flow $F_F$ and the concentration of the metabolite in this flow. The flow of the metabolite out of the bioreactor depends on the value of the permeate flow $F_H$ and the value of the bleed flow $F_B$, and the concentration of the metabolite in these respective flows. The consumption and secretion of a metabolite by the cells in the bioreactor depends on the viable cell density in the reactor and on a variable called the "specific transport rate" (sometimes also referred to as "metabolic rate"), which can also be referred to as ""specific consumption rate" (typically, if it is negative, i.e. the metabolite is being consumed by the cells) or the "specific secretion/production rate" (typically, if it is positive, i.e. the metabolite is being produced by the cells) of the metabolite by the cells. Therefore, the material balance for a metabolite i can be written for a general system (e.g. as illustrated on **Figure** 1), as equation (25) below:

$$\frac{d\,(m_i)}{dt} \quad = \quad \frac{F_F}{V} * m_{F,i} \quad - \quad \frac{F_H}{V} * m_{H,i} \quad - \quad \frac{F_B}{V} * m_{B,i} \quad + \quad \delta_{m,i}(t) * x_v \quad (25)$$

which expresses the conservation of mass in the system at any time point t. In equation (25), $\delta_{m,i}(t)$ is the specific transport rate of metabolite i by the cells in the culture (time-varying specific consumption or secretion rate of the metabolite), $m_i$ is the concentration of metabolite i in the reactor, $V$ is the volume of the culture in the bioreactor, $m_{F,i}$ is the concentration of metabolite i in the feed flow, $m_{H,i}$ is the concentration of metabolite i in the permeate flow, $m_{B,i}$ is the concentration of metabolite i in the bleed flow, $x_v$ is the viable cell density in the reactor, and $F_F$, $F_H$ and $F_B$ are the volumetric feed, harvest and bleed flow rates, respectively (although mass flow rates can equally be used with the appropriate coefficients for densities of the respective flows). In embodiments, the term $\delta_{m,i} * x_v$ can be replaced by a term $\delta_{m,i} * x_v * (\frac{m_i}{m_i + \varepsilon})$ where $\varepsilon$ is a constant that is chosen to ensure that the values of $m_i$ that are below the detection limit for the metabolite do not cause errors in the estimation of specific transport rates. Such embodiments are considered encompassed when referring to the use of equation (25). Typically, $\varepsilon$ is chosen to be approximately equal to the detection limit for the metabolite (e.g. it can be chosen as 0.05 where the metabolite concentration is standardised). Equation (25) assumes that the permeate flow 128A contains the only material leaving the system through the auxiliary harvest flow 128C (i.e. the auxiliary harvest flow and the return flow do not need to be included in the model as the metabolite only leaves the system through the permeate flow), and that the cell retention device 128 functions such that the permeate flow 128A can be assumed to contain no cells. In embodiments, $m_{H,i}$ and $m_{B,i}$ are considered to be the same and equal to $m_i$ (see equation 25a below). Equation (25) can be adapted to include an auxiliary harvest flow 128C (and corresponding $m_{A,i}$ and density $\rho_A$ if a mass flow rate is used) and a return flow 128B (and corresponding $m_{R,i}$ and $\rho_R$). Further, equation (25) can be amended to model the removal of some cells through the permeate flow. In other words, additional terms can be added to equation (25) and some can be removed depending on the set-up of the bioprocess and the assumptions made. Equation (25) (referred to herein as "material balance model" or "bulk metabolite concentration model") captures the relationships between extracellular fluxes of metabolites and the concentration of the metabolites in the culture medium. The model does model intracellular metabolic fluxes, which may be captured using metabolic models that model fluxes along reactions that together form the cell's metabolic network, and which are significantly more complex (and thus more difficult to solve and requiring extensive data and/or assumptions to parameterise). As such the models used herein advantageously provide information about the metabolic state of the cells while remaining relatively simple to solve and parameterised, allowing to obtain important system state variables such as the specific transport rates of metabolites (as will be explained further below) in a simple and efficient manner.

Further, equation (25) can be simplified by making a few assumptions. For example, assuming that the metabolite concentration is the same everywhere in the culture medium in the bioreactor, and hence also in the permeate flow and in the bleed flow (in other words, assuming that concentration gradients within the reactor are negligible such that $m_{B,i} = m_{H,i} = m_i$), and that the number of cells lost in the bleed and permeate flows is negligible, equation (25) can be written as:

$$\frac{d\,(m_i)}{dt} \quad = \quad \frac{F_F}{V}*m_{F,i} \quad - \quad \frac{(F_H+F_B)}{V}*m_i \quad + \quad \delta_{m,i}*(x_v) \quad (25a)$$

Assuming further that the volume of the culture is constant (i.e. $F_F = F_H + F_B$), that the flows are constant, and using a first-order finite difference approximation for the derivative, equation (25a) can be resolved for the metabolite specific consumption/secretion rate at time $t_k$ as:

$$\delta_{m,i}(t_k) = \frac{V*(m_{i,k+1}- m_{i,k})- F_F*m_{F,i,k}*(t_{k+1}-t_k)+F_F*m_{i,k}*(t_{k+1}-t_k)}{V*IVCD_k} \quad (26a).$$

In embodiments (such as e.g. as used in the examples below), the specific consumption rates are estimated (e.g. over a prediction horizon) using a regression model (e.g. machine learning model, partial least square model, etc), as will be explained further below.

Assuming that the volume of the culture is constant (such that Ff=Fb+Fh), equation (25a) can be re-written as equation (25b):

$$\frac{d\,(m_i)}{dt} \quad = \quad \frac{F_F}{V}*(m_{F,i} \quad - \quad m_i)+ \quad \delta_{m,i}*(x_v) \quad (25b)$$

For example, a state space model may include an equation capturing the change in concentration of a metabolite (e.g. a substrate), such as equation (25), (25a) or (25b), and one or more equations capturing the change in cell density, such as equation (11) or equations (11) to (14). Reference to the use of equation (25) encompasses the use of simplified equations (25a) or (25b). In such embodiments, the substrate concentration and viable cell density may be measured at every iteration and this may be used by a state observer to obtain estimates of the substrate concentration and viable cell density as well as estimates of the cell specific uptake rate and effective growth rate by integration of the state space model. These estimates may be used by a model predictive controller to identify a control strategy to jointly control the viable cell density and substrate concentration. In embodiments, specific consumption rate for observation k is calculated using the material balance of accounting for the mass of metabolite added from the feed, the mass removed from harvest and bleed streams and the change in metabolite concentration. Note that the consumption rate at the k^th observation is forward looking, meaning that it represents the consumption rate for the time interval k→k+1. For example, $\delta_{m,i}$ at observation k can be calculated as:

$$\delta_{m,i|k} = \frac{1}{ix_{v|k}}*\left[\left(\overline{F_{f|k}}*m_{i,f} - \left(\overline{F_{b|k}}+\overline{F_{h|k}}\right)*\overline{m_{i|k}}\right)*\Delta t_{|k} - V_{|k}*(m_{i|k+1}-m_{i|k})\right] \quad (33)$$

where $ix_{v|k}$ is an integrated viable cell density calculated over the k to k+1 interval, with $\Delta t_{|k} = t_{k+1} - t_k$, $\overline{F_{\alpha|k}} = \frac{F_{\alpha|k}+F_{\alpha|k+1}}{2}$ where $\alpha$ is b, h or f, i.e. the terms in equation (33) are the average flow rates of the bleed, harvest and feed streams over the k to k+1 interval. A positive $\delta_{m,i}$ represents a consumption of the metabolite and a negative value represents secretion. Average specific consumption rate trajectories can be calculated for each metabolite by taking the average trajectory of all batches in a training set data. Observation times (maturities) are not uniform between batches complicating this calculation. Batch modelling methods as known in the art and as implemented in e.g. SIMCA® from Sartorius Stedim Data Analytics can be used to generate a representative maturity vector and calculate an average. A "batch evolution models" (BEM) describe the time-series evolution of process conditions, referred to as the process 'path'. Process paths are obtained by fitting an (O)PLS (orthogonal partial least square) model (expressed as $X=TP^t+E$ and $Y=UQ^t+F$ where X is a matrix of predictors, Y is a matrx of responses, T and U are X scores that are projections of X onto a new space of latent variables and projections of Y onto a new space, P and Q are orthogonal loading matrices that define the new spaces, and E and F are error terms) where $X$ includes the one or more process variables that are believed to be of potential relevance, measured at multiple times (maturity values) over the evolution of the process, and $Y$ includes the corresponding maturity values. For example, a set of $n$ process variables may have been measured at $m$ maturity values, and these $nxm$ values may be included as a coefficient in the matrix $X$. The corresponding matrix $Y$ is an $mx1$ matrix (i.e. a vector of length $m$) of maturity values. The $T$ matrix therefore comprises scores values for each of the $m$ maturity values and each of $l$ identified latent variables that describe the aspects of the process variables that are most correlated with maturity. this can be used to align measurements to a common set of maturities.

Any methods to calculate an integrated viable cell density may be used in the methods described herein. For example, $IVCD_k$ may be calculated using equation (27):

$$IVCD_k = \left(\alpha\, x_{v|k} + \beta\, x_{v|k+1}\right) * \left(t_{k+1} - t_k\right) \qquad (27)$$

where the coefficients $\alpha$ and $\beta$ weight the relative influence of the two viable cell density values and are such that $\alpha + \beta = 1$. For example, the two values may be weighted identically, i.e. $\alpha = \beta = 0.5$. In embodiments, $\alpha$ and $\beta$ are chosen such that $\alpha > \beta$ (for example $\alpha=0.6$ and $\beta=0.4$). These coefficients may be chosen to reflect the observed cell growth behaviour, and may be chosen independently for each time point. Where exponential growth can be assumed, the integrated viable cell density can be calculated using a log transform. For example, the integral cell density may be calculated using equation (27a):

$$IVCD_k = x_{v|k} * e^{u_k(t_{k+1}-t_k)} \qquad (27a)$$

where

$$\mu_k = \frac{ln\left(x_{v|k+1}/x_{v|k}\right)}{t_{k+1}-t_k} \qquad (27b)$$

The equations for $\delta_{m,i}$ at time k as described above can be solved using the known (typically measured) biomass and metabolite concentrations at times k and k+1 to obtain a metabolite transport rate at every time point value where the above measurements are available. Further, this can be performed individually for each measured metabolite. The resulting metabolite transport rates are expressed as an amount of metabolite (mass or moles) per cell per unit of maturity (i.e. typically per unit of time). Note that in equations for specific transport rates, the signs of all the terms can be inverted depending on whether a negative rate is taken to mean that the cells consume the metabolite and a positive rate is taken to mean that the cells produce the metabolite (i.e. the rate is seen from the perspective of the culture medium), or the opposite (i.e. a positive rate means that the cells consume the metabolite and a negative rate means that the cells produce the metabolite, in other words the rate is seen from the perspective of the cells compartment).

In embodiments, a product of the bioprocess can be modelled separately, in a similar way to metabolites (since the product is expected to be a material that is secreted from the cells). However, compared to the metabolite equation (25), the product is assumed to not be present in the feed. Thus, a special form of equation (25) can be used for a product:

$$\frac{d\,\varphi}{dt} = u_{inf} * k_\varphi * x_v - \left(\frac{S_\varphi * F_h + F_b}{V} + \frac{1}{V}\frac{dV}{dt}\right) * \varphi \qquad (34)$$

where $\varphi$ is the product concentration, $k_\varphi$ is the specific productivity for the product, and $S_\varphi$ is the product sieving coefficient. This can be set to a default value of 1, meaning that the product freely flows through the cell retention device. The parameter $u_{inf}$ is optional and can be removed (set to 1) for any product that is not a virus. When the product is a virus harvested from the cells or viral particles in the spent media, the product is not produced until after infection. The parameter $u_{inf}$ enables to model this by setting this parameter to 0 before the virus has been introduced and 1 after the virus has been inoculated. As a result, the model accounts for production of the virus only after the virus is introduced in the cells.

In embodiments, data driven models are used to describe metabolic function, to provide modelling of productivity, quality and any other behaviour that is not captured in the growth mode kinetic equations above. Metabolic models can be based on the correlation structure of the metabolite specific consumption rates ($\delta_{m,i}$) and can have any structure that can be expressed in the general form $\gamma = f(\delta, u, m, s)$ where the output of the function (y) is a specific productivity ($k_\varphi$), consumption rate ($\delta_{m,i}$), lysis rate ($k_l$), growth rate ($\mu_{eff}$) and/or death rate ($\mu_d$), and the inputs of the function are a set of process conditions (u, such as e.g. any measured process parameter such as as temperature, pH, osmolality, dissolved oxygen and $CO_2$), metabolite concentration in the bulk fluid (m), and system states from the growth model (s, such as e.g. live cell density, dead cell density, lysed cell density). For example, function f can take the form of a regular regression, machine learning (e.g. any regression machine learning model including but not limited to random forest regression, decision tree regression, artificial neural networks, and support vector regression), PLS or OPLS. For example, any of the methods described in WO 2021/164957 may be used to estimate any of the above parameters (e.g. any of the metabolite specific consumption rates, productivity rates, etc.). In embodiments, the function f is a PLS (partial least square) model. In embodiments, estimation of the consumption rates included in the kinetic growth model over the prediction horizon of the controller is provided by a machine learning model (or any other regression method, such as a PLS model). The model or the predictions may also be updated based on the consumption rate observed from process measurements.

Model identification is the process of finding the set of state space coefficients that produce a growth trajectory that match the measured experimental data. In embodiments, some parameters of the model are identified using measured data, for example using a stochastic optimization algorithm called particle swarm optimization (PSO) as described in Adams and Warren (2008). This is a global optimization method that has proven to be effective at identification of the CCM growth kinetic parameters. The number of iterations and step size for this optimisation method can be selected by user input. For example, a two step procedure can be used where a relatively large step size is first used to explore the parameter space to find the general location of the global minima followed by a second with a reduced step size to hone in on the optimal set. This can be used in combination with machine learning, for example where machine learning is used to identify specific consumption rates and/or productivity rates, and an optimisation algorithm is used together with measured data to identify growth kinetic parameters such as effective growth and death rates.

A state space model may be a self-adapting state space model. A self-adapting state space model refers to a state space model where one or more coefficients in the model are updated during operation of the bioprocess based on one or more measurements of state space variables collected during operation of the process. This is by contrast to e.g. simply using a constant value for these coefficients throughout operation of the bioprocess, or even using a different value for these coefficients as provided by a user for different iterations of the control loop. The use of automatically updated coefficients in the state space model further improves the stability and reliability of the model predictive control as the model more accurately reflects the current state of the bioprocess. This is particularly important in the context of a perfusion bioprocess where cells may be maintained in culture during prolonged periods of time, as the metabolism of the cells themselves may vary in that time (resulting in e.g. different growth rates, different substrate consumption rates, etc.), as well as relevant characteristics of the bioreactor system (such as e.g. performance of the filtration system for the permeate). For example, the state space model may include kinetic coefficients representing a specific growth rate and/or a specific substrate consumption rate. The control method may compare the predicted value of the VCD and/or substrate concentration to measurements collected from the process. Deviations between the predicted and measured values may be used to generate optimal updates to predicted values given measurement noise and model uncertainty, for example using a state observer (e.g. a Kalman Filter, as described above). Instead or in addition to this, deviations between the predicted and measured values may be used to update kinetic coefficients in the state space model to continuously improve predictions of future process states. The term "process states" may refer to the states defined as states in the states space model. Thus, the state space models described above may be updated at regular intervals, such as e.g. every iteration, every x iterations, or every iteration of the control loop that satisfies one or more criteria (e.g. every iteration that satisfies the criterion of having a more accurate (compared to measurements obtained at all iterations), measurement for one or more variables of the state space model, such as e.g. an off-line measurement). Updating the state space model may comprise updating the value of one or more coefficients of the model, such as e.g. one or more kinetic coefficients. This may be achieved by considering the coefficients of the model to form part of the state space variables to be estimated at each iteration where the model is to be updated. In other words, instead of being fixed to predetermined values provided by a user, one or more coefficients of the state space model may be considered to represent state space variables that are estimated by the state observer. The update values for these coefficients are then used by the model predictive controller to predict the trajectory of the process in the prediction horizon, and hence determine an optimal control strategy. For example, in the model of equations (25)/(25a), the value of $\delta_{m,i}$ may be updated based on a comparison between a predicted and measured value of the metabolite concentration $m_i$. As another example, in the model of equations (11) to (14), the value of one or more of the effective growth, effective death, and lysing rates ($\mu_{eff}$, $\mu_d$, and $k_l$), may be updated based on a comparison between a predicted and measured value of the viable cell density $x_v$, lysed cell density $x_l$ and dead cell density $x_d$, if available. In embodiments, in the model of equations (11) to (14), the value of the effective growth rate ($\mu_{eff}$) may be updated based on a comparison between a predicted and measured value of the viable cell density $x_v$. In such embodiments, the dead and lysed cell densities may be updated based on a comparison between a predicted and measured value of the viable cell density $x_v$ using equation (14) and a state observer.

Updating the sate space model may also be referred to as "parameterising" or "reparameterising" the state space model. Thus, the method of any aspect may comprise parameterising the state space model. Parameterising the state space model may comprise obtaining a measurement corresponding to a state variable for a bioprocess, predicting the value of one or more state variables for the bioprocess using the state space model with a first set of parameters, and determining whether the measurement is within a range of the values predicted by the state space model with the first set of parameters. Parameters of the state space model may include coefficients associated with the state space model, such as e.g. any coefficient of equations (11) to (25)/(25a). The method may further comprise providing a second set of parameters based on the difference between the measurement and the values predicted by the state space model with the first set of parameters.

A simplified version of equations (11) to (14) combines all effects of growth and death rate into a single variable, $\mu_{tot}$ (equation (26)). In this form, and allowing for volume changes, we can write a new set of equations including equation (26) and equations (27)-(28):

$$\frac{dx_v}{dt} = \left( \mu_{tot} + \frac{F_h - F_f}{V} \right) x_v \qquad (26)$$

$$\frac{dV}{dt} = F_f - F_h - F_b \qquad (27)$$

$$\frac{d\mu_{tot}}{dt} = 0 \qquad (28)$$

Note that equation (26) can also be used with the assumption that no volume change is expected, i.e. $F_f = F_b + F_h$. However, allowing for the volume to change by including equation (27) or an equivalent thereof that models volume change enables volume control to be performed jointly with control of other variables, such as the viable cell density in the example using equations (26)-(28). Equation (27) can also be used together with a kinetic growth model that does not make the simplifications in equation (26) (e.g. equation (11) or equations (11)-(14)), and/or together with a model that captures the rate of change of concentration of one or more metabolites (e.g. equations (25) or (25a)). This enables respectively the joint control of the volume as well as the cell density (e.g. where equation (27) is combined with equation (11) or equations (11)-(14)) and concentration of one or more metabolites (e.g. where equation (27) is combined with equations (25) or (25a)), or all of the above (where equation (27) is combined with (i) equation (11) or equations (11)-(14), and (ii) equations (25) or (25a)).

**Simulation of downstream process**

The methods described herein make use of a simulation of a downstream chromatographic process receiving the harvest stream of the upstream process 10 as input feed, in order to determine constraints and/or objectives for control of the upstream process. Any chromatographic simulation process known in the art can be used for this purpose. In particular, any mechanistic modeling approach (also referred to herein as "first principles models"), data driven approach or hybrid mechanistic and data driven approach may be used. For example, any of methods review in Shekhawat & Rathore (2019), any of the mechanistic modeling approaches available in the CADET simulation platform (von Lieres & Andersson, 2010; cadet.github.io/master/index.html), or hybrid mechanistic and data drive approaches such as that in Narayanan et al. (2021) can be used. However, the present disclosure also provides a new hybrid modeling approach which is usable in methods of the disclosure. This is referred to herein as "universal chromatography model" (UCM) and will now be described in more detail.

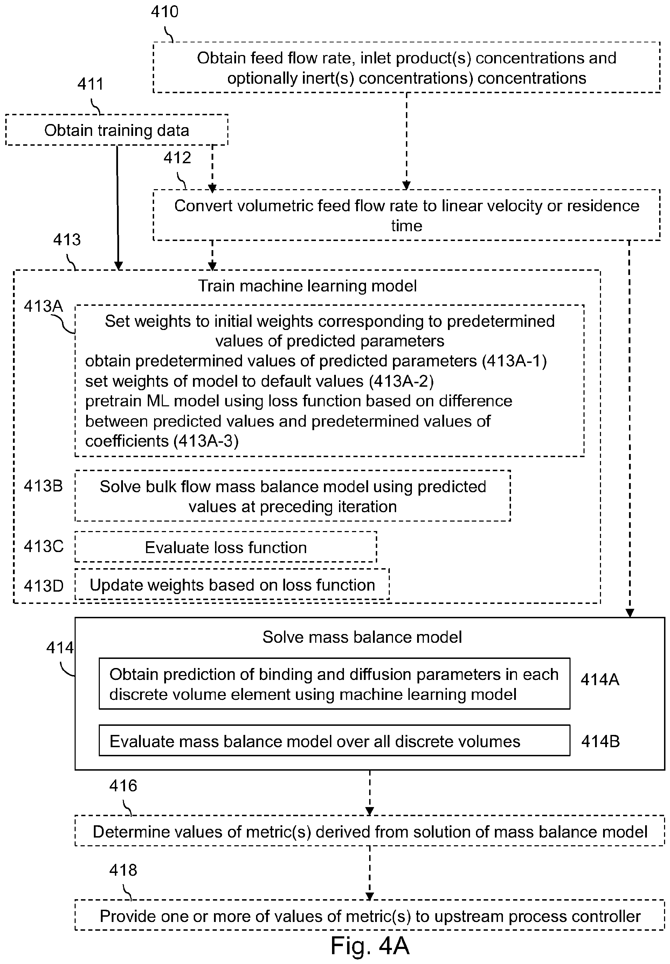

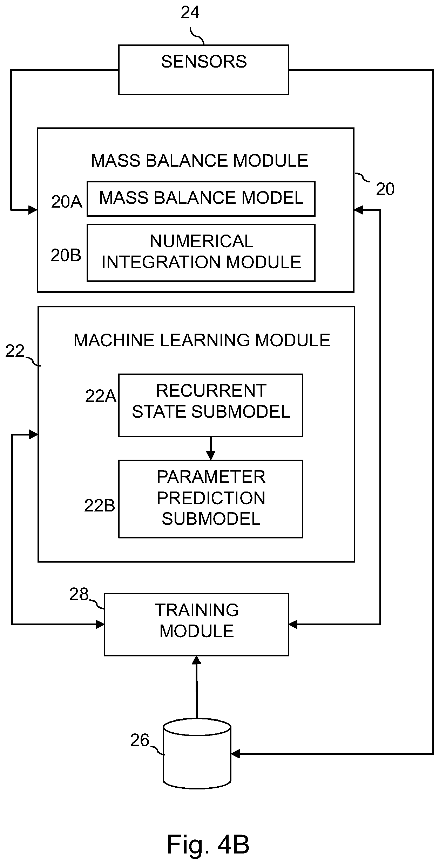

**Figure 4** illustrates schematically (A) a method of simulating a chromatographic process according to the UCM method described herein and/or obtaining a trained machine learning model according to UCM method described herein, and (B) components used in a method of simulating a chromatographic process and/or obtaining a trained machine learning model according to embodiments of the UCM method. These methods make use of a hybrid, physics informed chromatography model comprising a simple physics-based model limited to mass balance equations in the bulk flow of the liquid phase, and a machine learning model trained to predict the value of phenomenological parameters of the physics based model capturing all binding kinetics details, diffusion effects and any other interactions (e.g. all resin/membrane interactions, competitive binding, etc.).

As explained above, a chromatographic process is a process whereby a feed flow comprising one or more products is passing through a chromatography unit comprising a stationary phase that the one or more products interact with (loading phase). The stationary phase can then be washed with a first buffer (wash phase), and the product can be eluted with a second buffer (elution phase). Finally, the stationary phase can be regenerated for use in a new chromatography cycle. The UCM methods described herein are applicable to simulate any part of the chromatography process, provided that training data for the corresponding phase is used to train the machine learning model as will be described further below. For example, the methods described herein can be used to simulate the loading phase of a chromatography process (e.g. to obtain simulated breakthrough curves and metrics derived therefrom), using a machine learning model trained using training data comprising loading phase data (e.g. breakthrough curves). As another example, the methods described herein can be used to simulate the loading and elution phases of a chromatography process, using respective machine learning models trained using training data comprising loading phase data and elution phase data, respectively. The wash phase can be ignored or can also be simulated using a machine learning model trained using training data comprising wash phase data. Note that the same machine learning model can be used to simulate multiple phases (such as e.g. complete cycles comprising load, wash, elute and regeneration, or parts thereof such as e.g. just a load-wash-elute process) provided that the machine learning model was trained using training data comprising the multiple phases modelled.

A method of simulating such a chromatographic process can comprise step 414 of solving (e.g. using a mass balance module 20 implemented in a processor) a bulk flow mass balance model (e.g. implemented in a mass balance model 20A stored in the mass balance module 20) over one or more discrete volume elements along the chromatography unit by numerical integration (e.g. using a numerical integration module 20B of the mass balance module). The bulk flow mass balance model is an ordinary differential equations model that represents the change in concentration of bound and unbound fractions of the one or more products in a discrete volume element. The ordinary differential equations model comprises binding and diffusion terms, wherein a binding term captures the binding of a product to the stationary phase and a diffusion term captures the diffusion of a bound product. These binding and diffusion terms are each parameterised by a single respective parameter that is predicted by a machine learning model trained to take inputs comprising the concentration of the bound and unbound fractions of the one or more products in a discrete volume element and produce as output a prediction of the parameters of the binding and diffusion terms. Thus, solving the bulk flow mass balance model can comprise at step 414A using a machine learning module 22 implemented in a processor, storing or accessing a trained machine learning model, and using the trained machine learning model to predict the values of the diffusion and binding term parameters using inputs comprising the concentration of the bound and unbound fractions of the one or more products in a discrete volume element.

The methods described above advantageously make use of a very simple physics based model that only represents bulk flow and the effects of binding and diffusion on bulk flow via phenomenological terms (i.e. terms that do not explicitly model the detailed physico-chemical phenomena underlying the dynamics of binding of the products to the stationary phase and diffusion of the bound product), combined with a data driven phenomenological parameterisation model to provide the bulk level phenomenological parameters representing diffusion and binding. The phenomena that are not explicitly modelled occur in different compartments of the chromatography unit, such as e.g. in pores and on films on surfaces, along multiple dimensions and involve physico-chemical processes that are not necessarily well characterised. The present methods advantageously bypass all of this, using a machine learning algorithm to identify all parts of the dynamic behaviour of the system that are not easily explained by first principles. Advantageously, the approach relying on differential volume elements and including simple diffusion and binding terms that are captured as ODEs means that the model has the same architecture regardless of the geometry of the chromatography unit. In other words, the model does not explicitly represent diffusion geometry, and the same model can be used for columns with different geometry, enabling straightforward application for *in silico* scale up experiments. Further, the use of ODEs over discrete volume elements means that the simulation can be run without the need for adaptive step-size partial differential equation solvers. As a result, the simulation is both more stable and more computationally efficient than prior art methods.

As the skilled person understands, numerical integration is an iterative process whereby a model is integrated over subsequent discrete time periods. Thus, solving the model can comprise iteratively evaluating (step 414B) the model over all discrete volumes at a respective time using solutions from the previous iteration, where the binding and diffusion parameters are evaluated at each iteration using the machine learning model and concentrations in the respective discrete volume elements at the preceding iteration. This can use, at every time point, initial conditions for the first discrete volume of the column set by observed concentrations of the products (an inerts, if used) received at step 410, and a known feed flow value (volumetric feed flow is used in the model defined below) also received at step 410.

The chromatography unit can comprise a single column. The chromatography unit can comprise a pluralities of columns connected in series and simulating the chromatography process can comprise connecting a plurality of instances of the bulk flow mass balance model each corresponding to a respective column such that the outlet concentration obtained by solving a bulk flow mass balance model for a first column of the plurality of columns sets the inlet concentration used for solving the bulk flow mass balance model for a second column of the plurality of columns connected in series with the first column.

The bulk flow mass balance model can comprise linear terms representing the flow of unbound compounds (products and optionally inerts) into and out of each discrete volume element, and the binding and diffusion terms, wherein a binding term captures non-linearities of the process of adsorption of unbound product on the static phase through the prediction of the respective parameter of the term by the machine learning model at each iteration of the numerical integration. The bulk flow mass balance model can comprise equations (8) and (9) and optionally equation (10) below:

$$\frac{d\vec{c}_{u,n}}{dt} = \frac{f_k}{\Delta V}\left(\vec{c}_{u,n-1} - \vec{c}_{u,n}\right) - \tilde{k}_a \circ \vec{c}_{u,n} + \vec{k}_d \circ \vec{c}_{b,n} \qquad (8)$$

$$\frac{d\vec{c}_{b,n}}{dt} = \tilde{k}_a \circ \vec{c}_{u,n} - \vec{k}_d \circ \vec{c}_{b,n} \qquad (9)$$

$$\frac{d\vec{c}_{i,n}}{dt} = \frac{f_k}{\Delta V}\left(\vec{c}_{i,n-1} - \vec{c}_{i,n}\right) \qquad (10)$$

where $f_k$ is the feed flow rate, $\Delta V$ is the volume of the discrete volume element n, $\vec{c}_{u,n}$ is a vector of concentrations of the unbound products in the discrete volume element n, $\vec{c}_{b,n}$ is a vector of concentrations of the bound products in the discrete volume element n, $\vec{c}_{u,n-1}$ is a vector of concentrations of the unbound products in the discrete volume element preceding the discrete volume element $n$, where discrete volume elements are sequentially labelled from input to output of the chromatography unit, $\vec{c}_{i,n}$ is a vector of concentrations of one or more inerts in the discrete volume element $n$, $\vec{c}_{i,n-1}$ is a vector of concentrations of the inerts in the discrete volume element preceding the discrete volume element $n$, and $\circ$ is an elementwise product. In these equations, $\tilde{k}_a$ and $\vec{k}_d$ describe the overall effect, at any given sampling time, of binding and diffusion on the bulk concentration for each non-inert species in the discrete volume. Thus, the bulk flow mass balance model can further represent the change in concentration of one or more inerts in a discrete volume element. In such embodiments, the machine learning model inputs further comprise the concentration of the one or more inerts in the discrete volume. This system of equations only represents three macrolevel phenomena in discrete volumes along the chromatography unit: flow of unbound products in and out of the volume (and optionally flow of inerts in and out of the volume), diffusion of bound products, and binding of unbound products to the stationary phase. The latter two are parameterized with "catch-all" parameters in vectors $\tilde{k}_a$ and $\vec{k}_d$, which are calculated as the output of the machine learning model. The bulk flow mass balance model can be solved over N discrete volume elements (N being an integer), where N is a parameter of the chromatographic process simulation method which represents the granularity at which the chromatography unit is modelled. N is at least 2, and can be between 5 and 20, between 5 and 15, or selected from 8, 9, 10, 11, 12. The present inventors have found values of N equal to about 10 to afford good accuracy of simulation, stability and computational efficiency. The discrete volume elements can comprise a plurality of discrete volume elements along the chromatography unit in which binding and diffusion occurs, preceded by a discrete volume element in which no binding or diffusion occurs (entry dead volume) and followed by a discrete volume element in which no binding or diffusion occurs (exit dead volume). An embodiment of such a model is illustrated on **Figure 12.**

Thus, in more detail, solving the bulk flow mass balance model at step 414 can comprise iteratively integrating the model over a plurality of discrete time intervals of predetermined duration, wherein at each iteration predictions for the parameters of the binding and diffusion terms are obtained for each of the discrete volume elements using the machine learning model (step 414A), and a current state vector comprising the concentrations of bound products in each discrete volume element at a latest iteration, the concentrations of unbound products in each discrete volume element at the latest iteration, and optionally the concentrations of inerts in each discrete volume element (if used) a the latest iteration. The predictions for the parameters of the binding and diffusion terms can further use the feed flow rate (which can also be provided as input to the machine learning model), and any ither input provided to the machine learning model such as e.g. a parameter indicative of the age of the column. At each iteration, these predictions are used to evaluate the mass balance model over the discrete volumes at step 414B by identifying a discrete state-space model using the bulk flow mass balance model parameterised using said predictions, and determining an updated state vector for the time interval corresponding to the current iteration using the values of the current state vector and the coefficients of the discrete state-space model. For example, solving the bulk flow mass balance model can comprise linearising equations (9)-(10) around the current state (defined by $\vec{c}_{u,k,n}$, $\vec{c}_{b,k,n}$, $\vec{c}_{i,k,n}$, $f_k$) by obtaining estimates for the parameters $\tilde{k}_a$ and parameter $\vec{k}_d$ from the machine learning model, calculating a discrete state-space model using equation (30), and updating the state for one or more intervals using equation (31) below:

$$\left(e^{\begin{bmatrix} A & B \\ 0 & 0 \end{bmatrix}}\right)^T = \begin{bmatrix} A_d & B_d \\ 0 & I \end{bmatrix} \qquad (30)$$

$$x_{k+1} = A_d x_k + B_d u_k \qquad (31).$$

Calculating a discrete state-space model can comprise calculating matrices $A_d$ and $B_d$ using equation (30). In embodiments, calculating a discrete state-space model can comprise reusing the coefficients of a calculated discrete state-space model (e.g. $A_d$ and $B_d$) from a previous iteration when it is determined that the coefficients of the linearized bulk flow mass balance model at the current iteration are within a predetermined range from those of the the linearized bulk flow mass balance model at the previous iteration. This can advantageously improve computational efficiency of the method by removing the need to re-calculate the discrete state-space model at every iteration, when the dynamics of the system have not changed much between subsequent iterations.

The machine learning model can further take as input the feed flow rate $f_k$. Advantageously, this means that the model (combining the mass balance model and the machine learning model) is able to capture the effects of flow rate on the dynamics of binding and diffusion (via the machine learning model), thereby making it possible to use the same model to simulate columns at different flow rates. Indeed, fluid-mechanic, binding and diffusion effects can be learned from experimental data generated at different flow-rates, making the resulting trained model applicable at least to any range of flow rate for which training data (or training data with similar flow rates) was available. Further, because the flow variable, $f_k$, is not used by the machine learning model in a first-principle derived equation, this variable may be "transformed" by the user before being passed to the machine learning model. Thus, the method can optionally comprise obtaining a volumetric feed flow rate at step 410, converting said feed flow rate to a linear velocity or residence time at step 412 and providing said converted feed flow rate as input to the machine learning model at step 414A. For example, while the mass balance model uses a volumetric flow rate, the flow rate can be converted to linear velocity or residence time prior to providing it as input to the machine learning model. This is expected to result in models that are less scale dependent. This property is particularly advantageous for applications where there is a desire to identify models from experiments conducted in small columns and make predictions using those models for larger-scale (commercial manufacturing relevant) columns.

In embodiments, the machine learning model further takes as input a parameter indicative of the age of the chromatography unit. For example, the machine learning model can take as input the number of cycles that the chromatography unit has been operated for, the number of total volumes of the unit that have gone through the unit, or any other value derived therefrom (e.g. % of estimated lifetime as the number of cycles that the chromatography unit has bene used for relative to the expected number of cycles in the lifetime of the column, etc.). This advantageously enables the machine learning model to accurately capture effects of an aging column on the dynamics of the chromatography process.

The machine learning model can be a recurrent machine learning model, wherein a recurrent machine learning model is a machine learning model that is able to account of the values of one or more predictions made at one or more preceding iterations of the numerical integration when making predictions at a current iteration of the numerical integration. For example, the machine learning model can be a machine learning model that further takes as input a recurrent states vector $\vec{x}_{r,k,n}$ which comprises one or more state values for a current iteration $k$ and discrete volume element $n$, and produces as output an updated recurrent state vector $\vec{x}_{r,k+1,n}$ for use at the subsequent iteration. The present inventors have found a single state value for each discrete volume element to be sufficient to enable the machine learning model to capture the dynamics of binding and diffusion in a chromatography process. Thus, the recurrent states vector $\vec{x}_{r,k,n}$ can contain a single state value for each discrete volume element $n$ for the current iteration $k$. As illustrated on **Figure 4B,** the machine learning model can comprise a parameter prediction submdodel 22B and a recurrent state submodel 22A (both of which can be executed by the machine learning module). The recurrent state submodel is configured (i.e. trained) to predict the updated recurrent states vector $\vec{x}_{r,k+1,n}$ for use at the subsequent iteration based on the inputs of the machine learning model and the parameter prediction submdodel is configured (i.e. trained) to predict the parameters of the binding and diffusion terms based on the inputs of the machine learning model. The recurrent states vector can be concatenated with the other input variables (concentrations of the bound and unbound products, feed flow rate and concentrations of inerts if used). Prior to use as input by the machine learning model, the recurrent states vector can be transformed using any function configured to produce a bounded value from an unbounded value, such as a tanh or sigmoid function. This advantageously ensures that the state values do not increase to dominate the other input variables. In embodiments comprising a parameter prediction submdodel and a recurrent state submodel, the concatenated input can be fed into both the parameter prediction submdodel and the recurrent state submodel. The parameter prediction submodel 22B is configured (i.e. trained) to provide predictions of the vectors $\tilde{k}_a$ and $\vec{k}_d$ for use at the current iteration $k$ for each volume $n$ (i.e. $\tilde{k}_{a,k,n}$ and $\vec{k}_{d,k,n}$) associated with concentrations $\vec{c}_{u,k,n}$, $\vec{c}_{b,k,n}$, $\vec{c}_{i,k,n}$, flow rate $f_k$, and current state vector $\vec{x}_{r,k,n}$. At every iteration, the concentrations $\vec{c}_{u,k,n}$, $\vec{c}_{b,k,n}$, $\vec{c}_{i,k,n}$ are obtained by integration of the model provided by equations (8)-(10) (which itself uses predictions from the machine learning model for the $\tilde{k}_a$ and $\vec{k}_d$ parameters at the previous iteration). Further, at every iteration the machine learning model as illustrated additionally outputs an updated state vector for use at the next iteration.

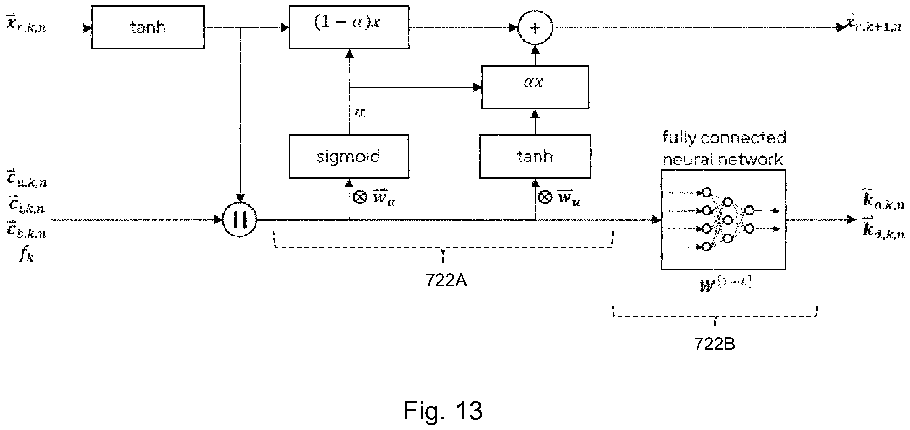

The recurrent state submodel and the parameter prediction submodel can be parameterised by respective weights that are all learned simultaneously. The trained machine learning model can be associated with learned weights that are the same for every discrete volume element and every iteration of the numerical integration. The recurrent state submodel 22A can comprise a first branch comprising a node that takes the concatenated input and applies a first activation function with learned weights vector $\vec{w}_a$, and a second branch comprising a node that takes the concatenated input and applies a second activation function with learned weights vector $\vec{w}_d$. Both activation functions can be functions configured to produce a bounded output from an unbounded input. The first activation function (in the first branch) can be configured to produce an output ($\alpha$) bounded between 0 and 1. For example, the first activation function can be a sigmoid function. The first branch can further comprise a node that takes as input the recurrent states vector $\vec{x}_{r,k,n}$ of the current iteration of the numerical integration and the output of the first activation function and produces an output that weights the values of the recurrent states vector $\vec{x}_{r,k,n}$ based on the output of the first activation function (e.g. weighting by 1-$\alpha$). The second activation function produces an output that can be interpreted as a new proposed value for the recurrent states vector. The first activation function produces an output that can be interpreted as a "forgetting factor". The second branch can further comprise a node that takes as input the output of the first activation function and the output of the second activation function, and produces an output that weights the output of the second activation function based on the output of the first activation function (e.g. weighting by $\alpha$). The outputs of the first and second branches can be summed to produce the output of the recurrent state submodel. This output can be interpreted as a weighted average between the current value of the recurrent states vector $\vec{x}_{r,k,n}$ and proposed new values of the recurrent states vector $\vec{x}_{r,k,n}$, wherein the current and proposed new values are weighted by (1-$\alpha$) and $\alpha$, respectively (i.e. based on the forgetting factor). An embodiment of such a machine learning model is illustrated on **Figure 13**.

The machine learning model can be a nonlinear regression model. the machine learning model can comprise a neural network, which can be fully connected and can comprise any number of layers. In embodiments, the neural network comprises at least 2 hidden layers, between 1 and 4 hidden layers, or 2 or 3 hidden layers. In embodiments, the neural network comprises between 8 and 60 nodes in each hidden layer. For example, the neural network can comprise between 1 and 4 hidden layers with 8 to 60 nodes each, such as 2 or 3 hidden layers with at least 10 nodes each. For example, the neural network can comprise 2 hidden layers with about 20 nodes each. The neural network can additionally comprise a plurality of input nodes (1 for each input) and a plurality of output nodes (one for each output). The output nodes can each comprise an activation function that is a function configured to produce a bounded output from an unbounded input, such as e.g. a tanh or sigmoid function. The function can be a smooth function (e.g. not a step function). The neural network can consist of the above input nodes, output nodes and hidden layers. Note that the trained machine learning models are typically specific to a particular stationary phase and product(s) (and inert(s) if used), but are applicable to any column size, configuration, product(s) concentration(s) (and inert(s) concentration(s), if used) and feed flow rate (although it is advantageous for the product / inert concentrations and feed flow rates used for simulation to be within ranges of values for which training data was available, or within a certain distance from those, such as e.g. within 50% of those, as this is likely to improve the accuracy of the resulting predictions).

As the skilled person understands, solving a bulk flow mass balance model over one or more discrete volume elements along the chromatography unit by numerical integration comprises integrating the model for each of the one or more discrete volume elements at a plurality of discrete time points, thereby obtaining simulated data (also referred to as a trajectory) comprising the concentration of the products in the effluent of the chromatography unit at the plurality of discrete time points. In embodiments, the method can further comprise at step 416 determining using said simulated data, one or more of: effluent product concentrations for a plurality of simulated fractions using the simulated data and a predetermined fractionation scheme, a product adsorption rate, a product desorption rate, a total captured amount of a product, a dynamic binding capacity of the chromatography process with respect to a product, a product binding capacity per cycle, a product percent recovery, a maximum breakthrough concentration with respect to a product, a maximum percent breakthrough with respect to a product, and costs associated with the simulated chromatography process. Any of these metrics can be used as part of a term of the objective function used to control the upstream process as described herein. In particular, the method can comprise determining using the simulated data for the chromatography process, costs associated with said process using an economic model that takes into account one or more of: a cost associated with regeneration of the chromatography unit, a cost associated with buffer consumed in the chromatography process, a cost of waste generated in the chromatography process, and a gain associated with the amount of product recovered. Thus, the method can comprise step 418 of providing values of one or more of said metrics to an upstream process controller or control method. The method can comprise (e.g. at step 416) determining effluent product concentrations for a plurality of simulated fractions using the simulated data and a predetermined fractionation scheme. This can be used when deploying the method for simulation, and when using the simulation for training of the machine learning model when the training data comprises concentration data associated with a time range during a chromatographic process, i.e. data collected using a fraction collector and a known fractionation scheme.

The method can further comprise, prior to step 414, a step 413 of training the machine learning model, using training data comprising feed concentration data and effluent concentration data from a plurality of chromatography processes. Alternatively, the method can comprise receiving a previously trained model, wherein the machine learning model has been trained using training data comprising feed concentration data and effluent concentration data from a plurality of chromatography processes. Feed concentration data is data indicative of inlet product concentration and effluent concentration data is data indicative of outlet product concentration. The training data can comprise feed end effluent concentration data each associated with a respective discrete time points during a chromatographic process and/or feed and effluent concentration data associated with a time range during a chromatographic process, such as data collected using a fraction collector. The training data further comprises feed flow rate data. This can be a volumetric flow rate, a linear speed or a residence time, and conversion between any of these (e.g. to provide a volumetric flow rate for use by the mass balance model and any rate of choice for use by the machine learning model) can also be performed as explained in relation to step 412. The training data can comprise data indicative of concentration of one or more products (and optionally one or more inerts) in the effluent of the chromatography unit. This can be referred to as outlet concentration or effluent concentration data. This data can include time resolved concentrations (e.g. from inline sensors) and fractionated effluent concentrations. The training data can comprise data indicative of concentration of one or more products (and optionally one or more inerts) in the feed of the chromatography unit. This can be referred to as inlet concentration or feed concentration data. Each such data is typically in the form of a plurality of concentrations or information indicative of the concentrations as a function of time. Therefore, they can also be referred to as trajectories. The data indicative of concentration of one or more products in the effluent of the chromatography unit can be in the form of one or more breakthrough curves, which include time resolved concentrations of product(s) in the effluent of the chromatography unit, i.e. a data set of concentration of product(s) in effluent as a function of time. The training data can comprise data that has been collected for the same products, optionally the same buffer (i.e. the same mobile phase), using the same stationary phase at a plurality of sets of process parameters comprising a feed flow rate and a feed concentration. The training data can comprise data that has been collected at a plurality of feed flow rates. The training data can comprise multiple replicates for at least one set of process parameters. The training data can comprise fraction concentration data, i.e. effluent concentration data points each associated with respective time range during a chromatographic process. In such cases the effluent concentration data can include a plurality of effluent concentration data points for each chromatography process in the training data. For example, the training data can comprise, for each chromatography process at least 5, 10 or more effluent concentration data points associated with respective time points or time ranges. As the skilled person understands, in embodiments where the machine learning model takes as input the feed flow rate and/or any other input such as a parameter indicative of the age of the chromatography unit, this data is typically also part of the training data, for each experimentally determined set of concentration data. Thus, the method can comprise at step 413 using a training module 28 implemented by a processor to train the machine learning model using training data comprising effluent product concentration data from a plurality of chromatography processes. The training module 28 can obtain the training data from a data store 26 and use this to train the machine learning model (e.g. including the recurrent state submodel 22A and the parameter prediction submodel 22B) stored in the machine learning module 22. The training data may have been obtained from sensors 24 or from a user interface at step 411. The training module 28 can communicate with the mass balance module 20 to solve the mass balance model 20A using the numerical integration module 20B using predictions from the machine learning module 22. The simulated data obtained through this process can be used by the training module 28 to update the weights of the machine learning model 22 using the simulated data and corresponding training data obtained from data source 26.

Training the machine learning model can comprise at step 413B, for each of one or more training datasets each comprising feed concentration data, effluent concentration data and feed flow rate data for a chromatographic process corresponding to a plurality of time points during the chromatographic process, solving the bulk flow mass balance model using values of the parameters of the binding and diffusion terms predicted by the machine learning model at a preceding iteration of the training at discrete time intervals corresponding to plurality of time points from the training dataset, with feed rate trajectories corresponding to the feed flow rate data from the training dataset and feed concentration data from the training dataset, thereby obtaining simulated data corresponding to each of the one or more training dataset. Training the machine learning model can comprise at step 413C, evaluating a loss function quantifying the difference between the simulated data and the corresponding one or more training datasets. Training the machine learning model can comprise at step 413D, updating the weights of the machine learning model based on the weights of the machine learning model at the current iteration and the value of the loss function, optionally using a gradient descent procedure, until one or more predetermined stopping criteria are satisfied. The one or more predetermined stopping criteria can apply to the number of iterations, the value of the loss function and/or the difference between the updated weights and the weights at the current iteration. The iterative training process can start with default weights, random weights, weights of a previously trained model (e.g. for a similar chromatographic process, e.g. similar products, inerts and/or mobile phase), or weights initialised using a "warm start" procedure as will now be described.

A "warm-start" procedure can also be referred to as "pretraining", and sets weights of the machine learning model to values that are promising starts for training of the model, thereby increasing the computational efficiency of the training process and increasing the likelihood that the training process will converge to a suitable solution. In embodiments, training the machine learning model can comprise at a first iteration of said iterative training, setting (step 413A) the weights of the machine learning model to initial weights that correspond to predetermined values of the parameters of the binding and diffusion terms. Setting the weights of the machine learning model to said initial weights can comprise obtaining said predetermined values of the parameters of the binding and diffusion terms (step 413A-1), setting the weights of the machine learning model to default values (step 413A-2), and pretraining (step 413A-3) the machine learning model, using a loss function that is based on the difference between predicted values from the model and the predetermined values of the parameters of the binding and diffusion terms. In particular, said pretraining comprises iteratively updating the weights of the machine learning model by: predicting, using the machine learning model, values of the parameters of the binding and diffusion terms using inputs to the machine learning model sampled from the training data, evaluating a loss function quantifying the difference between the predicted values of the parameters of the binding and diffusion terms and the predetermined values of the parameters of the binding and diffusion terms; and updating the weights of the machine learning model based on the weights of the machine learning model at the current pretraining iteration and the value of the loss function. Advantageously, this approach increases the chance of the model training converging, compared to a random weight or other uninformed weight initialisation procedure. This is particularly important in the present context because the machine learning model captures complex dynamic phenomena that, if improperly parameterised, can lead to instability in the solution of the mass balance module, in some cases preventing the entire training method from converging.

Step 413A-1 of obtaining the predetermined values of the parameters of the binding and diffusion terms can comprise one or both of the following processes. In a first process, predetermined values can be obtained by: (i) setting the values of the parameters of the diffusion terms and the parameters of the binding terms or corresponding parameters associated with corresponding non-linear binding terms to respective default values, (ii) solving the bulk flow mass balance model by numerical integration using these default values and predetermined feed concentration and feed flow rate data, optionally wherein said predetermined feed concentration and feed flow rate data are selected as the maximum values of the feed flow rate and feed concentration observed in the training data, (iii) determining whether the numerical integration was not able to calculated any of the solutions of the model, and (iv) when the numerical integration was not able to calculated any of the solutions of the model, updating the default values of the parameters of the binding and diffusion terms by reducing the default values by a predetermined factor. The above steps can be repeated until an iteration where the numerical integration was able to calculate all of the solutions of the model, and the values of the parameters of the binding and diffusion terms are selected as those used at said iteration. In a second process, which can be performed after the first process or instead of the first process, predetermined values can be obtained by: (i) setting the values of the parameters of the diffusion terms and the parameters of the binding terms or corresponding parameters associated with corresponding non-linear binding terms to respective default values (e.g. default values obtained using the first process above); (ii) for each of one or more training datasets each comprising feed concentration data, effluent concentration data and feed flow rate data for a chromatographic process corresponding to a plurality of time points during the chromatographic process, solving the bulk flow mass balance model using said default values of the parameters of the binding and diffusion terms thereby obtaining simulated data corresponding to each of the one or more training dataset; (iii) evaluating a loss function quantifying the difference between the simulated data and the corresponding one or more training datasets; and (iv) updating the default values of the parameters of the binding and diffusion terms based on the value of the loss function, optionally using a gradient descent procedure. The above steps can be repeated until one or more predetermined stopping criteria are satisfied. Advantageously, each of the first and second processes above increase the chance of the training process converging. In particular, the first process ensures that pretraining of the model is based on values of the parameters of the binding and diffusion terms that are unlikely to cause numerical instability when solving the bulk flow mass balance model. The second process ensures that pretraining of the model is based on values of the parameters of the binding and diffusion terms that are informed by data in a computationally efficient process, prior to starting the more computationally intensive training of the machine learning model. A parameter of a diffusion term can be denoted as $\bar{k}_a$ and

can be a linearized version of a corresponding parameter $\vec{k}_a$, calculated as $\tilde{k}_a = \vec{k}_a \circ (\vec{c}_{tot} - \vec{c}_b)$, where $\vec{k}_a$ is a corresponding parameter associated with a corresponding nonlinear binding term (where the corresponding nonlinear binding term is $\vec{k}_a \circ (\vec{c}_{tot} - \vec{c}_b) \circ \vec{c}_{u,n}$), and $\vec{c}_{tot}$ is the total binding capacity of the column (also referred to as static binding capacity of the column). The linear binding term corresponding to this is therefore $\tilde{k}_a \circ \vec{c}_{u,n}$. The value of $\vec{c}_{tot}$ can also be set to a default value that can be identified using the same process as explained above. As the skilled person understands, the reference to "diffusion terms", "binding terms", and "flow terms" refers to multiple terms because a separate equation is evaluated for each discrete volume element n and because there can be multiple products and optionally multiple inerts. However, for a single product in a single discrete volume element there would typically be a single diffusion term, a single binding term and a single flow term (although the same binding term can appear in the equations for the bound and unbound fractions of the product with a different sign).

**Systems**

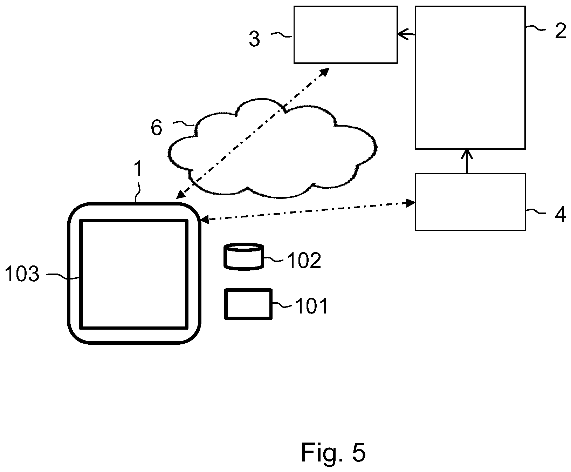

**Figure 5** shows an embodiment of a system for controlling a bioprocess according to embodiments of the present disclosure. The system comprises a computing device 1, which comprises a processor 101 and computer readable memory 102. In the embodiment shown, the computing device 1 also comprises a user interface 103, which is illustrated as a screen but may include any other means of conveying information to a user such as e.g. through audible or visual signals. The computing device 1 is operably connected, such as e.g. through a network 6 or a wired connection, to a bioprocess control system comprising a bioreactor 2 (such as e.g. as illustrated on Figure 1), one or more sensors 3, and one or more effectors 4. The computing device 1 may be a smartphone, tablet, personal computer or other computing device. The computing device 1 is configured to implement a method for controlling a bioprocess, as described herein. In alternative embodiments, the computing device 1 is configured to implement part of a method for controlling a bioprocess, and to communicate with a remote computing device (not shown), to implement other parts of a method of controlling a bioprocess, as described herein. For example, the computing device 1 may be a distributed control unit and may communicate with a remote computing device that implements the functions of one or both of a model predictive controller and a chromatography simulator. For example, the computing device 1 may be a distributed control unit and may communicate with a remote computing device that implements the functions of one or all of the mass balance module 20, machine learning module 22 and training module 28 illustrated on Figure 4B. Communication between the computing device 1 and the remote computing device may be through a wired or wireless connection, and may occur over a local or public network such as e.g. over the public internet. Each of the sensor(s) 3 and optional effector(s) 4 may be in wired connection with the computing device 1 (or the remote computing device, if present), or may be able to communicate through a wireless connection (i.e. through a network 6), such as e.g. through WiFi, as illustrated. The connection between the computing device 1 and the effector(s) 4 and sensor(s) 3 may be direct or indirect (such as e.g. through a remote computer). The connection between the computing device 1 and the effector(s) 4 and sensor(s) 3 may be direct or indirect (such as e.g. through a remote computer). The one or more sensors 3 are configured to acquire data that relates to a bioprocess being performed in the bioreactor 2). The one or more effectors 4 are configured to control one or more manipulated variables of the bioprocess being performed in the bioreactor 2.

The one or more sensors 3 may each be on-line sensors (sometimes also referred to as "inline sensors"), which automatically measure a property of the bioprocess as it progresses (with or without requiring a sample of the culture to be extracted), or off-line sensors (for which a sample is obtained whether manually or automatically, and subsequently processed to obtain the measurement). Each measurement from a sensor (or quantity derived from such a measurement) represents a data point, which is associated with a time (or corresponding maturity) value. The one or more sensors 3 may comprise a sensor that is configured to record the biomass in the bioreactor 2, referred to herein as a "biomass sensor". This is typically in the form of a viable cell density or parameter from which viable cell density can be estimated. The biomass sensor may record a physical parameter from which the biomass in the bioreactor (typically in the form of the total cell density or the viable cell density) can be estimated. For example, biomass sensors based on optical density or capacitance are known in the art. The one or more sensors may further comprise one or more sensors that measure the concentration of one or more metabolites (where the term "metabolites" as used herein includes both substrates of cellular metabolism such as e.g. glucose, as well as products of the metabolism of the cells in the bioreactor), referred to herein as "metabolite sensors". A metabolite sensor may measure the concentration of a single or a plurality of metabolites (such as e.g. from a few metabolites to hundreds or even thousands of metabolites), in the culture as a whole, the culture medium compartment, the biomass compartment (i.e. the cells as a whole), or specific cellular compartments. Examples of metabolite sensors are known in the art and include NMR spectrometers, mass spectrometers, enzyme-based sensors (sometimes referred to as "biosensors", e.g. for the monitoring of glucose, lactate, etc.), etc. Most of the commonly used metabolite sensors measure the concentration of a metabolite in the culture medium. Thus, unless specified otherwise, references to the concentration of a metabolite refer to the concentration of the metabolite in the culture medium in the bioreactor.

As used herein, sensors 3 (such as e.g. metabolite and biomass sensors) may also refer to systems that estimate the concentration of a metabolite or the amount of biomass from one or more measured variables (e.g. provided by other sensors). For example, a metabolite sensor may in practice be implemented as a processor (e.g. processor 101) receiving information from one or more sensors (e.g. measuring physical/chemical properties of the system) and using one or more mathematical models to estimate the concentration of a metabolite from this information. For example, a metabolite sensor may be implemented as a processor receiving spectra from a near infrared spectrometer and estimating the concentration of metabolites from these spectra. Such sensors may be referred to as "soft sensors" (by reference to their "measurements" being obtained using a software rather than by direct measurement). The one or more sensors 3 may further include one or more sensors that measure further process conditions such as pH, volume of the culture, volumetric / mass flow rate of material in / out of the bioreactor, medium density, temperature, etc. Such sensors are known in the art. Whether one or more sensors 3 that measure further process conditions are necessary or advantageous may depend on at least the operating mode and the models used in the control methods. For example, it may be advantageous to include one or more sensors measuring the amount and/or composition of the flows entering and/or leaving the bioreactor. Further, the one or more sensors may include a sensor that measures the volume of liquid in the bioreactor (such as e.g. a level sensor or weighing scales). The measurements from the sensors 3 are communicated to the computing device 1, which may store the data permanently or temporarily in memory 102. The computing device memory 102 may store one or more state observer models and/or one or more controller models as described herein. The processor 101 may execute instructions to provide targets for manipulated variables (the feed, harvest and bleed flow rates) using these models and the data from the one or more sensors 3 and/or simulated data, as described herein (such as e.g. by reference to **Figures 3 and 4).**

As used herein, the terms "computer system" includes the hardware, software and data storage devices for embodying a system or carrying out a method according to the above described embodiments. For example, a computer system may comprise one or more processing units (processors) such as a central processing unit (CPU) and/or a graphical processing unit (GPU), input means, output means and data storage, which may be embodied as one or more connected computing devices. Preferably the computer system has a display or comprises a computing device that has a display to provide a visual output display (for example in the design of the business process). The data storage may comprise RAM, disk drives or other computer readable media. The computer system may include a plurality of computing devices connected by a network and able to communicate with each other over that network. For example, a computer system may be implemented as a cloud computer. Thus, the processors may be webservers.

The methods described herein may be provided as computer programs or as computer program products or computer readable media carrying a computer program which is arranged, when run on a computer, to perform the method(s) described herein. The term "computer readable media" includes, without limitation, any non-transitory medium or media which can be read and accessed directly by a computer or computer system. The media can include, but are not limited to, magnetic storage media such as floppy discs, hard disc storage media and magnetic tape; optical storage media such as optical discs or CD-ROMs; electrical storage media such as memory, including RAM, ROM and flash memory; and hybrids and combinations of the above such as magnetic/optical storage media.

The present disclosure describes and demonstrate an advanced process control method for an upstream process (USP) that uses a predictive model of a DSP (downstream processing) system to determine operating constraints, as well as a predictive model of the USP system to determine operating settings of the USP that satisfy the DSP constraints, and an objective function that determines an economic or operational metric to identify optimal operating settings. This has many benefits relative to the current state of the art. For example, optimized upstream performance enabled by this approach can result in increased production rates (in an example the inventors estimated > 25% increase in USP volumetric productivity in combination with increased DSP efficiency to reduce the total cost of goods (COGS) by 35%.. Further, the approach ensures the stability of the integrated continuous upstream/downstream bioprocess in that the controller maintains the upstream operation in a way that assures efficient operation of downstream processes. Additionally, the approach enables economic optimization of the complete integrated continuous upstream/downstream continuous bioprocess. Additionally, the approach improves the stability of the operation of membrane based cell retention devices thereby enabling extended continuous process operation. Indeed, very low flow rates can cause the cell retention device to overpressure due to a build up of material on the membrane (which cannot be flushed through a normal flush back mechanism when the flow is too low), causing the system to shut down. More stable control of the process prevents these low flow conditions from occurring.

### *Examples*

Exemplary methods of controlling a continuous bioprocess, and exemplary methods of simulating a chromatographic process that can be used in the context of controlling a continuous bioprocess will now be described.

## *Example 1 - Autonomous Control of Continuous Bioprocesses*

### *Introduction*

Operation of continuous biopharmaceutical manufacturing systems practically require coordination between the upstream cell culture process (USP) and downstream product purification steps (DSP). This is different than traditional batch or fed-batch systems where USP and DSP systems are operated independent of each other. With continuous systems the output of the USP is connected to the DSP for processing in real-time. USP must be operated in a way (the output flow, product concentration and quality) that DSP has the capacity to handle. The current state of the art in continuous bioprocess is to operate the cell culture process with local regulatory controls (single-input, single-output, PID control) where bleed flow rate is adjusted to maintain cell density, permeate flow rate (harvest) is used to maintain bioreactor working volume and feed flow rate maintains the overall perfusion rate. Sometimes permeate flow is set to a constant rate and the feed flow rate is adjusted to maintain bioreactor volume as in **Figure 2A.** This type of configuration results in cyclic behaviour as shown in **Figure 2B.** Fluctuations in flow rates as shown in **Figure 2B** has been shown to result in deterioration in the performance of the cell retention device (such as ATF) and also lower viability. Further, this approach does not take into account anything in the DSP process, and at most can handle adjustment of set points in an *ad hoc* manner should this be required for the DSP.

The present inventors have realized that from the perspective of automation, it is ineffective to use typical regulatory control methods such as single loop PID controllers, and that supervisory control methods are needed to for end-to-end system level optimization. A specific challenge in optimization of continuous biopharmaceutical manufacturing processes is the discrete DSP chromatography operation with relative fast dynamics as compared to USP. Quite a lot can happen in a matter of seconds in a chromatography column, while not much happens over hours or even days in a stable USP. This difference in time scales requires novel techniques to transform into a manageable solution. The present inventors further postulated that beyond maintaining stable operation there are many optimization benefits associated with coordination of USP and DSP processes through supervisory control.

They therefore designed a new control approach as described herein, where a predictive model of DSP is used to communicate to USP one or more of operational constraints in inlet flow, product concentration and quality as well as economic cost/value. For example, a predictive model of USP can be used to search through the DSP feasible region to determine the most profitable operating point (as illustrated in Example 3).

### *Autonomous Control*

**Figure 6** shows a high-level diagram of a supervisory controller as described herein, referred to as "Autonomous Control". This includes a *Nonlinear Model Predictive Control* (NMPC) module as the controller as well as a state estimator (e.g., *Moving Horizon Estimation* (MHE) module). Both modules work in a closed loop manner together with the actual process to automate the process and achieve process (and optionally economic) targets as described further below. Control of the continuous process operates in an iterative manner where at each iteration of a control loop, a series of steps are performed to identify how the process should be controlled until the next iteration. The estimation module receives measurements from the controlled process and uses those, together with historical model predictions, to provide estimates of the state of the system. These are provided to the control module. The control module additionally receives constraints typically set by a user, such as operational targets, and/or information that allows calculation of further constraints such as economic metrics. The control module additionally receives constraints from a downstream processing model (e.g. a universal chromatography model as described in Example 4 - although other models can be used), and/or information associated with other costs associated with running the downstream process with predetermined operating parameters (labelled "requests"). Note that the constraints and/or requests may not be re-computed at every iteration. The control module uses all of these inputs to determine the values of manipulated variables that result in optimal control of the upstream process according to a control objective function that takes the DSP constraints / requests into account. The control module can also directly or indirectly produce control actions that cause the manipulated variables to be set to the values that have been determined.

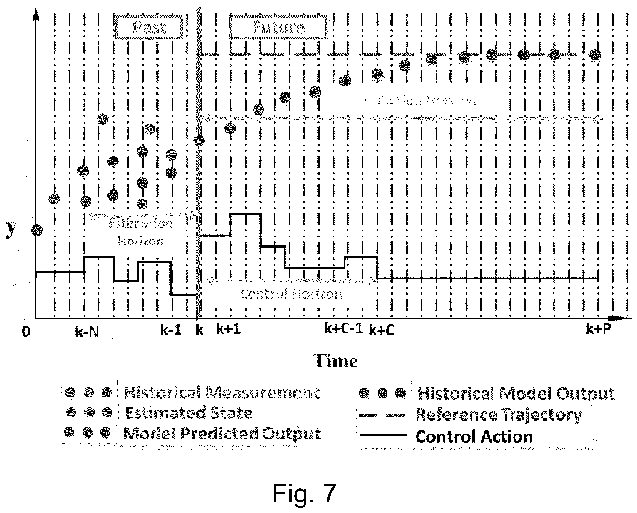

The estimation module (also referred to herein a "state estimator", in this case illustrated as a MHE), makes use of the historical measurements and model predictions over a finite horizon of time in the past (estimation horizon) to improve the overall Autonomous Control performance by providing optimal estimates of the current process states using a combination of historical model predictions and current process measurement. The control module, i.e., NMPC, focuses on the model predictions and planning information over a finite horizon of time in the future (prediction horizon and control horizon, respectively). **Figure 7** presents a general schematic of MHE and NMPC, where the vertical green line represents the current time point (k), the horizontal dashed line represents a reference trajectory (e.g. a set point), the solid stepping line represents the control actions taken on a manipulated variable at each iteration of the control loop (denoted by the k indices, k being the current iteration, k-1 being the preceding iteration, k+1 being the subsequent iteration, N being the number of past iterations taken into account by the estimation module (together forming the estimation horizon), P being the number of subsequent iterations over which the model predictive control models the trajectory of the system, and C being the number of subsequent iterations for which the model predictive control predicts a control action, the control settings being assumed to be constant between the Cth and the Pth subsequent iteration). The dots on the left of the vertical line (k) show measurements and model predictions at preceding iterations as well as the estimated state of the system provided based on these by the state estimator. The observer uses past optimal estimates of the process states (from the observer), past process model outputs, and past measurements, to predict the current process states. The dots on the right of the vertical line (k) show the predicted output provided by the MPC (using the current state estimates from the observer).

The functioning of the estimation module and NMPC will now be described in more detail.

NMPC is an optimization problem that searches within the process feasibility region and finds online the next best actions that needs to be taken now (at time interval *k*) in the process in order to achieve control objectives. As shown in **Figure 7,** the goal is to achieve the target gradually and over a prediction of time in the future to avoid control instability. The *prediction horizon* also allows the controller to make an informed decision based on any possible scheduled changes that may come in the future. To achieve a faster control, the manipulated variables are expected to remain unchanged once the *control horizon* ends (i.e. this simplifies calculations, making the control loop faster). The general formulation for the NMPC module in the Autonomous Control software is as follows:

$$\min_{\substack{\{x_i\}_{i=k+1}^{k+P} \\ ,\{u_i\}_{i=k}^{k+C-1}}} \text{ObjectiveFunction}$$

such that

$$x_{i+1} = f(x_i, \theta_i, u_i), \quad \forall i = k, \dots k + P - 1$$

$$y_i = h(x_i, \kappa_i, u_i), \quad \forall i = k, \dots k + P$$

$$q(x_i, u_i, y_i) \le 0, \quad \forall i = k, \dots k + P$$

$$g(x_i, u_i, y_i) \le 0, \quad \forall i = k, \dots k + P \tag{1}$$

$$x^l \le x_i \le x^u, \quad \forall i = k, \dots k + P$$

$$u^l \le u_i \le u^u, \quad \forall i = k, \dots k + P$$

$$u_i = u_{i-1}, \quad \forall i = k + C, \dots k + P$$

$$\Delta u_i = u_i - u_{i-1}, \quad \forall i = k, \dots k + P$$

$$x_i = \hat{x}_k, \quad \forall i = k$$

$$\theta = \left[\theta_{u_{max}}, \theta_{k_l}, \theta_{u_d}, \theta_{k_t}, \theta_{k_b}, \theta_{k_p}, \theta_{k_{glu}}, \quad ,\theta_{k_{lac}}\right]^T$$

where:

$$x \in \mathbb{R}^{n_x}, y \in \mathbb{R}^{n_y}, u \in \mathbb{R}^{n_u}, x^l, x^u \in \mathbb{R}^{n_x}, u^l, u^p \in \mathbb{R}^{n_u}, \kappa \in \mathbb{R}^{n_\kappa}, \theta \in \mathbb{R}^{n_\theta}$$

$$f: \mathbb{R}^{n_x \times n_\theta \times n_u} \to \mathbb{R}^{n_x}, \ h: \mathbb{R}^{n_x \times n_\kappa \times n_u} \to \mathbb{R}^{n_y}, g: \mathbb{R}^{n_x \times n_u \times n_y} \to \mathbb{R}^{n_g}, , q: \mathbb{R}^{n_x \times n_u \times n_y} \to \mathbb{R}^{n_q}$$

In equation 1:

- scalars *C* and *P* denotes the control horizon and prediction horizon, respectively; *k* is the current time interval (current iteration) while *i* index represents any time within the horizon;
- vectors *y, y$_{SP}$,* and *u* describe the process outputs, setpoints, and inputs, respectively;
- vector *x* describes the process states;
- function *f* is the upstream process model (i.e., for example a cell culture model, CCM) and $\theta$ is the vector of all the upstream process model parameters, e.g. growth model parameters (i.e., $\mu_{max}$, $\mu_d$, *k$_l$*, etc., in CCM);
- function *h* and vector $\kappa$ denote a process output model (e.g., VCD based on the conductivity measured by Viamass) and its parameters (e.g., Viamass calibration), respectively;
- scalars $n_\theta$, $n_x$, $n_y$, $n_u$ are the number of model parameters, state variables, process outputs, and process inputs, respectively;

- function q denotes DSP derived constraints applying to the states ($x_i$), inputs ($u_i$) and outputs ($y_i$) of the process;
- general function $g$ represents the inequality constraints in the USP, excluding the bounds on the states and the inputs;
- the lower and upper bounds on the states and inputs are denoted by the vectors $x^l$, $u^l$, $x^u$, and $u^u$, respectively;
- the definition of the *ObjectiveFunction* is presented below.

Inequality constraints typically involve more than one manipulated variable and therefore differ from simple bounds on variables; for example, an inequality constraint could be set out as $F_f$-($F_h$+$F_b$)≤0.5 which constrains the volume change in the bioreactor to be no more than 0.5 L/day. Inequality constraints can include one or more constraints that are derived from a model predicting critical quality attributes (CQA) of the product. Inequality constraints can include one or more constraints that apply to the product concentration (titer), such as e.g. a minimum required product concentration in the bulk fluid (i.e. in the harvest flow). Inequality constraints can include one or more operational constraints, such as e.g. minimum and maximum possible values of flows, or any other process parameter such as pH, T etc. based for example on equipment constraints. Inequality constraints can include one or more constraints that aim to avoid nutrient limitations, such as e.g. by maintaining a substrate such as glucose above a specified threshold. Inequality constraints ca include one or more constraints that apply to the cell density, such as e.g. maintaining the viable cell density below a maximum threshold due to known process limitations such as delivery of oxygen. Inequality constraints can include constraints on biologically relevant states. These typically aim to assure a stable and healthy culture. For example, constraints that apply to biologically relevant states can a constraint that specifies a minimum viability (VCD/(VCD + dead cell density) and/or a constraint that maximum limit of unmeasured states from the process model such as lysed cell density or biomaterial. Typically, at least a constraint on the maximum viable cell density and one or more constraints on biologically relevant states are included.

Further, as will be explained further below, the objective function itself can include terms that aim to maximise or minimize selected outputs of the process, and/or terms that aim to maintain desired modes of operation such as e.g. using a desired perfusion rate (denoted as $PR_i$) at different stages of the continuous bioprocess. For instance, $F_f$ is often set to follow the CSPR rule during the growth phase, while achieving 2 working volume per day during the perfusion phase.

The initial condition of the NMPC problem- (i.e., $\hat{x}_k$) is provided by the state estimation module in the Autonomous Control software (as explained below).

As is the case with any MPC, the performance of NMPC can be optimized by ensuring that the *ObjectiveFunction* that is defined in a manner that takes into account the objectives of the control process while accounting for the degrees of freedom in the process (e.g. only including objectives that are possible to achieve based on the available manipulated variables and constraints on these variables).

Parameters such as the prediction and control horizon, the weights, bounds are typically set through a tuning process which the skilled person would be familiar with.

The structure of the control objective function and some tips on NMPC tuning is presented next, followed by a discussion on state and parameter estimation to ensure proper initialization and model updates mentioned in point 3 and 4.

An example of a full cell culture model that can be used for USP modelling in the NMPC module is provided in the detailed description. Use of such a cell culture model as the USP model means that the NMPC described can offer control for process outputs included in the cell culture model (such as titer control and glucose control). The model is a kinetic growth model of a cell culture including at least:

(i) an equation that captures the rate of change in viable cell density ($x_v$) as a function of effective growth and death rates $\mu_{eff}$ and $\mu_d$, respectively -equation (11), which can also be simplified if it can be assumed that there is no loss of cells in a bleed flow, and/or if it can be assumed that the volume V is constant;

(ii) an equation that captures the rate of change in dead cell density ($x_d$) as a function of effective death and lysis rates ($\mu_d$ and $k_l$) -equation (12), which can also be simplified if it can be assumed that there is no loss of cells in a bleed flow and/or if it can be assumed that the volume V is constant;

(iii) an equation that captures the rate of change in lysed cell density ($x_l$) as a function of effective rate of lysis of dead cells ($k_l$)- equation (13), which can be simplified if it can be assumed that there is no loss of cells in a bleed flow and/or if it can be assumed that the volume V is constant;

(iv) an equation for the total cell density $x_t$ can be calculated as $x_t = x_{vcd} + x_{dead} + x_{lyse}$ (equation (14));

(v) an equation that captures the rate of change in concentration of an inhibitory biomaterial (also referred to as "inhibitory products" or "toxins") that accumulates in the cell culture as a result of cell maintenance, and that inhibits cell growth ($\varnothing_b$), as a function of the viable cell density with a factor $k_{inh}$ representing the threshold below which there is <5% inhibition (no significant inhibition) -equation (16) which can be simplified if it can be assumed that the inhibitory biomaterials do not exit the bioreactor through the bleed and harvest streams, if that is not the case then;

(vi) an equation that captures the effect of the inhibitory products on growth rate-equation (22')note that this assumes that there are no quadratory inhibitory variables (other than those captures in the inhibitory material term) or substrate limiting term, if that's not the case then equation (17) or equation (22) can be used;

(vii) an equation that captures the effect of accumulation of toxins released by lysed cells on death rate through a toxicity rate $k_t$ through the effective death rate being expressed as a function of a base death rate ($k_d$) and the product of the toxicity rate $k_t$ and the lysed cells density $x_l$ - see equation (15) where $\varnothing_t$ is equal to $x_l$ - note that in alternative embodiments the toxins can be considered to be the same as the inhibitory material and $\varnothing_b$ can be used instead of $x_l$; and

Thus, the model used in the present examples used equations (11) to (17). The model additionally included equations modelling metabolite concentration (equation 25, or simplified versions 25a, 25b), and an equation modelling a product concentration using equation (34). Additional optional features can be included in this model depending on the particular circumstances, including modelling the holdup of biomaterial and lysed cells (equations 13a, 13b, 16a, 16b), modelling the effect of substrate limiting variables, quadratic influence variables and/or inhibitors on cell growth (including e.g. growth inhibition from accumulated biomaterial which is included in the embodiment described here) (using equations 18, 19, 20, 21, 23 and/or 24), and modelling the concentration of antifoam using equation (32). The specific consumption rates can be calculated using equation (33), although in the present embodiments they were calculated in a data driven manner using a partial least square (PLS) model as described above. As explained above, estimation of the consumption rate over the prediction horizon of the controller is provided by a machine learning (or any appropriate method - such as a PLS model in the present examples). This can also be updated based on the consumption rate observed from process measurements. Thus, the state model used in the present examples can be referred to as a hybrid model in the sense that it combines a dynamic growth model and data driven parameter estimation.

*Control Objective Function*

When using an NMPC module as described herein, many different aspects of the USP can be controlled. In particular, the controller can adapt online all the offline DSP constraints and objectives, perform as a stabilizer controller (controlling VCD, reactor volume, and perfusion rate by manipulating feed flow, harvest flow, and bleed flow), offer control for other process outputs included in the cell culture model (such as titer control and glucose control), offer end-to-end economic control, and ensure product maximization.

Control of the process can differentiate between independent variables (also referred to as manipulated variables, i.e. those that can be set to values determined by the controller by operation of effectors in the process), and dependent variables (including controlled variables which are associated with set points or objectives representing target values or behaviours that the control of the process aims to achieve, and any other variables of the process model that are neither controlled nor manipulated).

A generic control objective that includes terms reflecting each of these possibilities can be expressed as follows (although note that not all terms are necessary and in fact some parts of the control objective can be purposefully excluded as will be explained further below):

$$ObjectiveFunction =$$

$$\sum_{i=k+1}^{k+P} \left\| y_{SP_i} - y_i \right\|_{Q_{OUT_i}}^2 + \sum_{i=k}^{k+C-1} \left\| \Delta u_i \right\|_{Q_{IN_i}}^2 + \sum_{i=k+1}^{k+P} \left\| F_{f_i} - y_{vol}^{SP} \times PR_i \right\|_{\Upsilon_{PR_i}}^2 \qquad (2)$$

$$+ \Upsilon_{c_i} \sum_{i=k+1}^{k+P} Cost_{total}(F_{h_i}, \varphi_i) + \Upsilon_{z_i} \sum_{i=k+1}^{k+P} \frac{1}{z_i^2 + 1}$$

where y are the outputs of the process, for example any one or more of the variables below:

$$\boldsymbol{y} = \left[ x_v, x_d, \; x_l, V, \varphi, m_{glucose}, \quad , m_{lactate} \right]^T$$

where $x_v$ is the viable cell density, $x_d$ is the dead cell density, $x_l$ is the lysed cell density, $\varphi$ is the product concentration, m is the concentration of one or more metabolites, here illustrated as glucose and lactate, wich can be substrates and/or biomaterials $\varnothing_b$.

$Y_{sp}$ are any set points for controlled variables of the process, for example any one or more of the variables below:

$$y_{SP} = \left[x_v^{SP}, x_d^{SP}, x_l^{SP}, V^{SP}, \varphi^{SP}, m_{glucose}^{SP}, \quad , m_{lactate}^{SP}\right]^T$$

u are independent (i.e. manipulated) variables of the process, for example any one or more of the variables below:

$$u = \left[F_f, F_h, F_b, pH, Temperature\right]^T$$

and:

$$z \in \{u, y, \quad\}, y_{SP} \in \mathbb{R}^{n_y}, Q_{IN} \in \mathbb{R}^{n_u \times n_u}, Q_{OUT} \in \mathbb{R}^{n_y \times n_y}$$

Each of these terms is explained in Table 1 below.

**Table 1.** Terms of a control objective function as described herein. All of these terms are individually optional, provided that at least one term is present. In practice at least the set point term (first row, and advantageously also the stabilizing term, second row) will be present. Note that as explained above the optimization of the objective function is also subject to a set of constraints. According to the present disclosure at least one of (a) a set of constraints associated with the DSP (e.g. constraints on the minimum and maximum values of $F_h$ and/or $\varphi$), and (b) an objective function term that is associated with the DSP is used (e.g. set points on $F_h$ and/or $\varphi$, and/or costs associated with the DSP at particular values of $F_h$ and $\varphi_t$).

| Term | Objective |
|---|---|
| $$\sum_{i=k+1}^{k+P} \left\| \boldsymbol{y}_{SP_i} - \boldsymbol{y}_i \right\|_{Q_{OUT_i}}^2$$ | Keep process outputs designed as controlled variables at their setpoint (denoted as $\boldsymbol{y}_{SP}$). The $\boldsymbol{Q}_{OUT}$ diagonal matrix is a matrix comprising weights corresponding to each process output; that is, a non-zero diagonal element means the corresponding process output is considered as a controlled variable. For instance, $\boldsymbol{Q}_{OUT_i} = diag([1, 0, 0, 0, 1, 0.5, 0, ,0]^T)$ means that at the $i^{th}$ time interval, VCD, the reactor volume, and the product concentration are controlled variables to the NMPC to be kept at a pre-defined desired variable ($\boldsymbol{y}_{SP}$). The values of the weights can be set by a user (manual tuning) depending on desired behaviour of the process. The values of the weights can be set automatically based on the order of magnitude of the error term for the particular output variable (value of the corresponding element of $\boldsymbol{y}_{SP_i} - \boldsymbol{y}_i$) such that all elements of the sum have the same order of magnitude. This prevents individual controlled variables from dominating the objective simply because their values have higher order of magnitude in the units in which they are expressed. |
| $$\sum_{i=k}^{k+C-1} \left\| \Delta \boldsymbol{u}_i \right\|_{Q_{IN_i}}^2$$ | Avoid aggressive control actions and process instability, i.e., avoiding significant changes in the current control actions when compared to the latest ones). Similar to $\boldsymbol{Q}_{OUT}$, the diagonal matrix $\boldsymbol{Q}_{IN}$ includes the corresponding weights for each process input. For example, considering $\boldsymbol{Q}_{IN_i} = diag([1, 1,1,0,10]^T)$ with $\boldsymbol{u} = [f_{feed}, f_{harvest}, f_{bleed}, pH, Temperature]^T$ sets zero as the weight assigned to $pH$, which sets this input variable to a non-manipulated variable for the NMPC problem. Moreover, the larger the weight, the less aggressive control action is allowed for the corresponding manipulated variable. |
| $$\sum_{i=k+1}^{k+P} \left\| F_{f_i} - y_{vol}^{SP} \times PR_i \right\|_{\Upsilon_{PR_i}}^2$$ | Follow the perfusion rate requested/desirable (denoted as $PR_i$) at different stages of the continuous bioprocess. For instance, $f_{feed}$ is often set to follow the CSPR rule during the growth phase, while achieving 2 working volume per day during the perfusion phase. This control option can be turned off by setting the weight $\Upsilon_{PR}$ to zero. |
| $$\Upsilon_{c_i} \sum_{i=k+1}^{k+P} Cost_{total}(F_{h_i}, \varphi_i)$$ | Cost of goods function enabling optimisation of online control of USP that leads to an economically optimized end-to-end continuous bioprocess. To the best of inventors' knowledge, the end-to-end economic control for continuous bioprocesses as never been described before. To minimize this cost of goods function, the economic NMPC is pushed to choose a feasible operating condition (flows, *Temperature, pH*) such that the maximum feasible $F_h$ and product concentration <p is achieved. Note that the weight $\Upsilon_c$ can be set to 0 to turn off the economic control. Because the numerical value of the cost of goods function is larger (by several orders of magnitude) than all the other terms in the objective function, careful tunning of the weight $\Upsilon_c$ is used to achieve a stable and proper control for all the other process outputs. |

| Term | Objective |
|---|---|
| $\Upsilon_{z_i} \displaystyle\sum_{i=k+1}^{k+P} \dfrac{1}{z_i^2 + 1}$ | Control the process online by maximizing any chosen process variables $z$. Note that scalar $z$ denotes any process variable (e.g., states, inputs, calculated key performance indicators of critical product attributes) that needs to be maximized alongside with all the other terms that need to be minimized. For example, the titer can be maximized by including $<p$ as one of the variables z, or the total product can be maximized by including $F_{h_i} * \varphi_i$ as one of the variables z. To activate the maximization control, the weight $\Upsilon_{z_i}$ should be set to a non-zero scalar value (conversely the weight can be set to 0 if there is no variable to maximise). The function $\dfrac{1}{z_i^2 + 1}$ advantageously enables maximization of a chosen variable while avoiding numerical issues. |

The CSPR rule is a simple approach that is often used to control continuous processes by controlling the feed flow rate to maintain a predefined ratio of feed flow rate and viable cell density (VCD, also referred to as "viable cell concentration", VCC). This is commonly referred to as "cell specific perfusion rate" (CSPR) control.

Note that all the weights associated with each term in the objective function are indexed by time (*i*), which aims to give the option to turn on (when set to positive reals value) or off (when set to zero) each of the corresponding terms, making the Autonomous Control scalable to different designs of experiment (DoEs) or changes in the control plans online and throughout the process operation. That being said, NMPC is an optimization problem and therefore, the user can active/deactivate each term at the objective function as needed to ensure that the problem remain feasible, meaning there is enough degrees of freedom in the NMPC optimization problem. As an example, consider a control scenario in which the flowrates (feed, harvest, bleed) are the only manipulated variables, the goal is to keep VCD and reactor volume at a specific target as well as maximizing the $F_h$ once the process is stable and at the perfusion phase. As long as the target VCD and operating conditions (temperature, oxygen, and pH) are kept unchanged, the overall cell growth rate ($\mu_{eff}$) is expected to remain unchanged for a stable process. A simple calculation at steady-state shows that:

$$F_b = V \times \mu_{eff} \tag{3}$$

Thus, the $F_b$ is expected to remain unchanged. Given that $F_f = F_h + F_b$, if the user wants to perform a harvest maximization control, it is important to let go of the perfusion rate control (should set $Q_{PR}$ to zero) to have enough degrees of freedom in the optimization problem. Alternatively, if the controller has the freedom to adjust *Temperature* and *pH* as needed, then $\mu_{eff}$ and correspondingly $F_b$ have the freedom to change throughout the optimization; there is enough degrees of freedom provided to NMPC to find the maximum $F_h$ feasible while sustaining the perfusion rate and output targets (first and third row terms in Table 1). Therefore, it is feasible from the optimization point of view to have a fixed setpoint for $F_f$ based on the desired perfusion rate, maximize $F_h$ and keep VCD and reactor volume at their setpoints.

The cost of goods function (*Cost_total*) in equation (2) and Table 1 is shown with input arguments including harvest flow and product concentration. These are the primary arguments in the included embodiment but could include other arguments such as process conditions or product quality (host cell protein). A more general description of the function is as follows. The cost of goods function is any function that uses the output of a predictive downstream process model (e.g. predictive chromatography unit model) to map inputs of the DSP to outputs associated with quantifiable costs. Inputs include an operating range of harvest titer (product concentration in the harvest flow) and an operating range of harvest rate (harvest flow rate or proportion thereof that is processed in the DSP). Additional optional inputs can include concentrations of impurities such as host cell proteins, DNA, aggregates, etc. in the harvest flow, and/or one or more metrics of degradation that are predicted or measured outside column model. Outputs include one or more metrics derived from a prediction of the DSP over a predetermined period of time (e.g. 8 hours or any other time scale that makes sense, such as e.g. the control or prediction horizon) which are associated with costs due to consumption of reagents, usage of the DSP, recovery and/or quality of the product. For example, outputs can include one or more metrics selected from: buffer(s) consumption in the DSP, DSP column usage (e.g. % of useable life), % recovery of the product (which can be expressed as a negative cost or as a cost of opportunity by expressing the % of product not recovered) in the DSP, and impurity profile after the DSP (which can alter the quality of the product and/or require additional separation steps with associated costs). In addition to costs associated with the DSP, the cost of goods function can also take into account one or more costs associated with the USP. Thus, the cost of goods function can reflect overall cost of the process, including USP and DSP.

The cost of goods function can be expressed in terms of costs (i.e. the economic value attributable to the outputs can be used in the cost of goods function) or in terms of the outputs themselves (which are expected to be proportional to the economic costs).

Note that in addition to the cost of goods function, the predictive downstream process model is used to set constraints for the optimization of the objective function, specified in equation (1) as $q(\boldsymbol{x_i}, \boldsymbol{u_i}, \boldsymbol{y_i}) \leq 0$.

*Estimation Module*

In order to run online a model-based advanced process control scheme such as MPCs in a closed loop manner, the online measurement of all the state variables in the model is required. Despite many hardware sensors present in the process to measure online the key states of the process, there are always some states of the system that cannot be measured online due to physical and economical limitations (e.g., metabolite concentrations, titer, etc.). In addition to the lack of sensors for some of the states, the measurements provided by the existing sensors are often associated with small noises that may propagate through the prediction horizon of the NMPC and add uncertainty to the final control actions.

Due to these issues, although MPC (and NMPC) has been broadly used in medium-scaled industrial applications in recent years, they are often limited to use a simplified version of a process model where all the states in the model are measurable online through the sensors present in the process. Such simplified MPCs can be successful to stabilize the process. However, a controller based on the full version of the process model (including all the states variables) can provide the opportunity to perform a higher level of control and optimization in addition to obtain process stability. The Autonomous Control described herein has the ability to adopt a full version of the upstream process model (e.g. a cell culture model as described herein) by deploying online state estimation coupled with NMPC. Because the cell culture models described herein can model metabolites and their effects on the overall growth rate, use of these models within a MCP means that it is possible to properly predict the nonlinearity of the system caused by metabolic shifts and cells behavior. In other words, the use of NMPC as described herein enables the Autonomous Control to properly capture this nonlinearity throughout the process. Although there have been some efforts in developing Linear MPC, the development of NMPC for the types of cells used in production of biotherapeutics (e.g. CHO cell-line) is limited.

Online state estimation approaches known in the art can be used to solve problems associated with both lack of measurements and process / measurement noises. That is, state estimators can provide online estimation for all the unmeasurable state variables by making use of all the available resources (online measurement from the sensors, the process and measurement noises, the process model that express how all the measurable and unmeasurable state are correlated). Among these approaches, *Moving Horizon Estimation* (MHE) has received an increasing attention especially when coupled with NMPC, due to their similarity. Like NMPC, MHE is an optimization problem subject to the process model, i.e., it can take into account all the process bounds and feasibility constraints, which makes it a similar problem to NMPC and therefore, a good candidate for online initialization of NMPC. MHE provides an optimal estimation of the states by minimizing the process and measurement noises over a finite horizon of time (referred to as *estimation horizon*). Once the new measurements at the current time arrive, MHE discards the oldest information to make room for the current measurements while keeping the length of estimation horizon constant. All the discarded information is summarized in a penalty term called *arrival cost.*

The MHE formulation in the Autonomous Control framework is as follows:

$$\min_{\{x_j, w_j\}_{j=k-N}^{k-1}} \sum_{j=k-N}^{k-1} \|w_j\|_{Q^{-1}}^2 + \sum_{j=k-N+1}^{k} \|v_j\|_{R^{-1}}^2 + \varphi_{k-N}$$

such that

$$
\begin{aligned}
x_{j+1} &= f(x_j, \theta_j, u_j) + w_j ; & \forall j = k - N, \dots k - 1 \\
y_j &= h(x_j, \kappa_j, u_j) + v_j ; & \forall j = k - N + 1, \dots k \\
q(x_j, u_j, y_j) &\le 0; & \forall j = k - N, \dots k \\
g(x_j, u_j, w_j, y_j) &\le 0; & \forall j = k - N, \dots k \\
x^l &\le x_j \le x^u ; & \forall j = k - N, \quad k
\end{aligned}
\tag{4}
$$

where:

$$w \in \mathbb{R}^{n_x}, v \in \mathbb{R}^{n_y}, Q \in \mathbb{R}^{n_x \times n_x}, R \in \mathbb{R}^{n_y \times n_y},$$

In equation (4), q and g have the same meaning as in equation (1), i.e. the NMPC and MHE are subject to the same g and q constraints (except that he time indices are different as they do not work over the same time periods). In equation (4), $k$ represents the current time interval and index $j$ is a time index within the estimation horizon $N$. $Q$ and $R$ are the covariance matrices of process and measurement noises, respectively; *w, v, x, y,* and $u$ represent vectors that describe the process noise, measurement noise, state variables, outputs, and inputs in the system, respectively. w and v are the decision variables of the optimization problem presented in equation (4), meaning that the solver will make a decision on what should be the value for w and v alongside with the states (x), such that the term

$$\sum_{j=k-N}^{k-1} \|w_j\|_{Q^{-1}}^2 + \sum_{j=k-N+1}^{k} \|v_j\|_{R^{-1}}^2 + \varphi_{k-N}$$

is minimized. Process noise can also be referred to as "process uncertainty" and represents the uncertainty/noise associated with the model vs true value for the states. This noise/-uncertainty is caused by any "unknown" source of model uncertainty that affects unknown states of the system. "Process noise" is a different concept from the term "model mismatch" and "model disturbance". In equation (4), $\theta_j, \kappa_j,$ f and h have the same meaning as in equation (1)/

The penalty term $\varphi_{k-N}$ in Equation (4) provides the arrival cost estimated at time k - N, which is defined as follows:

$$\varphi_{k-N} = \|\boldsymbol{x}_{k-N} - \overline{\boldsymbol{x}}_{k-N}\|^2_{\boldsymbol{P}^{-1}_{k-N}} \tag{5}$$

where $\overline{x}_{k-N}$ and $\boldsymbol{P}_{k-N}$ denote the expected value and covariance matrix of the approximated posterior distribution of the states at the time interval k - N. Extended Kalman Filter (EKF) is the common approach to approximate $\overline{x}_{k-N}$ and $\boldsymbol{P}_{k-N}$ at any given time interval k. Note that k - N is the starting time point in the finite horizon window (start of the estimation horizon).

The estimated states obtained from the MHE problem at the current time ($\hat{\boldsymbol{x}}_k$) are used as the initial condition in the NMPC formulation.

Moreover, regardless of how accurate a process model is, there is always a model mismatch when compared to the actual process. This mismatch can propagate over the prediction horizon and causes a loss in the NMPC performance. In some cases, the model mismatch may derive the process to its feasibility edges, which can cause critical issues such as too much or not enough bleed. In order to solve this problem, model mismatch can be reduced by updating one or more key model parameters. The state estimator module as described herein can optionally perform an online parameter estimation to keep the key model parameters such as $\mu_{max}$ updated throughout the continuous bioprocess. This can be achieved by treating these key parameters of the model as a state of the model, which are estimated using the Jacobian matrix of the system model as will be explained further below.

Based on above, both state and parameter estimations play an important role in the NMPC performance. Given that the Autonomous Control is a software to control the process online, the required computational time for each module in the Autonomous Control framework become a key factor. Therefore, a simultaneous state and parameter estimation becomes computationally favorable. With this in mind, it is advantageous to use robust estimation (e.g., *augmentation* techniques in the MHE framework, such as augmentation technique or AGS-EKF as described in Yu et al. 2023 and Wang et al. 2021) and robust control techniques (e.g., *multi-scenario NMPC, multi-stage NMPC*) to capture online the changes in the model parameters and avoids loss of Autonomous Control performance under faulty process operation. MHE have been used as state estimators in the past but have not been coupled with NMCP for bioprocess control, mainly due to observability issues in continuous bioprocesses and instability challenges in complex approaches such as MHE, which have been dealt with in the Autonomous Control; that is, the state estimator module in the Autonomous Control calculates the dead cells concentration implicitly and therefore, making the system observable. Moreover, it uses methods described in Valipour, M., & Ricardez-Sandoval, L. A. (2021) to define the appropriate bounds on the process and measurement noises that helps achieving an stable state estimator. Further, the MHE described below is able to to deal with measurements arriving at different time in the process (i.e., handling both online and offline measurements). These estimation enhancements make the Autonomous Control robust against occasional device malfunctions and sensors failure.

For this purpose, the current study uses the *augmentation* technique in the MHE scheme to perform an online simultaneous state and parameter estimation. Augmentation technique treats the uncertain model parameters as state variables and perform an estimation over all the actual states and augmented states (uncertain model parameters), all at once. Based on the above, the proposed MHE formulation to perform simultaneous state and parameter estimation is as follows:

$$\min_{\{x_j, \widetilde{w}_j\}^{k-1}_{j=k-N}} \sum_{j=k-N}^{k-1} \|\widetilde{w}_j\|^2_{\widetilde{Q}^{-1}} + \sum_{j=k-N+1}^{k} \|v_j\|^2_{R^{-1}} + \varphi_{k-N}$$

such that:

$$x_{j+1} = f\left(x_j, (\theta_j + \omega_j), u_j\right) + wj\,; \qquad \forall j = k - N, \ldots k - 1$$

$$\theta_{j+1} = \theta_j + \omega_j\,; \qquad \forall j = k - N, \ldots k - 1$$

$$y_j = h\left(x_j, \kappa_j, u_j\right) + v_j\,; \qquad \forall j = k - N + 1, \ldots k$$

$$q\left(x_j, u_j, y_j\right) \leq 0; \qquad \forall j = k - N, \ldots k$$

$$g\left(x_j, u_j, w_j, y_j\right) \leq 0; \qquad \forall j = k - N, \ldots k \qquad (6)$$

$$x^l \leq x_j \leq x^u\,; \qquad \forall j = k - N, \ldots k$$

$$\breve{w}_j = \left[w_j, \omega_j\right]^T\,; \qquad \forall j = k - N, \ldots k - 1$$

$$G = \begin{bmatrix} \dfrac{\partial f}{\partial w_{vcd}} & & \dfrac{\partial \theta_{k_{lac}}}{\partial w_{vcd}} \\ \vdots & \ddots & \vdots \\ \dfrac{\partial f}{\partial \omega_{k_{lac}}} & & \dfrac{\partial \theta_{k_{lac}}}{\partial \omega_{k_{lac}}} \end{bmatrix}$$

$$\breve{Q} = G^T . \overline{Q} . G$$

where:

$$\omega = \left[\omega_{u_{max}}, \omega_{k_l}, \omega_{u_d}, \omega_{k_t}, \omega_{k_b}, \omega_{k_p}, \omega_{k_{glu}}, \ldots, \omega_{k_{lac}}\right]^T$$

$$\omega \in \mathbb{R}^{n_\theta}, \breve{w} \in \mathbb{R}^{n_x + n_\theta}, \widetilde{Q, G, \overline{Q}} \in \mathbb{R}^{(n_x + n_\theta) \times (n_x + n_\theta)}$$

where $n_\theta$ is the number of uncertain parameters that are going to be considered as augmented states, G is the jacobian matrix that describes the correlation between the states (true states and the augmented states) and the uncertainties present in the augmented process model. That is, to capture the dynamic of the process with respect to the changes in the uncertain parameter, the corresponding Jacobian calculations in matrix **G** are updated online, updating the changes in each state variable with respect to the parameter uncertainty (e.g., $\dfrac{\partial f}{\partial \omega_{u_{max}}}$) as well as changes in the model parameters with respect to the parameter uncertainty (e.g., $\dfrac{\partial \theta_{u_{max}}}{\partial \omega_{u_{max}}} = 1$ and $\dfrac{\partial \theta_{k_l}}{\partial \omega_{u_{max}}} = 0$). The matrix $\overline{Q}$ represents the covariance matrix for the uncertainties assotiated with both states and augmented states (e.g., $w_{vcd}, \omega_{klac}$). $\theta$ is the vector of the model parameters, $\theta_m$ represents the elements, as in equation (1). Other terms have the same meaning as in relation to equation (4). When the parameter (*i.e.*, $\theta_m$) is certain (constant), the corresponding noise to that parameter is zero ($\theta_{j+1} = \theta_j$). For simplicity, the parameter estimation technique can assume that any uncertain parameter (augmented state) is associated with an uncertainty, where:

$$\frac{d\theta_m}{dt} = \omega_m; \qquad \forall m \in \left\{u_{max}, k_l, u_d, k_t, k_b, k_p, k_{glu}, \quad, k_{lac}\right\} \qquad (7)$$

### *Example 2 - Flexible Autonomous Control of Continuous Bioprocesses*

To provide an overview on Autonomous Control performance under different control strategies and requests, **Figure 9** highlights the overall control performance for three different control scenarios. The models described in Example 1 were used to control a process under three different control scenarios. In all three scenarios, a CSPR control over the growth phase is used. *Scenario I* focuses on a stabilization strategy while a simultaneous maximization control and stabilization control is used in *Scenarios II* and *III.* **Table 2** below outlines the difference in the basic assumptions between all these scenarios.

**Table 2.** Summary of control scenarios evaluated.

| Scenarios | Controlled Variables | Maximized Variables | Target Perfusion Rate | Manipulated Variables |
|---|---|---|---|---|
| *Scenario I* | $y_{vcd}, y_{vol}$ | NA | Yes | $F_f, F_h, F_b$ |
| *Scenario II* | $y_{vcd}, y_{vol}$ | $f_{harvest}$ | No | $F_f, F_b, F_b$ |
| *Scenario III* | $y_{vcd}, y_{vol}$ | $f_{harvest}, y_{product}$ | Yes | $F_f, F_h, F_b$, pH, Temperature |

In all three scenarios listed in Table 2, the lower bound for $f_{harvest}$ during the growth phase and perfusion phase is set to 2 L/day and 3.4 L/day, respectively. The estimation and prediction horizons are set to 5 days that gives the estimation and control scheme a long enough horizon of the historical and future information while control horizon is 2 days to avoid unnecessary computational time. Control actions are taken every two hours. In *Scenario II,* the manipulated variables are the flowrates (harvest, bleed, and feed), so no setpoint for the perfusion rate (i.e., $\Upsilon_{PR} = 0$) is used in order to keep the optimization problem feasible. Note that having a target perfusion rate means having a setpoint for the $f_{feed}$ throughout the perfusion phase. Moreover, the process model is assumed to be the true plant model (the predicted states by the CCM model described in Example 1 are assumed to be the true online measurements for $y_{vcd}$, $y_{deadcells}$, and $y_{vol}$) and the online estimation for the remaining state variables is provided by MHE.

As shown in **Figure 9,** the Autonomous Control framework resulted in a proper control under all of these different control scenarios; that is, the control variables (VCD and the reactor volume) are at their target under *Scenarios I-III* (Figure 9A) while harvest and product are at their maximum feasible values in *Scenarios II-III* (Figure 9C-D). Figures 9B and E show that for *Scenario II,* the $f_{feed}$ and *Osmolality* are at their upper process bound (6 *L/day* and 360 *mOsmol/kg,* respectively), resulting to a maximum feasible harvest flow rate of 4.56 *L/day* found by NMPC.

To avoid such a high osmolality while maximizing the $f_{harvest}$, *Scenario III* was designed with more manipulated variables available to the controller to adjust the *temperature* and *pH* as needed and so avoid the high *Osmolality*) caused by a high $f_{reed}$. The controller pushes the *temperature* to its upper operational bound, helping the cell growth while keeping the $flow_{feed}$ at the perfusion rate target with an average mean squared error of 21%.

Comparing the $f_{feed}$ for *Scenario I* and *Scenario III* shows the importance of the weights assigned to each term in the objective function (i.e., Equation 2). According to Figure 9B, the controller's focus under *Scenario I* was to keep the $f_{feed}$ at the perfusion rate target while for *Scenario III,* the controller made weighted decision to slightly compromise on the perfusion rate target to be able to maximize the final flowrate and product concentration in the harvest stream (the observed offset in the figure), as expected. In *Scenario III,* the manipulated variable *temperature* is at its upper bound (see Figure 9H), which limits further reduction in the offset between $f_{feed}$ and perfusion rate target as well as a higher $f_{harvest}$ and $y_{product}$.

These data show that the control methods described in Example 1 are able to achieve stable control of a perfusion bioprocess under a range of conditions and control scenarios. This demonstrates the stability and reliability of the approach, which are critical factors underlining its ability to further accommodate constraints and costs from the downstream process, as will be further demonstrated in Example 3 below.

## *Example 3 - Autonomous Control of Continuous Bioprocesses with Economic Modelling*

The present example demonstrates the use of a controller as described in Examples 1 and 2 which uses a control objective with a cost of goods function that models the costs in the DSP. Such an approach enables automated coordination of USP and DSP in continuous operation, can reduce costs of goods for bioprocesses, and eventually can enable fully automated operation of the compete process comprising USP and DSP for production of a bioproduct (i.e. "lights out manufacturing").

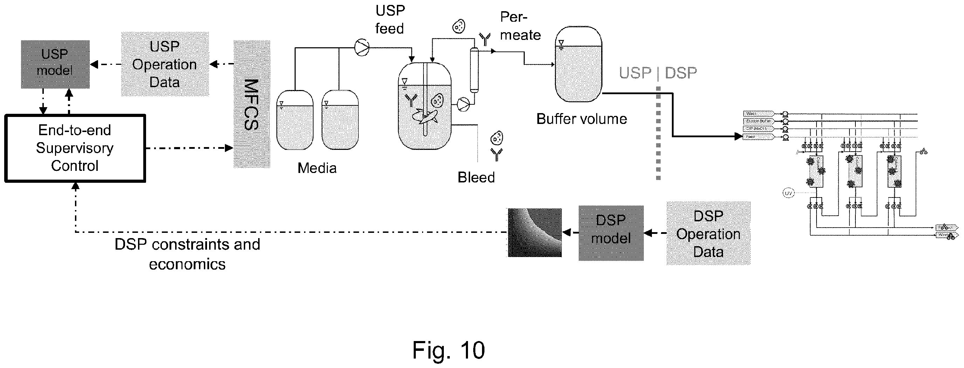

The approach demonstrated here, illustrated on **Figure 10,** used a universal chromatography model (labelled DSP model on Figure 10) as described in Example 4 below to define feasible operating regions (in terms of feed flow and titer) from the point of view of the downstream process (chromatography), as well as identify costs of goods for minimization (illustrated as the heatmap obtained from the output of the DSP model), associated with any feed flow and titer that results from a particular control strategy for the USP. As explained above, the upstream process (cell culture) is operated using dynamic optimization via a model predictive controller that searches the DSP constraint region for an optimum solution that minimizes the costs of goods associated with the DSP (via the cost of goods function), while achieving throughput requirements (through constraints on the flows and titer). In other words, the controller tries to minimize the cost of goods function subject to constraints applying to operation of the process. On Figure 10, MFCS is a manufacturing control system that handle communication between the control logic implemented in a processor, and the devices and regulatory control (DO, pH, temperature, etc.).

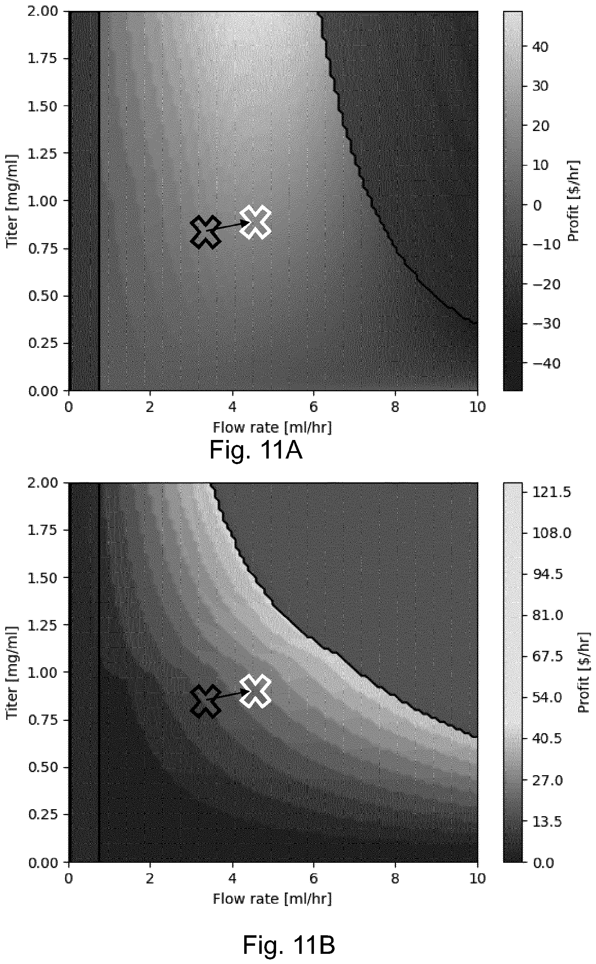

This is illustrated on **Figure 11** which shows the results of a cost of goods function calculation based on the outputs of the DSP model for two different types of static phases (as explained further in Example 5 below). The cost of goods function corresponds to the term $\sum_{i=k+1}^{k+P} Cost_{total}(F_{h_i}, \varphi_i)$ for a single product $\varphi$ (here a monoclonal antibody) calculated using an economic model that took into account the costs associated with regeneration (using a known fixed cost per regeneration associated with each column and an assumption of a fixed number of cycles lifespan for the columns, here 100 regenerations - although any numbers corresponding to the known or expected lifespan of a particular column can be used), the cost of buffers consumed in the chromatography process, the cost of waste generated through the process, and the gains associated with the amount of product recovered (assuming a price per mg of product). The costs and gains were combined into a single metric of profit in $/hour of running the process (taking into account time for running through a cycle of load-wash-elute-regeneration, buffer consumption rates, buffer preparation requirements including buffer storage costs, and amount of product recovered in each cycle). Thus, the cost of goods function can be any function that aims to optimize the costs of goods associated with the bioprocess or a part thereof including the DSP, or profits associated with these costs of goods.

The plots on **Figure 11** show the value of the cost of goods function evaluated for any titer and flow rate pair that can be selected by the USP controller, enabling the USP controller to select operating conditions (the white crosses on Figure 11) that reduce costs while increasing the total product production (in this example the USP is producing more product (g/day) at a similar titer so the overall process is making more product than the original unoptimized operating point). Further, the specific choice that is optimal can depend on the particular chromatographic process (e.g. the heatmaps in Figure 11A and Figure 11B which are associated with different static phases show that different sets of operating parameters for the DSP can have different costs).

This experiment demonstrated that there are significant opportunities to reduce the costs of goods through coordination of the USP and DSP. In particular, when modelled for a Cellca2 cell line (note that higher g/L/day are expected for other cell lines further reducing CoGs), a 35% reduction in CoGs (from 142 to 93 $/g) could be obtained by using the methods described herein compared to a state-of-the-art bioprocess control method (see **Table 3** below).

The reduction in cost in this case came from a combination of and increase in production (1.2 to 2.0 g/L/day) and more efficient DSP operation.

**Table 3.** Comparison of performance of bioprocess without (top) and with USP-DSP integration as described herein.

| Process | g/L day | Bioreact or Duration (days) | Bioreactor Scale (L) | Annual Throughp ut range (kg /year) | DSP Mode | Facility Utilizat ion | Estimated CoGs ($/gram DS) |
|---|---|---|---|---|---|---|---|
| Current Cont. CHO | 1.2 | 30 | 500 | 81 | 3XBioSMB8 0 | 70% | 142 |
| Autonom ous Control | 2 | 30 | 500 | 133 | 3XBioSMB8 0 | 70% | 93 |

## Example 4 - **Universal Chromatography Model**

### *Introduction*

The present inventors set out to solve problems associated with state-of-the-art chromatography modelling approaches, which are based on first-principles governing mass transport (i.e. capturing convection, diffusion through stagnant films, diffusion in pores, etc), are numerically unstable due to stiffness related issues in partial differential equations, require parameterisation from detailed experiments, and require modelling experts to adjust the model for each chromatography process.

They developed a chromatography modelling framework that uses a hybrid of a machine learning algorithm (neural networks are illustrated here) and physics derived equations to provide powerful predictions. The approach is directly usable by chromatography practitioners rather than modelling experts (as it does not require detailed understanding of the physico-chemical phenomena likely to influence the chromatographic process), has minimal data requirements, is computationally efficient, easy to integrate with models of other unit operations, and applies to resins, membranes, and monoliths without modification by the user.

The new approach is particularly useful in the context of continuous processing, enabling the control of an upstream process taking into account constraints and impacts on the downstream process. This includes chromatographic process optimisation, including the possibility of controlling a continuous bioprocess with economic optimisation.

Indeed, since the same approach can be used to model a chromatographic process over a wide range of operating conditions, it is possible to simulate the costs associated with different conditions in order to optimise the economics of the bioproduction process (e.g. cost per amount of recovered product).

*Universal Chromatography Model Framework Overview*

The approach described in this example combines machine learning models for intensive (volume independent) process predictions with a simple physics model that captures process scale. The architecture of both parts of the model are independent of column size or types of stationary phase (binding phase), i.e. the same architecture can be used for membranes, monoliths and resins.

The approach requires minimal amounts of experimental data, i.e. the only data needed is the data used to train the machine learning model, which is limited to breakthrough data (i.e. data about the concentration of adsorbate in the column effluent as a function of time, including at least the desired product(s) although the model can also take into account inerts - i.e. any other compound in the feed that can influence the interaction between the product(s) and the column). Note that the approach can even make use of fractionated breakthrough data instead of or in addition to time-resolved breakthrough data. This is particularly useful as in many cases detailed concentration information (particularly for compounds other than the specific desired product of the process, e.g. any host proteins or other contaminants) is only available for collected fractions of the effluent because they are collected offline.

The approach is capable of capturing complex interactions, is easy to use, is computationally efficient (sub-second execution) and works for poorly understood processes (e.g. advanced therapies) where accurate first principle models are simply not available.

The model structure in the proposed approach combines physics-based elements for bulk flow (i.e. mass balances) with a new neural network architecture for binding and diffusion, as illustrated on **Figure 4B,** using a mass balance module 20 implementing a physics-based bulk flow differential equations model (note this is an ordinary differential equations, ODE, model, not a partial differential equations, PDE, model), and a machine learning module 22 implementing a machine learning model to provide parameter estimates that are used by the mass balance module in the physics-based model. **Figure 12** shows the discretization scheme used to describe a generic chromatography column. Note that the same architecture is used regardless of the geometry of the column because the focus is on differential volume elements and the model does not explicitly model diffusion geometry. **Figure 12** illustrates schematically on the left a full chromatography column, and on the right the corresponding "stages" (also referred to as differential volume elements) represented in the model. The process receives a feed with a flow rate $f_k$ (which can be directly obtained from the upstream process, i.e. can be equal to the harvest rate $f_H$ or a proportion thereof (e.g. when the harvest flow is split between multiple downstream processes). The flow travels through an entry dead volume with volume $V_{entry}$, which is a volume that the mobile phase travels through outside of the column (i.e. prior to interacting with the stationary phase in the column). The flow then travels through a series of consecutive volumes labelled as stages 1 to N of volume $\Delta V$ (note that in the present examples all volumes $\Delta V$ are assumed to be the same for simplicity, but the volumes could in fact be different depending on the stage, in which case $\Delta V$ can be indexed with the stage number n, i.e. $\Delta V_n$ - in the equations provided below $\Delta V$ is the volume of the stage n, which can also be written as $\Delta V_n$), in which the product interacts with the stationary phase. Finally, the flow travels through an exit dead volume with volume $V_{exit}$, which is a volume that the mobile phase travels through outside of the column (i.e. after interacting with the stationary phase in the column). The mass balances governing the discrete volume elements throughout the column (stages 1 to N) are given by Equations (8) to (10):

$$\frac{d\vec{c}_{u,n}}{dt} = \frac{f_k}{\Delta V}\left(\vec{c}_{u,n-1} - \vec{c}_{u,n}\right) - \tilde{k}_a \circ \vec{c}_{u,n} + \vec{k}_d \circ \vec{c}_{b,n} \qquad (8)$$

$$\frac{d\vec{c}_{b,n}}{dt} = \tilde{k}_a \circ \vec{c}_{u,n} - \vec{k}_d \circ \vec{c}_{b,n} \qquad (9)$$

$$\frac{d\vec{c}_{i,n}}{dt} = \frac{f_k}{\Delta V}\left(\vec{c}_{i,n-1} - \vec{c}_{i,n}\right) \qquad (10)$$

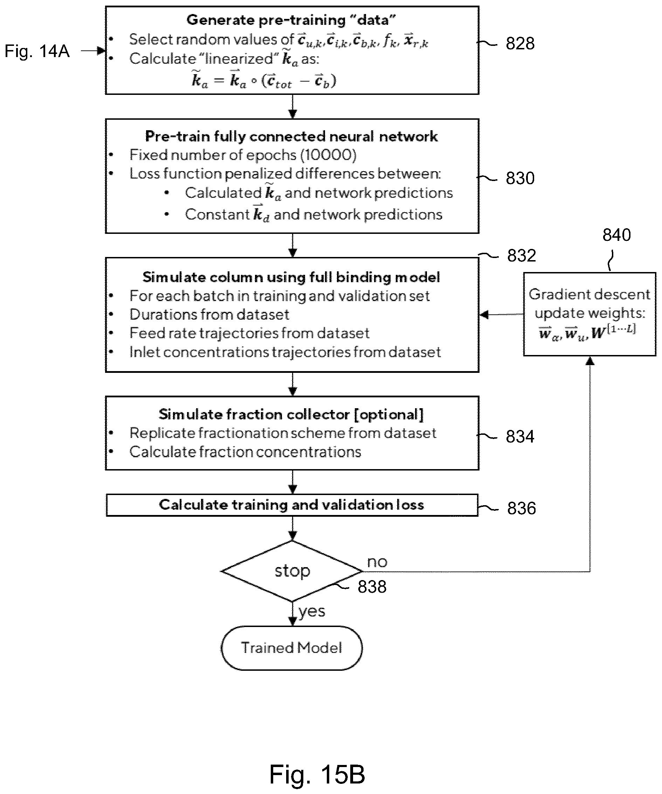

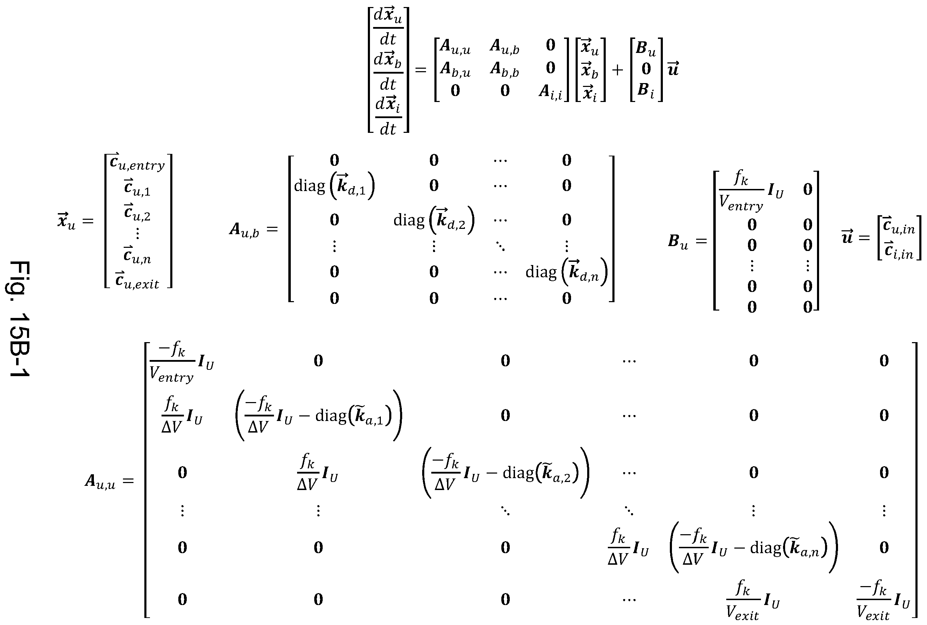

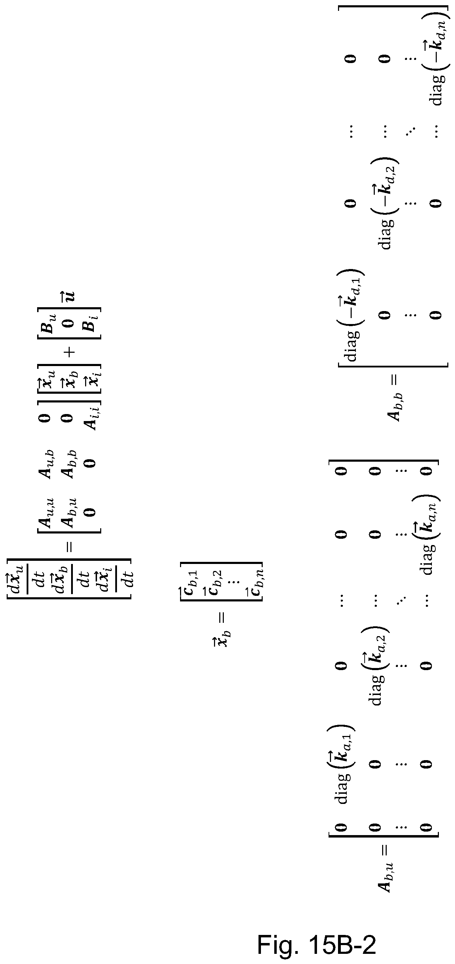

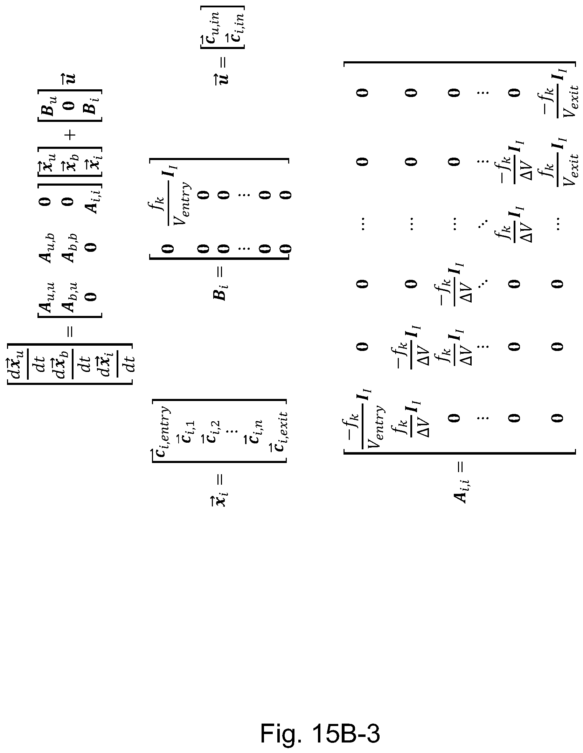

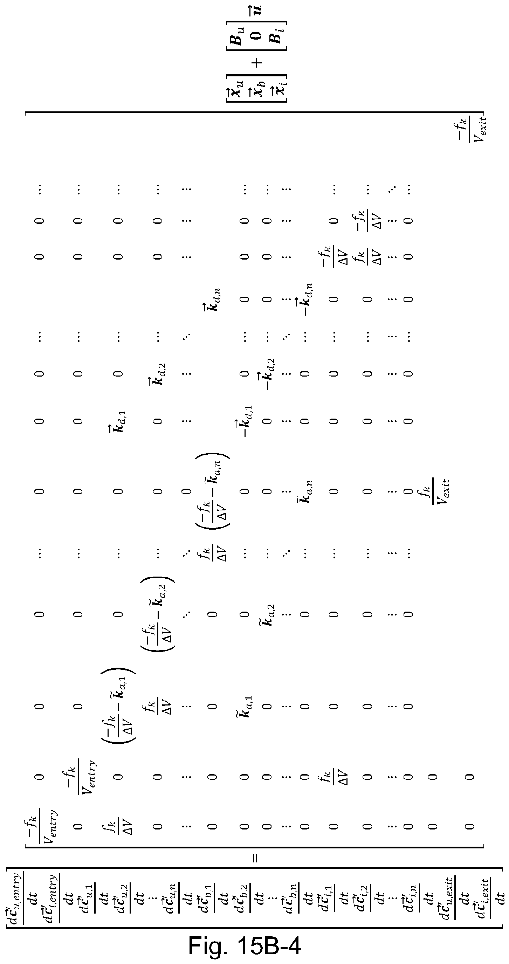

where $f_k$ is the feed flow rate, $\Delta V$ is the volume of each stage, $\vec{c}_{u,n}$ is a vector of concentrations of the unbound products in stage n (i.e. each element of the vector is a concentration of an unbound product within the current stage, i.e. within the nth volumetric element), $\vec{c}_{b,n}$ is a vector of concentrations of the bound products in stage n (i.e. each element of the vector is a concentration of a bound product within the current stage, i.e. within the $n^{th}$ volumetric element), $\vec{c}_{u,n-1}$ is a vector of concentrations of the unbound products in the stage preceding stage n (i.e. each element of the vector is a concentration of an unbound product that flows into the current stage, i.e. the $n^{th}$ volumetric element), $\vec{c}_{i,n}$ is a vector of concentrations of the inerts in stage n (i.e. each element of the vector is a concentration of in inert within the current stage, i.e. within the $n^{th}$ volumetric element), $\vec{c}_{i,n-1}$ is a vector of concentrations of the inerts in the stage preceding stage n (i.e. each element of the vector is a concentration of an inert that flows into the current stage, i.e. the $n^{th}$ volumetric element), and ∘ denotes the Hadamard product (elementwise product). In these equations that $\tilde{k}_a$ and $\vec{k}_d$ describe the overall effect, at any given sampling time, of binding and diffusion on the bulk concentration for each non-inert species in the discrete volume. The full system of equations developed across all stages is shown on **Figure 15B.**

Note that the term "product" here refers to any molecule that interacts with the stationary phase. Typically this is the desired product(s) to be purified, although it is also possible within the same framework to include additional molecules that are not desired products but that can also interact with the stationary phase to some extent (e.g. impurities). This is by contrast with an "inert" which is any compound that does not directly interact with the stationary phase but where the presence of the compound can influence the interaction of the product(s) with the stationary phase. Representing the inerts (equation (10)) is optional in this model. For example, in situations where inert concentrations are unknown (i.e. there is no inert concentrations available for training the model) and/or inerts are not believed to materially affect the behaviour of the products, the model can use only equations (8) and (9).

In the entry dead volume and the exit dead volume, all concentrations remain unchanged (the volumes are taken into account to accurately reflect an observed time delay proportional to the feed flow rate and inversely proportional to the entry and exit dead volumes). The entry dead volume and exit dead volume additionally help to ensure the numerical stability of the solutions as no step change in concentration can be provided directly to the column dynamics, since all changes in concentrations set by the user occur at the inlet (before the entry dead volume) and at the outlet (after the exit dead volume). Step changes can cause large gradients that make the discretised solutions less accurate. Inclusion of entry and exit dead volumes accurately reflect the reality of the system (since it is not possible to build a physical chromatography system with zero dead volume) so their inclusion in the model is both accurate and helpful for the numerical integration.

The values in vectors $\tilde{k}_a$ and $\vec{k}_d$ are phenomenological parameters that describe the overall effect, at any given sampling time, of binding and diffusion on the bulk concentration for each non-inert species in the discrete volume. These can capture many different physico-chemical processes that are not explicitly modelled but contribute to the overall (i.e. observed bulk level) concentrations through dynamics that manifest themselves at the bulk level as binding (i.e. governing equilibrium between overall bound and unbound products) and diffusion (i.e. movement of the bound products). In other words, the above system of equations only represents three macrolevel phenomena in discrete volumes along the column: flow of unbound products in and out of the volume (and optionally flow of inerts in and out of the volume), diffusion of bound products, and binding of unbound products to the stationary phase. The latter two are parameterized with "catch-all" parameters in vectors $\tilde{k}_a$ and $\vec{k}_d$. These vectors are calculated as the output of a machine learning model, which is described below.

As will be explained further below, the parameter $\tilde{k}_a$ is assumed to be a linearized version of a corresponding parameter $\vec{k}_a$, calculated as $\tilde{k}_a = \vec{k}_a \circ (\vec{c}_{tot} - \vec{c}_b)$, where $\vec{c}_{tot}$ is the total binding capacity of the column (also referred to as static binding capacity of the column). The parameter $\vec{k}_a$ follows a traditional second order rate law form (Langmuir adsorption isotherm, which is a isotherm often used to model chromatography). Overall binding in equations (1) and (2) can be expressed as a $\vec{k}_a \circ (\vec{c}_{tot,n} - \vec{c}_{b,n}) \circ \vec{c}_{u,n}$, where $\vec{k}_a$ describes a relationship that depends on both the number of still unbound binding sites $(\vec{c}_{tot,n} - \vec{c}_{b,n})$ and the number of unbound molecules $(\vec{c}_{u,n})$, following a Langmuir adsorption model. The use of $\tilde{k}_a$ in the equations above and as an output of the machine learning model means that we can remove $\vec{c}_{tot}$ as a parameter of the column model, which helps improve stability of the machine learning model training. This essentially makes the machine learning model responsible for accounting for the nonlinearity in the Langmuir model (since it is tasked with predicting a parameter $\tilde{k}_a$ that captures a nonlinear phenomenon, the parameter being used in linear equations (1) and (2). As will be explained further below, the relationship between $\tilde{k}_a$ and $\vec{k}_a$ is only used when pretraining the machine learning model, as thereafter all training and predictions exclusively use $\tilde{k}_a$.

The number of stages N is not limited and any number of stages can be used. In practice, there is a balance between accuracy, stability and computational efficiency. The higher the number of stages the higher the resolution of the physical (mass flow) model, the lower the burden on the machine learning model to accurately capture complex dynamics, but also the higher the chance of numerical integration running into issues of instability due to the stiffness of gradients within small volumes. Thus, the higher the number of stages the higher the computational burden to run the model but the lower the computational burden (and training data requirement) to train the machine learning model. Conversely, the lower the number of stages the lower the resolution of the physical (mass flow) model, the higher the burden on the machine learning model to accurately capture complex dynamics (since more of the accurate capture of the dynamics of the entire system is pushed from the physical model to the machine learning model), and the lower the chance of numerical integration instabilities. Similarly, the lower the number of stages the lower the computational burden to run the model but the higher the computational burden (and training data requirement) to train the machine learning model. Higher number of stages are expected to result in higher accuracy predictions but the present inventors have found that very accurate results can still be obtained with very small numbers of stages, with lower numbers of stages reducing the risk of numerical issues due to very small gradients as the value of $\Delta V$ decreases and increasing the robustness of the predictions (avoiding overfitting of the machine learning model since as explained below the same model is used to predict the binding and diffusion coefficients in each of the stages). The present inventors tested numbers of discrete volumes from 5 to 20 and all led to results with similar accuracy (although the computational efficiency decreased with increased numbers of volumes). For general use, the present inventors settled on the use of 10 volumes as likely to ensure robustness, accuracy and computational efficiency across a vast range of scenarios. However, they believe that any number of volumes preferably including a plurality of volumes (i.e. 2 or more volumes) could be used.

Note that the machine learning model is trained for a specific set of products and inerts, and a specific static and mobile phase. However, the model is independent of the geometry of the column (and takes concentrations and feed flow rate as inputs) and therefore immediately usable for scale up and assessment of the effect of variations in feed concentrations and flow rate.

The functioning of the machine learning will now be explained. In the present example, as illustrated on **Figure 4B,** the machine learning model combines a parameter prediction submodel 22B (in these examples, a neural network), and a recurrent state submodel 22A (also referred to herein as "recurrent module"). The combination is referred to herein as "recurrent neural network", although it is not a recurrent neural network in the traditional sense of a network where nodes in one layer affect subsequent inputs to the same nodes. Indeed, here a recurrent module is added which captures information from preceding states and provides an input to the neural network per se. The recurrent module is optional. The recurrent module enables to capture dynamics of the system by enabling the machine learning model to make predictions informed by the previous state of the system. The architecture of the machine learning model used in the present examples is shown in **Figure 13.** At each iteration *k of* integration of the physics based model (i.e. each time point that is being simulated), the model takes as inputs vectors of concentrations of unbound products ( $\vec{C}_{u,k,n}$ ; i.e. for each stage n and iteration k, a vector comprising the respective concentration of unbound product for each of the respective products), bound products ( $\vec{C}_{b,k,n}$ ; i.e. for each stage n and iteration k, a vector comprising the respective concentration of bound product for each of the respective products) and inerts ( $\vec{C}_{i,k,n,}$ ; i.e. for each stage n and iteration k, a vector comprising the respective concentration of inert for each of the respective inerts), and the flow rate $f_k$, for all discrete volumes *n.* In the implementation used in the present examples, data from all stages of the column are passed to the neural network in one call (although the implementation prevents the network from making use of information about other stages when making predictions for any particular stage in order to reduce the risk of overfitting). This advantageously enables to parallelize the computation, for example enabling implementation on a multi-core machine and/or GPU. Alternatively, the model could be used to predict parameters for each discrete volume n individually (which could still be run in parallel through multiple instances of the model). In either case, the machine learning model is trained to predict the parameters for each stage individually. In other words, the predictions for one discrete volume element (stage) do not depend on the predictions for another discrete volume element. Inclusion of the flow-rate variable allows the neural network to adapt to the changes in binding and diffusion effects observed in columns as flow changes. The modeling framework does not explicitly use physics-based equations derived from fluid-mechanics and binding dynamics, which is beneficial as it reduces the experimental burden of identifying the model. Instead, fluid-mechanic, binding and diffusion effects are learned from experimental data generated at different flow-rates. Because the flow variable, $f_k$, is not used by the neural network in a first-principle derived equation, this variable may be "transformed" by the user before being passed to the neural network model. Transformations such as converting from volumetric-flow to linear velocity or residence time have been demonstrated in other simulation methods to result in models that are less scale dependent. This property is useful for applications where there is a desire to identify models form experiments conducted in small columns and make predictions using those models for larger-scale (commercial manufacturing relevant) columns. The present methods have been demonstrated to be applicable to scale up settings eve without this transformation, but the transformation is simple to do in the present methods such that there is essentially only benefits to be expected from making the transformation.

The machine learning model as illustrated on **Figure 13** also takes as input a recurrent state vector $\vec{x}_{r,k,n}$ which is a state vector for each iteration *k* and discrete volume *n* that allows the vector to take into account information from one or more previous time steps captured in a "catch-all" arbitrary state. As will be explained further below, the recurrent states store values that are obtained through a nonlinear transformation of the states of the column (vectors of concentration and flow rate) at preceding iterations, where the recurrent states values are updated at every iteration based on the values of the recurrent states at the preceding iteration and the states of the column at the current iteration. As explained further below, there is in principle no limit to dimension of the elements in $\vec{x}_{r,k,n}$ (i.e. the number of recurrent states for each discrete volume element n that the model keeps track of at every iteration, i.e. the number of degrees of freedom in the recurrent state information). However, higher dimensions lead to higher complexity and potential overfitting, and the present inventors have found (see below) that 1 state was sufficient to accurately capture the dynamics of the system. Thus unless indicated otherwise a single recurrent state is used for each stage. Note that although the model can in principle take into account states from any number of preceding iterations, in practice due to the physics of the system being modelled it is expected that the benefits of taking previous states into account are mostly in taking into account states that occurred in recent iterations. Indeed, the system has no "memory" of recurrent states beyond the immediately preceding recurrent state and the recurrence is designed to promote updating these states in every iteration. Note that recurrent neural network architectures such as long-short term memory networks (LSTM) could be used instead but these recurrence structures are designed to promote longer "memories" and therefore do not appropriately reflect the dynamic behavior (physics) of the system. This distinction also has a practical impact on training since LSTMs take into account many previous states, thereby making them far less computationally efficient, and less likely to converge to robust and accurate solutions than the presently proposed solution. As illustrated on **Figure 13,** the states vector $\vec{x}_{r,k,n}$ is concatenated with the other input variables (concentration vectors and flow rate), optionally after going through a tanh function (hyperbolic tangent function) to ensure that the state value does not increase to dominate the other input variables. Note that the tanh function can be omitted or replaced by any other function that can take an unbounded input and produce a bounded output, such as e.g. tanh or logistic/sigmoid functions. The concatenated input is fed into both (a) a recurrent state submodel (722A) and (b) a parameter prediction submodel (722B). The role of the recurrent state submodel 722A is to provide an updated state vector $\vec{x}_{r,k+1,n}$ for use at the next iteration. The role of the parameter prediction submodel 722B is to provide predictions of the vectors $\tilde{k}_a$ and $\vec{k}_d$ for use at the current iteration k for a volume $n$ ($\tilde{k}_{a,k,n}$ and $\vec{k}_{d,k,n}$) associated with concentrations $\vec{c}_{u,k,n}, \vec{c}_{b,k,n}, \vec{c}_{i,k,n}$, flow rate $f_k$, and current state vector $\vec{x}_{r,k,n}$. The flow rate is a parameter of the simulation, the concentrations $\vec{c}_{u,k,n}, \vec{c}_{b,k,n}, \vec{c}_{i,k,n}$, are obtained by integration of the model provided by equations (8)-(10) (which itself uses predictions from the machine learning model for the $\tilde{k}_a$ and $\vec{k}_d$ parameters), and the state vector is predicted by the trained recurrent state submodel. The recurrent state submodel takes as input the same concatenated vector as that fed into the parameter prediction submodel 722B, and the current state vector (optionally after tanh or other bounding transformation). As illustrated, the recurrent state submodel 722A comprises two branches respectively including a sigmoid function with learnable weights vector $\vec{w}_a$ and a tanh function with learnable weights vector $\vec{w}_u$, both of which take as input the concatenated vector that is fed into the parameter prediction submodel 722B. Note that any function that can provide an output between 0 and 1 can be used instead of the sigmoid function, and any function that can provide a bounded output from an unbounded input can be used instead of the tanh function. The first branch with learnable weights vector $\vec{w}_a$ produces as output a factor $\alpha$ which can be seen as a "forgetting factor" and represents a probability that the state vector is not updated at the current iteration. The lower the value of alpha the more likely the current state is updated (i.e. the higher the value of $(1-\alpha)$ the more likely the current state is updated - note that $\alpha$ could be used instead with the opposite consequences). The second branch with learnable weights vector $\vec{w}_u$ determines a proposed update to the current state vector. If $\alpha=1$, the current state (illustrated as "x" on **Figure 13)** is simply used as it is for the next iteration (i.e. no update, top branch evaluates to 0). If $\alpha<1$, the current state (illustrated as "x" on the top branch of **Figure 13)** is updated to a value that is the sum of $(1-\alpha)x$ where x is the current state (i.e. current state multiplied by $1-\alpha$) and $\alpha x$ where x is an updated state that is output by the tanh function with learnable weights $\vec{w}_u$. In other words, a new updated state vector is obtained as a weighted sum of the current state and a new proposed state, with weighting factor $(1-\alpha)$ and $\alpha$, respectively for the current and proposed new states. The parameter prediction submodel 722B is illustrated here as a simple fully connected neural network with parameters $W^{[1...L]}$ which are weights associated with each neuron in each layer L. The weights $\vec{W}_a$, $\vec{W}_u$, and $W^{[1...L]}$ are all learned together using a training procedure that will be described below.

The recurrence structure in the proposed neural network was designed specifically for this application. It is dissimilar to recurrent neural networks from other fields, for instance the most common long shot-term memory (LSTM) structure. In essence, this application of the recurrent network removes the "long memory" part of the LSTM structure since it is very unlikely that a state observed many sampling instances in the past would influence the current dynamics of the system. Instead, the proposed structure can be viewed as adding "free" states which evolve with nonlinear dynamics determined by the neural network. It is left to the training algorithm to determine how to best apply these new nonlinear dynamics. The use of recurrent states enables to capture dynamic behavior caused by diffusion. This diffusion effect may occur in binding substrates, mobile phase, or other parts of the pathway between the bulk liquid phase and binding sites within the column. Many prior art models explicitly or implicitly ignore diffusion effects, which are hard to model with first principles models.

An important benefit of the proposed approach is that the same training weights are used by the neural network at every discrete volume in the spatial discretization of the column. In other words, the vectors in in $\overline{w}_a$, $\overline{w}_u$, and $W$ are not indexed by n. Practically, the implication of this decision is that the dynamic response of the column should be the same at the inlet that it is at the outlet. This design decision is technically accurate for new columns where the stationary phase should be homogeneous throughout the column volume. In older columns, it is possible that this assumption would be less accurate due to irreversible binding effects which may disproportionately influence the inlet portion of the column. This can be addressed by providing to the machine learning model one or more additional inputs (additional states) that are indicative of the age of the column. For example, the machine learning model can additionally take as input a variable indicative of the number of cycles (i.e. number of complete load, wash, elute, regenerate cycles) that the column has experienced, or a variable indicative of the total number of column volumes that the column has experienced. Instead or in addition to this, the machine learning model can be trained using training data comprising data for a plurality of complete cycles, enabling the model to learn how the column parameters change over time. However, there are substantial advantages to training a single binding dynamic model for the whole column in terms of greatly reducing the likelihood of overfit. This approach parallels techniques developed in image analysis using tied weights in convolutional neural networks. In both cases, by training the weights on "sliding views" of the same system, localized anomalies are down-weighted and a more representative set of weights emerges in the training process.

*Model training*

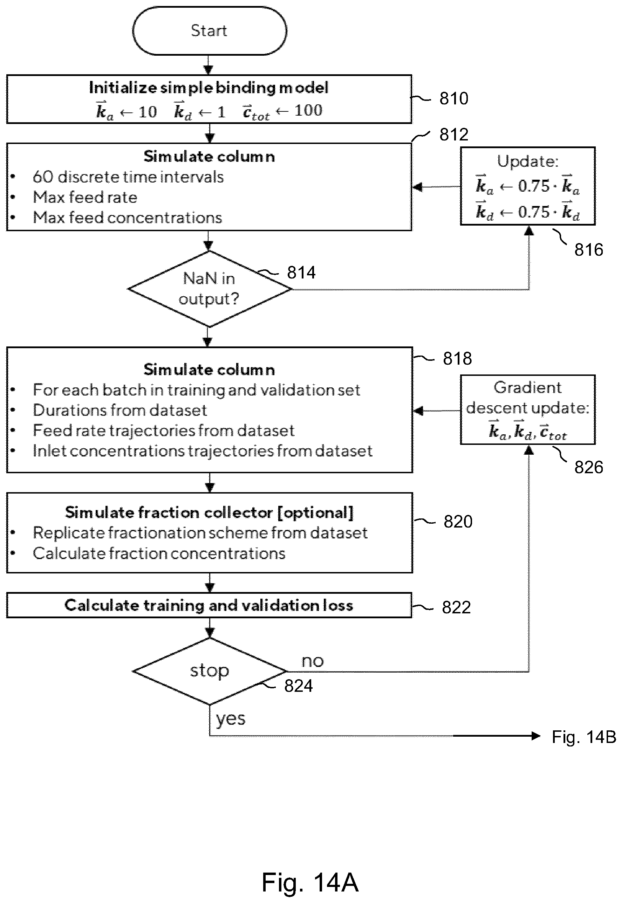

A training algorithm was developed by the inventors which enables reliable training of neural networks for binding kinetic behavior from commonly available, column eluate (outlet) data, including experimental data collected using a fraction collector (fractionated) on the column outlet stream. This algorithm is believed to be novel in at least three ways. First, the approach directly accounts for the aggregation of samples that occurs through fractionation in common, lab scale, chromatography experiments during product development. Ability to use fractionated sample data makes the proposed technique practically viable compared to alternatives that require complete time-series concentration data. Second, the inventors have developed a series of heuristic techniques for training the proposed recurrent neural network in stages to achieve numerically stable gradient descent during the training phase. Finally, the inventors have developed a method for linearizing and discretizing spatial dimensions (through the use of the differential volume elements model described above) and time dimensions (through the use of discretized time steps corresponding to iterations of the process of machine learning model prediction and solution of the column model ODEs) in the problem. This discretization algorithm removes the need for application of adaptive step-size partial differential equation solvers. As a consequence, the algorithms proposed are readily implementable in computational graph environments, like PyTorch, such that loss function errors can be backpropagated through the physics equations, to the neural network, and ultimately the training weights. The complete training algorithm flow sheet is shown on **Figure 14.**

At step 810, the model is initialized using default values for $\vec{k}_a$ and $\vec{k}_d$ (here illustrated as 10 and 1, respectively, for all n stages) and for $\vec{c}_{tot}$ (here illustrated as 100). The parameter $\vec{c}_{tot}$ is a vector of the respective total binding capacity (static binding capacity) of the column for each of the respective products. This parameter is only explicitly used in the pretraining steps illustrated on **Figure 14** as steps 810-830. At step 812, the column is simulated by solving the column model provided by equations (8)-(10) using these default values and the maximum feed concentration and maximum feed rate available in the training data. In the illustrated embodiment, the model is iteratively solved over 60 discrete time intervals. The length of time corresponding to any number of intervals k depends on the temporal discretization frequency (i.e. length of time corresponding to an iteration k), which is a user defined parameter (as is the total number of iterations). The present inventors have found that for most reasonable temporal discretizations, 60 sampling instances was long enough to observe any instability in the model since the most numerically unstable time in most column loading data comes early in the run since that is when concentrations are changing the most and therefore the gradients (stiffness) of the problem is highest. Therefore, for this pretraining step 60 iterations was found to be sufficient. Note that in practice when simulating a column (i.e. not in model training), the model can be allowed to run for as many iterations as necessary to capture the behaviour of the column that the user is interested in (e.g. full loading phase until a plateau in the breakthrough curve is reached). At k=0, initial values of $\vec{c}_{u,k,n}$, $\vec{c}_{b,k,n}$, $\vec{c}_{i,k,n}$ are assumed. These are typically 0 for the bound and unbound products (as when simulating a loading phase the column has not yet been loaded with product), and either 0 or constant values for any inerts that are present in the buffer initially filling the columns. These concentrations (concentrations of any inerts modelled in the buffer initially filling the column) are known for a particular chromatography process to be modelled. When modelling a wash or elution phase, the initial values of $\vec{c}_{u,k,n}$, $\vec{c}_{b,k,n}$, $\vec{c}_{i,k,n}$ can be obtained from simulation of the preceding phase. At step 814, it is checked whether the simulated output contains any NAs, i.e. whether the numerical integration ran into instability issues due to stiffness of gradients in the volume elements over the chosen time intervals. In the affirmative, the current default values for $\vec{k}_a$ and $\vec{k}_d$ are reduced by a predetermined factor, here illustrated as 25% (although any other value can be used), at step 816.

Steps 812, 814 and 816 are repeated iteratively until the current values for $\vec{k}_a$ and $\vec{k}_d$ are such that the model converges for all time points (i.e. there are no longer any instability issues). At step 818, the column is simulated using the values for $\vec{k}_a$ and $\vec{k}_d$ obtained at the last iteration of steps 812, 814 and 816, for each data set in the training and validation data, using a number of discrete time intervals corresponding to the durations from the respective data sets, with feed rate trajectories from the respective datasets and inlet concentrations trajectories from the respective datasets. If any of the training and validation datasets are fraction data, the corresponding simulations obtained at step 818 are converted to fraction results at step 820, by replicating the fractionation scheme from the respective data set (i.e. combining the volumes of effluent corresponding to the respective fractions) and calculating the corresponding concentrations. Note that this is optional and only performed if the data contains fraction data. At step 822, a training and validation loss (all losses shown are mean square error, MSE, although root mean square error, RMSE has also been used with the same results) are calculated as the difference between the simulated and observed data from the training set and validation set, respectively. At step 824, one or more stopping criteria applying to the losses calculated at step 822 are evaluated. In the present examples the stopping criteria included a criterion on the validation loss (i.e. validation loss was used for early stopping). In the present examples, the validation data was also used for learning rate scheduling, where the learning rate is adaptively reduced based on the rate of improvement of the validation data. This is optional and the model could also be trained using the training data alone. When one or more of the stopping criteria are satisfied, $\vec{k}_a$, $\vec{k}_d$ and $\vec{c}_{tot}$ are set to the values used at the current iteration. While the stopping criteria are not satisfied, a gradient descent procedure is used to obtain updated values of $\vec{k}_a$, $\vec{k}_d$ and $\vec{c}_{tot}$ at step 826. These are then used in a further iteration of steps 818, 820, 200 and 824.

As a result of the procedure explained above, "warm start" values of $\vec{k}_a$, $\vec{k}_d$ and $\vec{c}_{tot}$ are obtained. Specifically, steps 810-816 serve to ensure that the process of identifying starting values for $\vec{k}_a$, $\vec{k}_d$ and $\vec{c}_{tot}$ starts with initial guesses that do not lead to numerical instability when integrating the column model (simply by reducing the value of these parameters until small enough gradients occur in the model for it to be solvable at all iterations). This is performed on a single default setting (which can be informed by the training / validation data) and is therefore extremely computationally efficient. Steps 818-826 then serve to provide starting values that are informed by the training and validation data, by exploring a space of values that is unlikely to lead to numerical instability. This is less computationally efficient than steps 810-816 because it requires a separate simulation for each set of operating parameters in the training and validation data, but is still highly computationally efficient compared to steps that also update the weights of a machine learning model at each iteration. The resulting values are used to provide a "warm start" to the training of the machine learning model, which ensures that model training converges every time. Note that steps 810-826 (and steps 828-830 through which weight values for the machine learning model corresponding to these warm start values are obtained) can be omitted and the machine learning model can be trained with default weights $\overline{W}_a$, $\overline{W}_u$, and W[1...L] (which can be e.g. all the same value, random values, or values from a previously trained model if available). This is essentially equivalent to performing only steps 810 and 832-840. However, this has a much higher likelihood of the model failing to converge because of numerical instability.

At step 828, pre-training data is generated by randomly sampling values of $\vec{c}_{u,k,n}$, $\vec{c}_{b,k,n}$, $\vec{c}_{i,k,n}$, $f_k$ and $\vec{x}_{r,k}$ and calculating a linearized value of $\vec{k}_a$ ($\tilde{k}_a$) based on the $\vec{k}_a$ and $\vec{c}_{tot}$ obtained from steps 810-826, by calculating $\tilde{k}_a = \vec{k}_a \circ (\vec{c}_{tot} - \vec{c}_b)$. The random sampling is performed within respective ranges for each of the values defined based on the range of values seen in the forward pass steps of the initial fit at step 818. For example, ranges calculated as $\pm 20\%$ (or any other % such as 10, 15, 20, 25, or 30%) of the ranges seen in the forward pass steps of the initial fit at step 818 can be used. As explained above, the use of $\tilde{k}_a$ instead of $\vec{k}_a$ and $\vec{c}_{tot}$ reduces the number of parameters coming out of the neural network, reducing the number of possible solutions, which helps the solver converge, and including the relationship between $\vec{c}_{tot}$ and $\vec{k}_a$ in the neural network through $\tilde{k}_a$ makes the solution a lot more stable and computationally tractable. At step 830, the pre-training data obtained at step 828 is used to pretrain the neural network (in the illustrated embodiment a fully connected neural network and recurrent module) using a fixed number of epochs (e.g. 10000 although other numbers can be used), and a loss function that penalizes: (i) differences between the $\tilde{k}_a$ from step 828 and network predictions for $\tilde{k}_a$, and (ii) differences between the $\vec{k}_d$ from steps 810-826 and network predictions for $\vec{k}_d$. In essence, step 830 serves to train the model to provide predictions that match the "warm start" predictions obtained through steps 810-826. The corresponding pretrained weights $\overline{w}_a$, $\overline{w}_u$, and W[1...L] are then further trained using the training data and a back propagation strategy at steps 832-840. In particular, the column model is simulated at step 832 using the $\tilde{k}_a$, $\vec{k}_d$ values from steps 810-826, for each data set in the training and validation data, using a number of discrete time intervals corresponding to the durations from the respective data sets, with feed rate trajectories from the respective datasets and inlet concentrations trajectories from the respective datasets. If any of the training and validation datasets are fraction data, the corresponding simulations obtained at step 832 are converted to fraction results at step 834, by replicating the fractionation scheme from the respective data set (i.e. combining the volumes of effluent corresponding to the respective fractions) and calculating the corresponding concentrations. Note that this is optional and only performed if the data contains fraction data. At step 836, a training and validation loss (MSE, although again RMSE or other losses would produce the same results) are calculated as the difference between the simulated and observed data from the training set and validation set, respectively. At step 838, one or more stopping criteria applying to the losses calculated at step 836 or the gradient applied to the weights at step 840 are evaluated. When one or more of the stopping criteria are satisfied, the weights $\overline{w}_a$, $\overline{w}_u$, and W[1...L] are set to the values used at the current iteration, i.e. a trained model is obtained. While the stopping criteria are not satisfied, a gradient descent procedure is used to obtain updated values weights $\overline{w}_a$, $\overline{w}_u$, and W[1...L] at step 840 based on the training loss. As explained above, the validation loss is used both as part of an early stopping criterion, and for learning rate scheduling, where the learning rate is adaptively reduced based on the rate of improvement of the validation data. This is optional and the model could also be trained using the training data alone.

Solving of the column model (at steps 818 and 832) is explained in the "model deployment" section below, and follows the same principles at model training time and model deployment time. To solve the column model, at each discrete time point, k, the column model is discretized based on the current state (concentrations and recurrent state) of the system, as explained below. In particular, the concentrations and recurrent states are put through a forward pass of the neural network (722B) and the output values from that network ($\tilde{k}_a$, $\vec{k}_d$) are then plugged into the ODE equations of the column model, which is solved over a discrete time step.

*Model deployment*

**Figure 15** illustrates the method used to deploy the above described model, i.e. to simulate a column using a trained hybrid model as described above. **Figure 15A** illustrates the general principles applied. First, predictions for $\tilde{k}_{a,n}$, $\vec{k}_{d,n}$, and $\vec{x}_{r,k+1,n}$ for all $n \in [1, \cdots, N]$ are obtained using the machine learning model (here the neural network described above) and inputs: $\vec{x}_{r,k,n}$, $\vec{c}_{u,k,n}$, $\vec{c}_{b,k,n}$, $\vec{c}_{i,k,n}$, $f_k$. These $\tilde{k}_{a,n}$, $\vec{k}_{d,n}$ are used as parameters of the model in equations (8)-(10), to solve the column model at the current discrete time point k. In particular, the ODEs in (8)-(10) are linearised around the current state (defined by $\vec{C}_{u,k,n}, \vec{C}_{b,k,n}, \vec{C}_{i,k,n}, f_k$). In both training and subsequent application of the model, the recurrent states $\vec{x}_{r,k,n}$ are initialized at 0 (i.e. they are 0 at k=0). Since these states are arbitrary, that is a safe assumption as long as the training and application are consistent. As explained above, $\vec{C}_{u,k,n}, \vec{C}_{b,k,n}, \vec{C}_{i,k,n}$ are initialized respectively to 0 for the bound and unbound products, and 0 or a known concentration in the buffer filling the column, for the inerts. It is also possible to initialize these values to any known concentrations (e.g. from previous phase simulations or for situations in which some amount of irreversible binding is expected such that $\vec{C}_{b,k,n}$ is not 0 at k=0).

The matrices on **Figure 15B** show how the linearized system dynamics and linearized states are constructed. Once linearized (by obtaining the predictions from the machine learning model, which capture all nonlinearities, i.e. given a specific prediction of $\tilde{k}_a$, $\vec{k}_d$ for a current time step, equations (8)-(10) are linear, although formally (i.e. when considered not in the context of a single iteration with fixed predicted values for each of these parameters) they are nonlinear since these parameters change over time in a nonlinear manner), the system is solved over the discrete interval by calculating a discrete state-space model:

$$\left(e^{\begin{bmatrix}A & B\\0 & 0\end{bmatrix}}\right)^T = \begin{bmatrix}A_d & B_d\\0 & I\end{bmatrix} \qquad (30)$$

where A and B are the matrices shown in the top part of Figures 15B1-15B3, and developed on **Figure 15B-4,** i.e.:

$$\begin{bmatrix}\frac{d\vec{x}_u}{dt}\\\frac{d\vec{x}_b}{dt}\\\frac{d\vec{x}_i}{dt}\end{bmatrix} = A\begin{bmatrix}\vec{x}_u\\\vec{x}_b\\\vec{x}_i\end{bmatrix} + B\begin{bmatrix}\vec{c}_{u,i,n}\\\vec{c}_{i,i,n}\end{bmatrix} \text{ where } A = \begin{bmatrix}A_{u,u} & A_{u,b} & 0\\A_{b,u} & A_{b,b} & 0\\0 & 0 & A_{i,i}\end{bmatrix}, B = \begin{bmatrix}B_u\\0\\B_i\end{bmatrix} (8')$$

(referred to as equation 8' although this equation actually includes all of equations (8), (9) and (10)).

Having calculated $A_d$ and $B_d$ as the results of the matrix exponential in equation (30), it is trivial to calculate the updated state for one or more intervals (until the linearization has substantially changed) using:

$$x_{k+1} = A_d x_k + B_d u_k \qquad (31)$$

where xk+1 is any updated state (i.e. any of $\vec{C}_{u,k+1,n}, \vec{C}_{b,k+1,n}, \vec{C}_{i,k+1,n}$) and x is the corresponding current state (i.e. any of $\vec{C}_{u,k,n}, \vec{C}_{b,k,n}, \vec{C}_{i,k,n}$). By default, the values of $A_d$ and $B_d$ can be recalculated at every iteration. However, as calculating equation (4) is computationally expensive compared to the rest of the solving process, it is possible to increase computational efficiency significantly by calculating the values of the A and matrices at each iteration, comparing them to the previous iteration, and reusing the values of $A_d$ and $B_d$ from the previous iteration to calculate $x_{k+1}$ at the current iteration if the values of A and B have not changed by more than predetermined thresholds (i.e. the same $A_d$ and $B_d$ can be used to calculate updated states for a plurality of subsequent intervals, each associated with values of A and B that are within predetermined ranges from the values of A and B from which the values of $A_d$ and $B_d$ were calculated).

Interestingly, the inventors have observed anecdotally that any error introduced by the linearization scheme (particularly in the spatial dimension - i.e. any errors introduced by the discretization in stages) can be easily accounted for by the learned dynamics of the recurrent neural network. In other words, the approach is robust to the number of stages used. At the same time, the physics-based structure of the flow model prevents the data-driven components of the model from producing invalid outputs (such as negative concentrations).

## Example 5 - Validation of the Universal Chromatography Model

The use of the approach described in Example 4 has been extensively validated on a variety of datasets.

A practical use case of the approach described in Example 4 is to compare different chromatography technologies, such as e.g. different stationary phases (e.g. membranes, monoliths and resins), for example to control an upstream process taking into account the impact of upstream process output on the specific downstream process being used.

Because the framework is agnostic to the type of stationary phase (which effects are captured in the training of the machine learning model), it is possible to accommodate (and therefore also compare, if desired) different technologies directly within the same modelling framework (albeit using respective machine learning models trained using training data acquired using the respective technologies).

The present inventors demonstrated this potential by training two machine learning models (both comprising a neural network with the architecture on **Figure 13,** where the prediction submodel 722B is a fully connected neural network with 2 hidden layers each comprising 20 nodes, and the final layer comprises 2 notes each with a tanh activation function, respectively for prediction of $\tilde{k}_{a,k,n}$ and $\vec{k}_{d,k,n}$) on data from columns for purification of protein A using different stationary phases, namely MabSelect™ SuRe chromatography resin from Cytiva and Sartobind® Rapid A Membrane chromatography. Each machine learning model was trained as explained in example 1 for 1000 epochs using 4 experimentally determined breakthrough curves obtained with the respective chromatograph technology. The models were trained in approximately 3 minutes on an Intel I9 14900K processor. They were then used to simulate the columns using the same parameters, i.e. feed rate=2.5 ml/min, feed concentration=1, simulation duration=200 minutes, breakthrough threshold % =5.

**Figure 16** shows the results of this experiment for **(A-B)** a MabSelect™ SuRe Resin chromatography (calculated breakthrough time=53.77 min, calculated dynamic binding capacity=134.42 mg/ml) and **(C-D)** Sartobind® Rapid A Membrane chromatography (calculated breakthrough time=24.10 min, calculated dynamic binding capacity=50.20mg/ml). A and C show the results of the simulation, and B and D show model validation data and associated $R^2$ and RMSE between predicted (solid lines) and observed (points) breakthrough curves.

The models used to generate the data on **Figure 16** were then used to exhaustively estimate the economic impact of different recipes using the respective chromatography platform. In particular, the two chromatography platforms were simulated for a grid of flow rate values and titer values (concentration of the product in the feed). Note that the columns have the same volume so this is a direct comparison of the two platforms for the same operating parameters and mobile phases. As explained above, the costs and gain associated with each recipe were then estimated as explained above.

**Figure 11** (discussed above) shows the results of this experiment: the heatmaps show the profit associated with running a chromatographic process with the indicated values of titer and flow rate, using either a MabSelect™ SuRe Resin chromatography column (A) or a Sartobind® Rapid A Membrane chromatography column **(B).**

The inventors further tested the methodology in the context of obtaining predictions for multi-column chromatography systems from batch data. This allows a process development team, without access to a lab-scale multi-column setup, to predict how a multi-column chromatography device would improve on traditional batch chromatography. So called "continuous" chromatography using multiple columns connected in series during the loading phase of preparative chromatography is a relatively new technology in biomanufacturing. In other words, instead of the traditional batch chromatography in which the product is loaded into one column, washed and then eluted, in multi-column chromatography columns are placed in series. The product is loaded onto the first column and the breakthrough is captured onto the second column, then when the first column is saturated (or at a predetermined % of product breakthrough) the feed switches to directly load column 2 (which breaks through onto column 3). While column 2 is being loaded, column 1 is washed, eluted and regenerated. Once column 1 is ready again and column 2 is saturated (or at a predetermined % of product breakthrough), column 2 is washed, eluted and regenerated and the feed switches to column to column 3, while the breakthrough from column 3 goes to column 1. There are readily understandable theoretical advantages to using continuous chromatography systems. Perhaps the strongest of these arguments is the ability to use the complete column binding capacity without losing product during the loading phase. However, despite these advantages, adoption of continuous chromatography technology into commercial biomanufacturing has been limited. One of the implementation challenges preventing adoption of this technology is the relatively more complex task of designing recipes for multi-column systems compared to traditional, single-column, batch chromatography. Modeling tools are a potential solution to this adoption problem. Simple tools have been designed to aid in use of continuous chromatography systems, such as the Resolute® BioSC and Resolute® BioSMB platforms from Sartorius Stedim Chromatography Systems Ltd. These typically rely on simple, column capacity calculations to help determine durations for each step in the multi-column recipe. While these tools have been helpful for simple, traditional use-cases (e.g. protein A resin columns for mAb capture), they may not correctly predict performance for advanced columns (e.g. resin columns, monoliths, etc) and advanced therapeutics (e.g. viral chromatography applications). Furthermore, these tools typically do not accurately account for the impact of varying feed-rates, feed-titers, and impurity profiles. In most cases, this means that recipes generated are at best starting points, from which additional experimentation is needed to identify final recipes. The flexibility and broad applicability of the universal column modeling proposed in this work makes it an attractive alternative for use in multi-column recipe design. Furthermore, the computational efficiency of the proposed method makes it suitable for use in numerical optimization to automate multi-column recipe design.

To model a multi-column chromatography system, two instances of the model were connected such that the outlet concentration of the first simulated column becomes the feedstock of the second column. In this way, the mobile phase concentration of the multi-column system can be forecast for different recipe parameters. The model used was a 2-layer deep network with 30 activation functions (i.e. 30 nodes) per layer. Two recurrent states were used. The model was trained on batch breakthrough curve experiments in a resin protein A column (a 5 ml Cytiva MabSelect™ column). The product of interest was a monoclonal antibody. The model was trained with three breakthrough curves at differing feed rates (1.5 ml/min to 10 ml/min) and feed concentrations in the range of 0.8 to 1 g/L. The validation of the model was done with two additional breakthrough curves at novel feed rates and concentrations. Fractions for these experiments were collected in 1 ml volumes. The fraction titers were determined through size exclusion chromatography (SEC). The parameters varied through recipe design were: feed rate (ml/min), feed cone (g/L), simulation duration (minutes), and switching time (i.e. how long to load each column from fresh feed) (minutes). The inventors designed an interface through which the user can manipulate loading time, feed rates, and titers by changing sliders to simulate different conditions which could be experienced in production and develop a multi-column recipe robust to these variations. The results of the simulation (concentration of the product in the effluent of the columns as a function of time) also enable the calculation of the total captured amount (calculated as the sum of $c_b$ over all stages at the end of loading of the column multiplied by $\Delta V$, before regeneration occurs - this assumes that the loaded product will be completely recovered in the elution phase), binding capacity per cycle (calculated from the total captured amount for the cycle as total amount captured divided by (number of cycles * volume of the column)), percent recovery (calculated from the total captured amount as total captured amount divided by integrated concentration in the feed over the loading phase), maximum breakthrough concentration (maximum concentration of the product observed at the outlet of the last column in series, over the simulation) and maximum percent breakthrough (ratio of the maximum breakthrough concentration and feed concentration).

The inventors also extensively evaluated the robustness of the methods described in Example 4 for robustness (testing the effects of various parameters of the machine learning model training on the performance of the method using training data collected from purification process on a protein A column, including breakthrough curve experiments conducted at three different feed rates with multiple replicates at each feed rate, with simulations performed with a time discretization interval of 1 minute). This work showed that:

(a) having more than 1 recurrent state (where recurrent states are sum parameters remembering past states of the column during a simulation, as explained above) does not result in significant differences between the metrics derived from the simulations. This shows that 1 recurrent state is sufficient, although more could be included with the main cost likely to be computational efficiency (and potential overfitting in some cases).

(b) ML models can provide robust predictions regardless of the exact configuration of the neural network although models with 1 or 2 layers showed very similar results which were slightly better than 4 layers (testing models trained using 1 recurrent state, each comprising a prediction submodel that is a fully connected neural network with 4 hidden layers each with from 8 to 60 nodes, and tanh activation functions in the last (2 nodes) layer; and testing models each comprising a prediction submodel that is a fully connected neural network with 1, 2 or 4 hidden layers each with from 8 to 60 nodes, and tanh activation functions in the last (2 nodes) layer).

(c) The proposed approach is extremely robust to the amount of training data available (and in particular easily supports low resolution training data such as fractionated sample data) - shown by training ML models using 1 recurrent state, each comprising a prediction submodel that is a fully connected neural network with 2 hidden layers each with 20 nodes, and tanh activation functions in the last (2 nodes) layer, trained using training data with various levels of granularity (number of data points) obtained by downsampling available online data (1000x downsampling, 500x downsampling, 100x downsampling), where having around 10 points describing the breakthrough curve was sufficient to find a meaningful model.

(d) The proposed approach can be trained with minimal amounts of training data replicates and is able to learn from variability between replicates when replicates are available - shown by training models using 1 recurrent state, each comprising a prediction submodel that is a fully connected neural network with 2 hidden layers each with 20 nodes, and tanh activation functions in the last (2 nodes) layer, trained using training data comprising either 1 or 3 replicates.

Further, the inventors also extensively validated the method using data from a state of the art detailed first principles modeling tool. In particular, the inventors performed an exhaustive validation of the new methods against CADET (mechanistic) simulated data. CADET (Chromatography Analysis and Design Toolkit, von Lieres & Andersson 2010) is an open-source chromatography simulation platform available at cadet.github.io/master/index.html. CADET implements a detailed general rate model of column liquid chromatography. The software was used to set up a data-generation pipeline for numerical simulation model and subsequent validation with the universal chromatography model described in Example 1. The data pipeline stages included the following steps: (i) store user defined design space as sets of parameters; (ii) create numerical models in CADET from sets of parameters; (iii) integrate column outlet results to fractions; (iv) save fraction data to excel sheet for use by the universal chromatography model. The data was then used to train and validate the universal chromatography model.

A trial run was performed to show the ability of the universal chromatography model to extrapolate to flow rates unseen in the training data. By showing the error of the model solution compared to the validation data the inventors established that the model learns the underlying physics of the synthetic data and is able to predict mass transport of unseen flow rates. Multiple numerical models were set up with the following settings: a modulated Langmuir isotherm was used as the binding model, axial dispersion was sampled from the values $\{1*10^{-7}, 1*10^{-5}, 1*10^{-4}\}$ m$^2$s$^{-1}$ (low, medium and high axial dispersion), column volume was sampled from the values $\{1, 5, 15, 75, 200\}$ ml, length to volume ratio was sampled from the values $\{2.5, 5, 7.5\}$, column flow rate was sampled from the values $\{0.05, 0.25, 1.0, 2.5, 7.5\}$ml·min$^{-1}$ and loading time, column capacity, adsorption rate, desorption rate constant (excluding modulation effects). A collection of experiments was built using the Cartesian product of all parameters sets. The flow rate in each experiment was then scaled by a set $\{2.0, 3.0, 4.0, 5.0, 6.0\}$ to generate 5 time series per experiment. The time series were fractioned and collected in fractions corresponding to $\{30, 60, 120\}$s, to add some variation to amount of data points collected in the sets. For each experiment (5 time series), the universal chromatography model was trained on four timeseries and validated on a final series.

PDF reports were then automatically generated with results after mechanistic (CADET) and universal chromatography model training for training and validation sets. A selection of images from data generation on bottom half.

When evaluating the new numerical simulation method, the inventors looked for effects of steep gradients or "stiffness", effects of grid refinement, stability or oscillatory behavior as well as vector norms and their convergence. Here since the method being evaluated is a hybrid approach comprising a machine learning model (neural network), they also evaluated effects of parameters related to the neural network.

Based on the experiments described in Example 3, the inventors already postulated that larger network size (e.g. 10 or more nodes per layers over two layers) is beneficial for reducing loss. All experiments were therefore conducted with a model with 2 layers and 20 nodes per layer. They further added a specific fit for column capacity parameter in the mechanistic model.

The results of these extensive experiments (180 experiments in total) are summarized on Figure 18. This shows histograms of losses across validation and training datasets (respectively top left and top right panels), and distributions of losses across validation and training datasets (respectively bottom left and bottom right panels) illustrated as boxplots. These show that the vast majority of the results have a validation loss below 0.1 (10-'). The validation loss median is $4.3 \cdot 10^{-3}$, indicating that the universal chromatography model is able to recapitulate the behaviour of the much more complex and less computationally efficient general rate model with extremely high average accuracy. In general, the inventors also observed that more datapoints (here realized as higher fractionation frequency) yielded higher accuracy for the model, indicating that the method is capable of extremely high accuracy with good training data. Stiffness was not an issue with these settings as no instability or oscillatory behavior was observed after training meaning that these effects, if apparent, are nullified by the action of the machine learning model (i.e. the overall model is stable and does not have oscillatory behaviour problems). There were some outliers in terms of validation and training loss, the majority of which could be explained by the dataset including parts of the wash phase (which is not what the particular universal chromatography model tested was designed to capture since the machine learning model used was trained only on loading phase data). Detailed analysis of specific examples with a range of loss levels showed that the universal chromatography model was able to provide extremely accurate simulations (as noted above, this level of accuracy is far more common amongst all experiments than the few outliers with lower accuracy, all of which can be explained by either data generation problems or edge cases). The accuracy was higher for the training data than the validation data (which is an unseen flow rate), indicating that even higher validation accuracies could be obtained when evaluating on data that is within the design space of the training data (rather than using a parameter outside of the range of parameters that the model was trained on).

*References*

All references listed below and any documents mentioned in this specification are incorporated herein by reference in their entirety.

Shekhawat LK, Rathore AS. An overview of mechanistic modeling of liquid chromatography. Prep Biochem Biotechnol. 2019;49(6):623-638. doi: 10.1080/10826068.2019.1615504. Epub 2019 May 20. PMID: 31107163.

Narayanan, H., Seidler, T., Luna, M. F., Sokolov, M., Morbidelli, M., & Butté, A. (2021). Hybrid Models for the simulation and prediction of chromatographic processes for protein capture. Journal of Chromatography A, 1650, 462248.

von Lieres, E.; Andersson, J.: A fast and accurate solver for the general rate model of column liquid chromatography, Computers and Chemical Engineering 34,8 (2010), 1180-1191.

Adams T. A. and Seider W. D. Practical optimization of complex chemical processes with tight constraints, Computer and Chemical Engineering, vol. 32, (2008) 2099-2112.

Yu, T., et al. (2023). An iterative augmented unscented Kalman filter for simultaneous state-parameter-input estimation for systems with/without direct feedthrough. Mechanical Systems and Signal Processing, 205, 110793. Wang, Y., Xia, M., Yang, Q., Song, Y., Chen, Q., & Chen, Y. (2021). Augmented state estimation of line parameters in active power distribution systems with phasor measurement units. IEEE Transactions on Power Delivery, 37(5), 3835-3845.

Valipour, M., & Ricardez-Sandoval, L. A. (2021). Assessing the impact of EKF as the arrival cost in the moving horizon estimation under nonlinear model predictive control. Industrial & Engineering Chemistry Research, 60(7), 2994-3012.

*Equivalents and Scope*

Unless context dictates otherwise, the descriptions and definitions of the features set out above are not limited to any particular aspect or embodiment of the invention and apply equally to all aspects and embodiments which are described. Any section headings used herein are for organizational purposes only and are not to be construed as limiting the subject matter described. "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein. It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about" or "approximately", it will be understood that the particular value forms another embodiment. The terms "about" or "approximately" in relation to a numerical value is optional and means for example +/- 10%. Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps. Other aspects and embodiments of the invention provide the aspects and embodiments described above with the term "comprising" replaced by the term "consisting of" or "consisting essentially of", unless the context dictates otherwise.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof. While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention, which is defined by the appended claims. For the avoidance of any doubt, any theoretical explanations provided herein are provided for the purposes of improving the understanding of a reader. The inventors do not wish to be bound by any of these theoretical explanations.

**Claims**

1. A computer-implemented method for controlling a bioprocess comprising a cell culture in a bioreactor, wherein the bioprocess is operated as a perfusion bioprocess, the method comprising:

   receiving one or more constraints that apply to one or more controlled and/or manipulated variables of the controlled bioprocess;
   identifying a value of one or more manipulated variables of the controlled bioprocess that optimises an objective function that is a function of one or more variables of the bioprocess including one or more controlled variables, wherein values of the controlled variables are predicted using a dynamic model of the bioprocess , and the identified values are such that the constraints that apply to one or more controlled and/or manipulated variables of the controlled bioprocess are satisfied;
   wherein the constraints comprise constraints identified using a method of simulating a chromatographic process that is coupled with the bioprocess for processing of a harvest stream of the bioprocess and/or wherein the objective function comprises a term that is associated with predictions obtained from a method of simulating a chromatographic process using chromatographic process operating parameters derived from one or more manipulated and/or controlled variables of the bioprocess.

2. The method of claim 1, wherein the objective function comprises a cost of goods function that captures the costs associated with values of one or more variables of the bioprocess, wherein said costs comprise costs associated with the chromatographic process estimated using the method of simulating a chromatographic process, optionally wherein said costs further comprise costs associated with the bioprocess and/or

wherein the cost of goods function is a function of at least the harvest flow rate and concentration of one or more products in the harvest flow rate ( $\sum_{i=k+1}^{k+P} Cost_{total}(F_{h_i}, \varphi_i)$ ).

3. The method of claim 2, wherein the cost of goods function is a function that maps: (i) a pair of operating parameters comprising a value of a harvest flow rate or range of harvest flow rates, and a value of a harvest product concentration or range of harvest product concentration, and optionally the concentration of one or more further compounds in the harvest flow, to (ii) one or more metrics derived from a prediction of the method of simulating a chromatographic process over a predetermined period of time which are associated with positive or negative costs due to the materials and devices used in the chromatographic process, and/or the characteristics of the product recovered through the chromatography process.

4. The method of any of claims 2 or 3, wherein said identifying comprises determining the costs associated with one or more candidate sets of variables of the bioprocess comprising a harvest flow rate and a harvest product concentration using said harvest flow rate and harvest product concentration as input to a method of simulating a chromatographic process, or receiving previously determined costs associated with a plurality of candidate sets of variables of the bioprocess comprising a harvest flow rate and a harvest product concentration using said harvest flow rate and harvest product concentration as input to a method of simulating a chromatographic process.

5. The method of any preceding claim, wherein the objective function comprises one or more of: (i) a term that penalises differences between the prediction of the value of the controlled variables from the dynamic model and corresponding predetermined target values; (ii) a term that penalises changes in the value of the one or more manipulated variables; (iii) a term that penalises differences between a feed flow rate and a target feed flow rate associated with a predetermined perfusion target perfusion rate; (iv) a term that rewards maximisation of one or more predetermined variables, optionally wherein the term that rewards maximisation of one or more predetermined variables is expressed

as $\Upsilon_{z_i} \sum_{i=k+1}^{k+P} \frac{1}{z_i^2+1}$ where z is any predetermined variable to be maximized, and $\Upsilon_{z_i}$ is a weight set to a non-zero scalar value that sets the importance of the term relative to other terms of the objective function, k is a current time and k+P is a prediction horizon over which the dynamic model is evaluated.

6. The method of any preceding claim, wherein the objective function is a function as described in Equation (2) and/or wherein identifying a value of one or more manipulated variables of the controlled bioprocess that optimises an objective function is performed according to equation (1).

7. The method of any preceding claim, wherein the method comprises using a state observer to provide an estimate of the value of one or more state variables of the bioprocess, the one or more state variables of the bioprocess comprising the one or more controlled variables, using one or more measurements of the state variables of the bioprocess and the dynamic model of the bioprocess .

8. The method of claim 7, wherein the state observer is a moving horizon estimator and/or a moving horizon estimator that models one or more parameters of the dynamic model of the bioprocess as a state variable to be estimated.

9. The method of any preceding claim, wherein the method comprises identifying one or more values of the one or more manipulated variables that minimises the control objective over a time period, and controlling the bioprocess to use one of the one or more values for each of the one or more manipulated variables.

10. The method of any preceding claim, wherein identifying a value of the one or more manipulated variables that optimises a control objective comprises using the dynamic model of the bioprocess to predict a state trajectory of the bioprocess with one or more candidate values of the one or more manipulated variables, by finding values of the one or more state space variables that represent a solution of the dynamic model at one or more future time points, and using the prediction of the value of the subset of controlled variables in said state trajectory as variables of the control objective, optionally wherein the method comprises selecting and comparing candidate values using an optimisation algorithm.

**11.** The method of any preceding claim, wherein said dynamic model comprises a kinetic growth model, optionally a model according to equations (11) to (14).

**12.** The method of any preceding claim, wherein the method of simulating a chromatographic process comprises, for a chromatography process whereby a feed flow comprising one or more products is passing through a chromatography unit comprising a stationary phase that the one or more products interact with:

solving a bulk flow mass balance model over one or more discrete volume elements along the chromatography unit by numerical integration,
wherein the bulk flow mass balance model is an ordinary differential equations model that represents the change in concentration of bound and unbound fractions of the one or more products in a discrete volume element, wherein the ordinary differential equations model comprises binding and diffusion terms, wherein a binding term captures the binding of a product to the stationary phase and a diffusion term captures the diffusion of a bound product, wherein said binding and diffusion terms are each parameterised by a single respective parameter that is predicted by a machine learning model trained to take inputs comprising the concentration of the bound and unbound fractions of the one or more products in a discrete volume element and produce as output a prediction of the parameters of the binding and diffusion terms.

**13.** The method of claim 18, wherein the bulk flow mass balance model comprises linear terms representing the flow of unbound compounds (products and optionally inerts) in and out of the discrete volume element, and the binding and diffusion terms, wherein a binding term captures non-linearities of the process of adsorption of unbound product on the static phase through the prediction of the respective parameter of the term by the machine learning model at each iteration of the numerical integration; and/or wherein the bulk flow mass balance model comprises equations (8) and (9) and optionally equation (10) below:

$$\frac{d\vec{c}_{u,n}}{dt} = \frac{f_k}{\Delta V}\left(\vec{c}_{u,n-1} - \vec{c}_{u,n}\right) - \tilde{k}_a \circ \vec{c}_{u,n} + \vec{k}_d \circ \vec{c}_{b,n} \quad (8)$$

$$\frac{d\vec{c}_{b,n}}{dt} = \tilde{k}_a \circ \vec{c}_{u,n} - \vec{k}_d \circ \vec{c}_{b,n} \qquad (9)$$

$$\frac{d\vec{c}_{i,n}}{dt} = \frac{f_k}{\Delta V}\left(\vec{c}_{i,n-1} - \vec{c}_{i,n}\right) \qquad (10)$$

where $f_k$ is the feed flow rate, $\Delta V$ is the volume of the discrete volume element $n$, $\vec{c}_{u,n}$ is a vector of concentrations of the unbound products in the discrete volume element n, $\vec{c}_{b,n}$ is a vector of concentrations of the bound products in the discrete volume element $n$, $\vec{c}_{u,n-1}$ is a vector of concentrations of the unbound products in the discrete volume element preceding the discrete volume element n, where discrete volume elements are sequentially labelled from input to output of the chromatography unit, $\vec{c}_{i,n}$ is a vector of concentrations of one or more inerts in the discrete volume element $n$, $\vec{c}_{i,n-1}$ is a vector of concentrations of the inerts in the discrete volume element preceding the discrete volume element n, and $\circ$ is an elementwise product.

**14.** The method of claim 18 or claim 19, wherein the machine learning model is a recurrent machine learning model, wherein a recurrent machine learning model is a machine learning model that is able to account of the values of one or more predictions made at one or more preceding iterations of the numerical integration when making predictions at a current iteration of the numerical integration.

**15.** A system for controlling a bioprocess, the system including:

at least one processor; and
at least one non-transitory computer readable medium containing instructions that, when executed by the at least one processor, cause the at least one processor to perform the method of any of preceding claim;

optionally wherein the system further comprises, in operable connection with the processor, one or more of:

a user interface;
one or more biomass sensor(s);
one or more metabolite sensor(s);
one or more volume/level sensors;
a control unit;
one or more effector device(s), optionally wherein the one or more effector devices are operable to set the value of one or more manipulated variables, and
a chromatography control unit.

Fig. 1

Fig. 2A

Fig. 2B

310

Determine current values of one or more process parameters

320

Obtain estimates of state variables of the process using state observer

330

Controller receives constraints

340

Controller identifies values of MV that optimise objective function

350

Control process using identified values

Fig. 3

410

Obtain feed flow rate, inlet product(s) concentrations and
optionally inert(s) concentrations) concentrations

411

Obtain training data

412

Convert volumetric feed flow rate to linear velocity or residence
time

413

Train machine learning model

413A
Set weights to initial weights corresponding to predetermined
values of predicted parameters
obtain predetermined values of predicted parameters (413A-1)
set weights of model to default values (413A-2)
pretrain ML model using loss function based on difference
between predicted values and predetermined values of
coefficients (413A-3)

413B
Solve bulk flow mass balance model using predicted
values at preceding iteration

413C
Evaluate loss function

413D
Update weights based on loss function

414
Solve mass balance model

Obtain prediction of binding and diffusion parameters in each
discrete volume element using machine learning model
414A

Evaluate mass balance model over all discrete volumes
414B

416
Determine values of metric(s) derived from solution of mass balance model

418
Provide one or more of values of metric(s) to upstream process controller

Fig. 4A

Fig. 4B

Fig. 5

**User Entered Information**
- Operational targets, economic metrics, constraints

Fig. 6

Fig. 7

Fig. 8A, D

Fig. 8B, C

Fig. 9A-B

C

D

Fig. 9C-D

Fig. 9E-F

Fig. 9G-H

Fig. 10

Fig. 11A

Fig. 11B

$$\frac{d\vec{c}_{u,n}}{dt} = \frac{f_k}{\Delta V}\left(\vec{c}_{u,n-1} - \vec{c}_{u,n}\right) - \tilde{k}_a \circ \vec{c}_{u,n} + \vec{k}_d \circ \vec{c}_{b,n}$$

$$\frac{d\vec{c}_{b,n}}{dt} = \tilde{k}_a \circ \vec{c}_{u,n} - \vec{k}_d \circ \vec{c}_{b,n}$$

$$\frac{d\vec{c}_{i,n}}{dt} = \frac{f_k}{\Delta V}\left(\vec{c}_{i,n-1} - \vec{c}_{i,n}\right)$$

*Where ∘ denotes the Hadamard product (elementwise product)

# Fig. 12

Fig. 13

Fig. 14A

Fig. 14A →

**Generate pre-training "data"**
- Select random values of $\vec{c}_{u,k}, \vec{c}_{i,k}, \vec{c}_{b,k}, f_k, \vec{x}_{r,k}$
- Calculate "linearized" $\tilde{k}_a$ as:

$$\tilde{k}_a = \vec{k}_a \circ (\vec{c}_{tot} - \vec{c}_b)$$

~ 828

**Pre-train fully connected neural network**
- Fixed number of epochs (10000)
- Loss function penalized differences between:
  - Calculated $\tilde{k}_a$ and network predictions
  - Constant $\vec{k}_d$ and network predictions

~ 830

**Simulate column using full binding model**
- For each batch in training and validation set
- Durations from dataset
- Feed rate trajectories from dataset
- Inlet concentrations trajectories from dataset

~ 832

840

Gradient descent
update weights:
$\vec{w}_\alpha, \vec{w}_u, W^{[1\cdots L]}$

**Simulate fraction collector [optional]**
- Replicate fractionation scheme from dataset
- Calculate fraction concentrations

~ 834

**Calculate training and validation loss**   ~ 836

stop

no

838

yes

Trained Model

# Fig. 15B

Start

**Forward pass of neural network:**
Calculate $\widetilde{k}_{a,n}$, $\vec{k}_{d,n}$, and $\vec{x}_{r,k+1,n}$ for all
$n \in [1, \cdots, N]$ using neural network and
inputs: $\vec{x}_{r,k,n}$, $\vec{c}_{u,k,n}$, $\vec{c}_{b,k,n}$, $\vec{c}_{i,k,n}$, $f_k$

Linearize ODEs around current state (Fig. 15B)

# Fig. 15A

$$\begin{bmatrix} \frac{d\vec{x}_u}{dt} \\ \frac{d\vec{x}_b}{dt} \\ \frac{d\vec{x}_i}{dt} \end{bmatrix} = \begin{bmatrix} A_{u,u} & A_{u,b} & 0 \\ A_{b,u} & A_{b,b} & 0 \\ 0 & 0 & A_{i,i} \end{bmatrix} \begin{bmatrix} \vec{x}_u \\ \vec{x}_b \\ \vec{x}_i \end{bmatrix} + \begin{bmatrix} B_u & 0 & B_i \end{bmatrix} \vec{u}$$

$$\vec{x}_u = \begin{bmatrix} \vec{c}_{u,entry} \\ \vec{c}_{u,1} \\ \vec{c}_{u,2} \\ \vdots \\ \vec{c}_{u,n} \\ \vec{c}_{u,exit} \end{bmatrix}$$

$$A_{u,b} = \begin{bmatrix} \text{diag}\left(\vec{k}_{d,1}\right) & 0 & \cdots & 0 & 0 \\ 0 & \text{diag}\left(\vec{k}_{d,2}\right) & \cdots & 0 & 0 \\ \vdots & \vdots & \ddots & \vdots & \vdots \\ 0 & 0 & \cdots & \text{diag}\left(\vec{k}_{d,n}\right) & 0 \end{bmatrix}$$

$$\vec{u} = \begin{bmatrix} \vec{c}_{u,in} \\ \vec{c}_{i,in} \end{bmatrix}$$

$$B_u = \begin{bmatrix} \frac{f_k}{V_{entry}} I_U & 0 \\ 0 & 0 \\ 0 & 0 \\ \vdots & \vdots \\ 0 & 0 \\ 0 & 0 \end{bmatrix}$$

$$A_{u,u} = \begin{bmatrix} \frac{-f_k}{V_{entry}} I_U & 0 & 0 & \cdots & 0 & 0 \\ \frac{f_k}{\Delta V} I_U & \left(\frac{-f_k}{\Delta V} I_U - \text{diag}(\widetilde{k}_{a,1})\right) & 0 & \cdots & 0 & 0 \\ 0 & \frac{f_k}{\Delta V} I_U & \left(\frac{-f_k}{\Delta V} I_U - \text{diag}(\widetilde{k}_{a,2})\right) & \ddots & 0 & 0 \\ \vdots & \vdots & \ddots & \ddots & \vdots & \vdots \\ 0 & 0 & 0 & \frac{f_k}{\Delta V} I_U & \left(\frac{-f_k}{\Delta V} I_U - \text{diag}(\widetilde{k}_{a,n})\right) & 0 \\ 0 & 0 & 0 & \cdots & \frac{f_k}{\Delta V} I_U & \frac{-f_k}{V_{exit}} I_U \end{bmatrix}$$

Fig. 15B-1

$$\begin{bmatrix} \dfrac{d\vec{x}_u}{dt} \\[4pt] \dfrac{d\vec{x}_b}{dt} \\[4pt] \dfrac{d\vec{x}_i}{dt} \end{bmatrix} = \begin{bmatrix} A_{u,u} & A_{u,b} & 0 \\ A_{b,u} & A_{b,b} & 0 \\ 0 & 0 & A_{i,i} \end{bmatrix} \begin{bmatrix} \vec{x}_u \\ \vec{x}_b \\ \vec{x}_i \end{bmatrix} + \begin{bmatrix} B_u \\ 0 \\ B_i \end{bmatrix} \vec{u}$$

$$\vec{x}_b = \begin{bmatrix} \vec{c}_{b,1} \\ \vec{c}_{b,2} \\ \vdots \\ \vec{c}_{b,n} \end{bmatrix}$$

$$A_{b,u} = \begin{bmatrix} \operatorname{diag}\left(\vec{k}_{a,1}\right) & 0 & \cdots & 0 \\ 0 & \operatorname{diag}\left(\vec{k}_{a,2}\right) & \cdots & 0 \\ \vdots & \vdots & \ddots & \vdots \\ 0 & 0 & \cdots & \operatorname{diag}\left(\vec{k}_{a,n}\right) \end{bmatrix}$$

$$A_{b,b} = \begin{bmatrix} \operatorname{diag}\left(-\vec{k}_{d,1}\right) & 0 & \cdots & 0 \\ 0 & \operatorname{diag}\left(-\vec{k}_{d,2}\right) & \cdots & 0 \\ \vdots & \vdots & \ddots & \vdots \\ 0 & 0 & \cdots & \operatorname{diag}\left(-\vec{k}_{d,n}\right) \end{bmatrix}$$

Fig. 15B-2

$$\begin{bmatrix} \dfrac{d\vec{x}_u}{dt} \\[4pt] \dfrac{d\vec{x}_b}{dt} \\[4pt] \dfrac{d\vec{x}_i}{dt} \end{bmatrix} = \begin{bmatrix} A_{u,u} & A_{u,b} & 0 \\ A_{b,u} & A_{b,b} & 0 \\ 0 & 0 & A_{i,i} \end{bmatrix} \begin{bmatrix} \vec{x}_u \\ \vec{x}_b \\ \vec{x}_i \end{bmatrix} + \begin{bmatrix} B_u \\ 0 \\ B_i \end{bmatrix} \vec{u}$$

$$\vec{x}_i = \begin{bmatrix} \vec{c}_{i,entry} \\ \vec{c}_{i,1} \\ \vec{c}_{i,2} \\ \vdots \\ \vec{c}_{i,n} \\ \vec{c}_{i,exit} \end{bmatrix}$$

$$\vec{u} = \begin{bmatrix} \vec{c}_{u,in} \\ \vec{c}_{i,in} \end{bmatrix}$$

$$B_i = \begin{bmatrix} \dfrac{f_k}{V_{entry}} & 0 & 0 & \cdots & 0 & 0 \\ 0 & 0 & 0 & \cdots & 0 & 0 \end{bmatrix}$$

$$A_{i,i} = \begin{bmatrix} \dfrac{-f_k}{V_{entry}} I_l & 0 & 0 & \cdots & 0 & 0 \\[6pt] \dfrac{f_k}{\Delta V} I_l & \dfrac{-f_k}{\Delta V} I_l & 0 & \cdots & 0 & 0 \\[6pt] 0 & \dfrac{f_k}{\Delta V} I_l & \dfrac{-f_k}{\Delta V} & \cdots & 0 & 0 \\[6pt] \vdots & \vdots & \ddots & \ddots & \vdots & \vdots \\[6pt] 0 & 0 & \cdots & \dfrac{f_k}{\Delta V} & \dfrac{-f_k}{\Delta V} I_l & 0 \\[6pt] 0 & 0 & \cdots & \cdots & \dfrac{f_k}{V_{exit}} I_l & \dfrac{-f_k}{V_{exit}} I_l \end{bmatrix}$$

Fig. 15B-3

$$
\begin{bmatrix} \dfrac{d\vec{c'}_{u,entry}}{dt} \\ \dfrac{d\vec{c'}_{i,entry}}{dt} \\ \dfrac{d\vec{c'}_{u,1}}{dt} \\ \dfrac{d\vec{c'}_{u,2}}{dt} \\ \vdots \\ \dfrac{d\vec{c'}_{u,n}}{dt} \\ \dfrac{d\vec{c'}_{b,1}}{dt} \\ \dfrac{d\vec{c'}_{b,2}}{dt} \\ \vdots \\ \dfrac{d\vec{c'}_{b,n}}{dt} \\ \dfrac{d\vec{c'}_{i,1}}{dt} \\ \dfrac{d\vec{c'}_{i,2}}{dt} \\ \vdots \\ \dfrac{d\vec{c'}_{i,n}}{dt} \\ \dfrac{d\vec{c'}_{u,exit}}{dt} \\ \dfrac{d\vec{c'}_{i,exit}}{dt} \end{bmatrix}
=
\begin{bmatrix} \vec{x}_u \\ \vec{x}_b \\ \vec{x}_i \end{bmatrix}
+
\begin{bmatrix} B_u \\ \mathbf{0} \\ B_i \end{bmatrix}
\vec{u}
$$

Fig. 15B-4

Fig. 16A

| Run ID | $R^2$ | RMSE | Chart |
|--------|-------|------|-------|
| 1_10 | 99.8905% | 0.0084 | |
| 0.8_2 | 99.8065% | 0.0139 | |

Fig. 16B

Fig. 16C

| Run ID | $R^2$ | RMSE | Chart |
|--------|-------|------|-------|
| 3070_10 | 99.8707% | 0.0390 | |
| 3070_3 | 99.4343% | 0.0984 | |
| 694_10 | 98.5926% | 0.0282 | |
| 694_3 | 99.7463% | 0.0136 | |

Fig. 16D

Fig. 17

Europäisches
Patentamt

European
Patent Office

Office européen
des brevets

# EUROPEAN SEARCH REPORT

**Application Number**

EP 24 16 1030

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/283561 A1 (BUDDHIRAJU VENKATA SUDHEENDRA [IN] ET AL) 8 September 2022 (2022-09-08) * paragraphs [0002] - [0007], [0017] - [0034], [0042], [0043], [0047] - [0050]; figures 1-5B * | 1-15 | INV. C12M1/36 G05B13/04 G01N30/86 C12M1/00 |
| X | US 2021/269888 A1 (HYCKENBERG KEY [SE] ET AL) 2 September 2021 (2021-09-02) * paragraphs [0019] - [0082]; figures 1-5 * | 1-15 | |
| A | JOAQUÍN GOMIS-FONS ET AL: "Model-based design and control of a small-scale integrated continuous end-to-end mAb platform", BIOTECHNOLOGY PROGRESS, AMERICAN CHEMICAL SOCIETY, HOBOKEN, USA, vol. 36, no. 4, 6 April 2020 (2020-04-06), page n/a, XP072294381, ISSN: 8756-7938, DOI: 10.1002/BTPR.2995 * the whole document * | 1-15 | |
| A | GRÄNICHER GWENDAL ET AL: "A high cell density perfusion process for Modified Vaccinia virus Ankara production: Process integration with inline DNA digestion and cost analysis", BIOTECHNOLOGY AND BIOENGINEERING, vol. 118, no. 12, 23 September 2021 (2021-09-23), pages 4720-4734, XP093050292, Hoboken, USA ISSN: 0006-3592, DOI: 10.1002/bit.27937 Retrieved from the Internet: URL:https://onlinelibrary.wiley.com/doi/fu ll-xml/10.1002/bit.27937> * the whole document * | 1-15 | |

**TECHNICAL FIELDS SEARCHED (IPC)**

C12M
G05B
G01N
B01D

-/--

The present search report has been drawn up for all claims

| Place of search | Date of completion of the search | Examiner |
|---|---|---|
| Berlin | 23 August 2024 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 16 1030

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2023/237614 A1 (SARTORIUS STEDIM DATA ANALYTICS AB [SE]) 14 December 2023 (2023-12-14) * the whole document * ----- | 1-15 | |

TECHNICAL FIELDS SEARCHED (IPC)

The present search report has been drawn up for all claims

| Place of search | Date of completion of the search | Examiner |
|---|---|---|
| Berlin | 23 August 2024 | Pantelidis, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 1030

This annex lists the patent family members relating to the patent documents cited in the above-mentioned European search report.
The members are as contained in the European Patent Office EDP file on
The European Patent Office is in no way liable for these particulars which are merely given for the purpose of information.

23-08-2024

| Patent document cited in search report | Publication date | Patent family member(s) | | Publication date |
|---|---|---|---|---|
| US 2022283561 A1 | 08-09-2022 | EP | 4036672 A1 | 03-08-2022 |
| | | US | 2022283561 A1 | 08-09-2022 |
| US 2021269888 A1 | 02-09-2021 | CN | 112352041 A | 09-02-2021 |
| | | EP | 3814481 A1 | 05-05-2021 |
| | | JP | 7412839 B2 | 15-01-2024 |
| | | JP | 2021528978 A | 28-10-2021 |
| | | KR | 20210027287 A | 10-03-2021 |
| | | US | 2021269888 A1 | 02-09-2021 |
| | | WO | 2020002713 A1 | 02-01-2020 |
| WO 2023237614 A1 | 14-12-2023 | EP | 4289927 A1 | 13-12-2023 |
| | | WO | 2023237614 A1 | 14-12-2023 |

EPO FORM P0459

For more details about this annex : see Official Journal of the European Patent Office, No. 12/82

**REFERENCES CITED IN THE DESCRIPTION**

**Patent documents cited in the description**

- WO 2023237614 A
- WO 2021165480 A
- WO 2021164957 A

**Non-patent literature cited in the description**

- **LIERES** ; **ANDERSSON**. *Chromatography Analysis and Design Toolkit*, 2010
- **SHEKHAWAT LK** ; **RATHORE AS**. An overview of mechanistic modeling of liquid chromatography. *Prep Biochem Biotechnol*, 20 May 2019, vol. 49 (6), 623-638
- **NARAYANAN, H.** ; **SEIDLER, T.** ; **LUNA, M. F.** ; **SOKOLOV, M.** ; **MORBIDELLI, M.** ; **BUTTÉ, A.** Hybrid Models for the simulation and prediction of chromatographic processes for protein capture. *Journal of Chromatography A*, 2021, vol. 1650, 462248
- **VON LIERES, E.** ; **ANDERSSON, J.** A fast and accurate solver for the general rate model of column liquid chromatography. *Computers and Chemical Engineering*, 2010, vol. 34 (8), 1180-1191
- **ADAMS T. A.** ; **SEIDER W. D.** Practical optimization of complex chemical processes with tight constraints. *Computer and Chemical Engineering*, 2008, vol. 32, 2099-2112
- **YU, T. et al.** An iterative augmented unscented Kalman filter for simultaneous state-parameter-input estimation for systems with/without direct feedthrough. *Mechanical Systems and Signal Processing*, 2023, vol. 205, 110793
- **WANG, Y.** ; **XIA, M.** ; **YANG, Q.** ; **SONG, Y.** ; **CHEN, Q.** ; **CHEN, Y.** Augmented state estimation of line parameters in active power distribution systems with phasor measurement units. *IEEE Transactions on Power Delivery*, 2021, vol. 37 (5), 3835-3845
- **VALIPOUR, M.** ; **RICARDEZ-SANDOVAL, L. A.** Assessing the impact of EKF as the arrival cost in the moving horizon estimation under nonlinear model predictive control. *Industrial & Engineering Chemistry Research*, 2021, vol. 60 (7), 2994-3012